# EUROPEAN PATENT SPECIFICATION

(11) **EP 1 546 174 B1**
(45) Date of publication and mention of the grant of the patent: **22.06.2011**
(21) Application number: 03732052.0
(22) Date of filing: 22.01.2003
(51) Int. Cl.: C12N 15/113

(54) **METHODS AND COMPOSITIONS FOR RNA INTERFERENCE**
VERFAHREN UND ZUSAMMENSETZUNGEN FÜR RNA-INTERFERENZ
METHODES ET COMPOSITIONS D'INTERFERENCE ARN

(30) Priority: 22.01.2002 US 55797
(43) Date of publication of application: 29.06.2005
(73) Proprietor: COLD SPRING HARBOR LABORATORY, Cold Spring Harbor, NY 11724 (US)
(72) Inventor: BERNSTEIN, Emily, Huntington, NY 11743 (US); CAUDY, Amy, Melville, NY 11747 (US); HANNON, Gregory J., Huntington, NY 11743 (US); PADDISON, Patrick J., Centerport, NY 11721 (US); CONKLIN, Douglas, Cold Spring Harbor, NY 11724 (US); SCOTT, Hammond, Chapel Hill, NC 27599 (US)
(74) Representative: Chapman, Paul Gilmour
(86) International application number: PCT/US2003/001963
(87) International publication number: WO 2003/062394

(56) References cited:
- WO-A-99/32619
- WO-A2-03/020931
- US-B1- 6 506 559
- BOSHER J M ET AL: "RNA interference can target pre-mRNA: consequences for gene expression in a Caenorhabditis elegans operon." GENETICS NOV 1999, vol. 153, no. 3, November 1999 (1999-11), pages 1245-1256, XP002426569 ISSN: 0016-6731
- MANCHE L ET AL: "INTERACTIONS BETWEEN DOUBLE-STRANDED RNA REGULATORS AND THE PROTEINKINASE DAI" MOLECULAR AND CELLULAR BIOLOGY, AMERICAN SOCIETY FOR MICROBIOLOGY, WASHINGTON, US, vol. 12, no. 11, November 1992 (1992-11), pages 5238-5248, XP001009924 ISSN: 0270-7306
- HASUWA H ET AL: "Small interfering RNA and gene silencing in transgenic mice and rats" FEBS LETTERS, ELSEVIER, AMSTERDAM, NL, vol. 532, no. 1-2, 4 December 2002 (2002-12-04), pages 227-230, XP004395375 ISSN: 0014-5793
- BRUMMELKAMP THIJN R ET AL: "A system for stable expression of short interfering RNAs in mammalian cells.", SCIENCE (NEW YORK, N.Y.) 19 APR 2002, vol. 296, no. 5567, 19 April 2002 (2002-04-19), pages 550-553, ISSN: 1095-9203
- ELBASHIR SAYDA M ET AL: "RNA interference is mediated by 21- and 22-nucleotide RNAs", GENES AND DEVELOPMENT, COLD SPRING HARBOR LABORATORY PRESS, PLAINVIEW, NY, US, vol. 15, no. 2, 15 January 2001 (2001-01-15), pages 188-200, XP002206453, ISSN: 0890-9369, DOI: DOI:10.1101/GAD.862301

## Description

### Background of the Invention

"RNA interference", "post-transcriptional gene silencing", "quelling" - these different names describe similar effects that result from the overexpression or misexpression of transgenes, or from the deliberate introduction of double-stranded RNA into cells (reviewed in Fire A (1999) Trends Genet 15: 358-363; Sharp PA (1999) Genes Dev 13: 139-141; Hunter C (1999) Curr Biol 9: R440-R442; Baulcombe DC (1999) Curr Biol 9: R599-R601; Vaucheret et al. (1998) Plant J 16: 651-659). The injection of double-stranded RNA into the nematode *Caenorhabditis elegans,* for example, acts systemically to cause the post-transcriptional depletion of the homologous endogenous RNA (Fire et al. (1998) Nature 391: 806-811; and Montgomery et al. (1998) PNAS 95: 15502-15507). RNA interference, commonly referred to as RNAi, offers a way of specifically and potently inactivating a cloned gene, and is proving a powerful tool for investigating gene function. Although the phenomenon is interesting in its own right; the mechanism has been rather mysterious, but recent research - for example that recently reported by Smardon et al. (2000) Curr Biol 10: 169-178- is beginning to shed light on the nature and evolution of the biological processes that underlie RNAi.

RNAi was discovered when researchers attempting to use the antisense RNA approach to inactivate a *C. elegans* gene found that injection of sense-strand RNA was actually as effective as the antisense RNA at inhibiting gene function (Guo et al. (1995) Cell 81: 611-620). Further investigation revealed that the active agent was modest amounts of double-stranded RNA that contaminate *in vitro* RNA preparations. Researchers quickly determined the 'rules' and effects of RNAi which have become the paradigm for thinking about the mechanism which mediates this affect. Exon sequences are required, whereas introns and promoter sequences, while ineffective, do not appear to compromise RNAi (though there may be gene-specific exceptions to this rule). RNAi acts systemically - injection into one tissue inhibits gene function in cells throughout the animal. The results of a variety of experiments, in *C. elegans* and other organisms, indicate that RNAi acts to destabilize cellular RNA after RNA processing.

The potency of RNAi inspired Timmons and Fire (1998 Nature 395: 854) to do a simple experiment that produced an astonishing result. They fed to nematodes bacteria that had been engineered to express double-stranded RNA corresponding to the *C. elegans unc-22* gene. Amazingly, these nematodes developed a phenotype similar to that of *unc-22* mutants that was dependent on their food source. The ability to conditionally expose large numbers of nematodes to gene-specific double-stranded RNA formed the basis for a very powerful screen to select for RNAi-defective *C. elegans* mutants and then to identify the corresponding genes.

Double-stranded RNAs (dsRNAs) can provoke gene silencing in numerous in vivo contexts including *Drosophila, Caenorhabditis elegans,* planaria, hydra, trypanosomes, fungi and plants. However, the ability to recapitulate this phenomenon in higher eukaryotes, particularly mammalian cells, has not been accomplished in the art. Nor has the prior art demonstrated that this phenomena can be observed in cultured eukaryotic cells. Additionally, the 'rules' established by the prior art have taught that RNAi requires exon sequences, and thus constructs consisting of intronic or promoter sequences were not believed to be effective reagents in mediating RNAi. The present invention aims to address each of these deficiencies in the prior art and provides evidence both that RNAi can be observed in cultured eukaryotic cells and that RNAi constructs consisting of non-exon sequences can effectively repress gene expression.

### Summary of the Invention

One aspect of the present invention provides a method for attenuating expression of a target gene in a mammalian cell, the method comprising introducing into the mammalian cell *in vitro,* a chromosomally integrating expression vector comprising a sequence encoding a short hairpin RNA (shRNA), wherein the shRNA comprises a double stranded region of between 20 and 29 bases in length, the double stranded region comprising a sequence that hybridizes under stringent conditions to a coding region_of the target gene, whereby expression of the target gene is attenuated.

Another aspect of the present invention provides a chromosomally integrating expression vector for use in a method of attenuating expression of a target gene in a mammalian cell, the expression vector comprising a sequence encoding a short hairpin RNA (shRNA), wherein the shRNA comprises a double stranded region of between 19 and 29 base pairs in length, the double stranded region comprising a sequence that hybridizes under stringent conditions to a coding region of the target gene.

The present invention further provides use of a chromosomally integrating expression vector for the manufacture of a medicament for use in therapy, the expression vector comprising a sequence encoding a short hairpin RNA (shRNA), wherein the shRNA comprises a double stranded region of between 19 and 29 base pairs in length, the double stranded region comprising a sequence that hybridizes under stringent conditions to a coding region of the target gene, whereby expression of the target gene is attenuated.

In certain embodiments, the cell is suspended in culture; while in other embodiments the cell is in a whole animal, such as a non-human mammal.

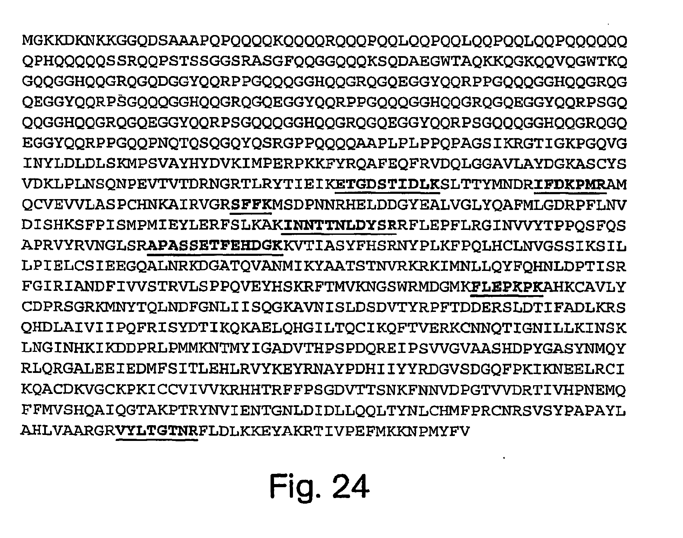

The cell may be engineered with (i) a recombinant gene encoding a Dicer activity, (ii) a recombinant gene encoding an Argonaut activity, or (iii) both. For instance, the recombinant gene may encode, for a example, a protein which includes an amino acid sequence at least 50 percent identical to SEQ ID No. 2 or 4; or be defined by a coding sequence which hybridizes under wash conditions of 2 x SSC at 22 °C to SEQ ID No. 1 or 3. In certain embodiments, the recombinant gene may encode, for a example, a protein which includes an amino acid sequence at least 50 percent identical to the Argonaut sequence shown in Figure 24. In certain embodiments, the recombinant gene may encode a protein which includes an amino acid sequence at least 60 percent, 70 percent, 80 percent, 85 percent, 90 percent, or 95 percent identical to SEQ ID No. 2 or 4. In certain embodiments, the recombinant gene may be defined by a coding sequence which hybridizes under stringent conditions, including a wash step selected from 0.2X-2.0X SSC at from 50 °C-65 °C, to SEQ ID No. 1 or 3.

Rather than use a heterologous expression construct(s), an endogenous Dicer gene or Argonaut gene can be activated, e.g, by gene activation technology, expression of activated transcription factors or other signal transduction protein(s), which induces expression of the gene, or by treatment with an endogenous factor which upregulates the level of expression of the protein or inhibits the degradation of the protein.

The target gene may be an endogenous gene of the cell, or the target gene may be a heterologous gene relative to the genome of the cell, such as a pathogen gene, e.g., a viral gene.

The cell may be treated with an agent that inhibits protein kinase RNA-activated (PKR) apoptosis, such as by treatment with agents which inhibit expression of PKR, cause its destruction, and/or inhibit the kinase activity of PKR.

The cell may be a primate cell, such as a human cell.

In certain preferred embodiments, the length of the dsRNA is at least 20, 21 or 22 nucleotides in length, e.g., corresponding in size to RNA products produced by Dicer-dependent cleavage.

Expression of the target gene may be attenuated by at least 5 fold, and more preferably at least 10, 20 or even 50 fold, e.g., relative to the untreated cell or a cell treated with a dsRNA construct which does not correspond to the target gene.

In accordance with the invention expression of a target gene in cultured cells, comprises introducing an expression vector having a "coding sequence" which, when transcribed, produces double stranded RNA (dsRNA) in the cell in an amount sufficient to attenuate expression of the target gene, wherein the dsRNA comprises a nucleotide sequence that hybridizes under stringent conditions to a nucleotide sequence of the target gene. In accordance with the invention the vector includes a coding sequence which forms a hairpin. The vectors are chromosomally integrated, e.g., to produce a stably transfected cell line. Preferred vectors for forming such stable cell lines are described in US Patent 6,025,192 and PCT publication WO/9812339.

In accordance, the present invention provides a double stranded (ds) RNA for inhibiting expression of a mammalian gene. The dsRNA comprises a first nucleotide sequence that hybridizes under stringent conditions, including a wash step of 0.2X SSC at 65 °C, to a nucleotide sequence of at least one mammalian gene and a second nucleotide sequence which is complementary to the first nucleotide sequence.

In one embodiment, the first nucleotide sequence of said double-stranded RNA is at least 20, 21, 22, 25 nucleotides in length.

In accordance with the invention, the first nucleotide sequence of said double-stranded RNA may be identical to at least one mammalian gene. The first nucleotide sequence of said double-stranded RNA may hybridize under stringent conditions to at least one human gene. The first nucleotide sequence of said double-stranded RNA may be identical to at least one human gene.

The double-stranded RNA is a hairpin, and may be expressed transiently or stably. In accordance with the invention, the double-stranded RNA is a hairpin comprising a first nucleotide sequence that hybridizes under stringent conditions to a nucleotide sequence of at least one mammalian gene, and a second nucleotide sequence which is a complementary inverted repeat of said first nucleotide sequence and hybridizes to said first nucleotide sequence to form a hairpin structure.

The first nucleotide sequence of said double-stranded RNA hybridizes to the coding sequence of at least one mammalian gene.The first nucleotide sequence of said double-stranded RNA hybridizes to a coding sequence of at least one mammalian gene. The first nucleotide sequence of said double-stranded RNA may be identical to a coding sequence of at least one mammalian gene.

The present invention may be used in a method for inhibiting RNAi by inhibiting the expression or activity of an RNAi enzyme. Thus, the subject method may include inhibiting the activity of Dicer and/or the 22-mer RNA.

Still another use relates to a method for altering the specificity of an RNAi by modifying the sequence of the RNA component of the RNAi enzyme.

In another use, gene expression in an undifferentiated stem cell, or the differentiated progeny thereof, is altered by introducing dsRNA of the present invention. The stem cells may be embryonic stem cells. The embryonic stem cells are derived from non-human mammals, preferably from non-human primates.

Non-human embryonic stem cells may be isolated by methods known to one of skill in the art from the inner cell mass (ICM) of blastocyst stage embryos. In one embodiment the embryonic stem cells are obtained from previously established cell lines. In a further embodiment, non-human embryonic stem cells are derived *de novo* by standard methods.

Non-human embryonic stem cells may be the result of nuclear transfer. The donor nuclei are obtained from any adult, fetal, or embryonic tissue by methods well known in the art. The donor nuclei may be transferred to a recipient oocyte which had previously been modified.The oocyte may be modified using one or more dsRNAs. Exemplary modifications of the recipient oocyte include any changes in gene or protein expression that prevent an embryo derived from said modified oocyte from successfully implanting in the uterine wall. Since implantation in the uterine wall is essential for fertilized mammalian embryos to progress from beyond the blastocyst stage, embryos made from such modified oocytes could not give rise to viable organisms. Non-limiting examples of such modifications include those that decrease or eliminate expression of cell surface receptors (*i.e*., integrins) required for the recognition between the blastocyst and the uterine wall, modifications that decrease or eliminate expression of proteases (*i.e*., collagenase, stromelysin, and plasminogen activator) required to digest matrix in the uterine lining and thus allow proper implantation, and modifications that decrease or eliminate expression of proteases (i.e., strypsin) necessary for the blastocyst to hatch from the zona pellucida. Such hatching is required for implantation.

In another variation, non-human embryonic stem cells may be obtained from fertilization of modified oocytes, or the differentiated progeny thereof, can be modified or further modified with one or more dsRNAs. In a preferred variation, the modification decreases or eliminates MHC expression. Cells modified in this way will be tolerated by the recipient, thus avoiding complications arising from graft rejection. Such modified cells are suitable for transplantation into a related or unrelated patient to treat a condition characterized by cell damage or cell loss.

In another application of the invention, the undifferentiated stem cell is an adult stem cell. Exemplary adult stem cells include, but are not limited to, hematopoietic stem cells, mesenchymal stem cells, cardiac stem cells, pancreatic stem cells, and neural stem cells. Exemplary adult stem cells include any stem cell capable of forming differentiated ectodermal, mesodermal, or endodermal derivatives. Non-limiting examples of differentiated cell types which arise from adult stem cells include: blood, skeletal muscle, myocardium, endocardium, pericardium, bone, cartilage, tendon, ligament, connective tissue, adipose tissue, liver, pancreas, skin, neural tissue, lung, small intestine, large intestine, gall bladder, rectum, anus, bladder, female or male reproductive tract, genitals, and the linings of the body cavity.

An undifferentiated adult stem cell, or the differentiated progeny thereof, may be altered with one or more dsRNAs to decrease or eliminate MHC expression. Cells modified in this way will be tolerated by the recipient, thus avoiding complications arising from graft rejection. Such modified cells are suitable for transplantation into a related or unrelated patient to treat a condition characterized by cell damage or cell loss.

In another application of the invention, a non-human embryonic stem cell, an undifferentiated adult stem cell, or the differentiated progeny of either a non-human embryonic or adult stem cell is altered with one or more dsRNA to decrease or eliminate expression of genes required for HIV infection. In one application, the stem cell is one capable of giving rise to hematopoietic cells. Modified cells with hematopoietic potential can be transplanted into a patient as a preventative therapy or treatment for HIV or AIDS.

Preparations may be used for identifying compounds, especially small organic molecules, which inhibit or potentiate the RNAi activity. Small molecule inhibitors, for example, can be used to inhibit dsRNA responses in cells which are purposefully being transfected with a virus which produces double stranded RNA.

The dsRNA construct may comprise one or more strands of polymerized ribonucleotide. It may include modifications to either the phosphate-sugar backbone or the nucleoside. The double-stranded structure is formed by a single self-complementary RNA strand. RNA duplex formation may be initiated either inside or outside the cell. The dsRNA construct may be introduced in an amount which allows delivery of at least one copy per cell. Higher doses of double-stranded material may yield more effective inhibition. Inhibition is sequence-specific in that nucleotide sequences corresponding to the duplex region of the RNA are targeted for genetic inhibition. In certain embodiments, dsRNA constructs containing a nucleotide sequences identical to a portion of the target gene are preferred for inhibition. RNA sequences with insertions, deletions, and single point mutations relative to the target sequence (i.e., RNA sequences similar to the target sequence) have also been found to be effective for inhibition. Thus, sequence identity may be optimized by alignment algorithms known in the art and calculating the percent difference between the nucleotide sequences. Alternatively, the duplex region of the RNA may be defined functionally as a nucleotide sequence that is capable of hybridizing with a portion of the target gene transcript. Sequences with insertions, deletions, and single point mutations relative to the target non-coding sequence may also be used.

The present invention may be used to develop transgenic non-human mammals which include a transgene encoding a dsRNA construct, wherein the dsRNA is identical or similar to the coding sequence of the target gene, preferably which is stably integrated into the genome of cells in which it occurs. Non-human animals can be derived by oocyte microinjection, for example, in which case all of the nucleated cells of the animal will include the transgene, or can be derived using embryonic stem (ES) cells which have been transfected with the transgene, in which case the animal is a chimera and only a portion of its nucleated cells will include the transgene. In certain instances, the sequence-independent dsRNA response, e.g., the PKR response, is also inhibited in those cells including the transgene.

In still other embodiments, dsRNA itself can be introduced into an ES cell in culture in order to effect gene silencing, and that phenotype will be carried for at least several rounds of division, e.g., into the progeny of that cell.

### Brief Description of the Drawings

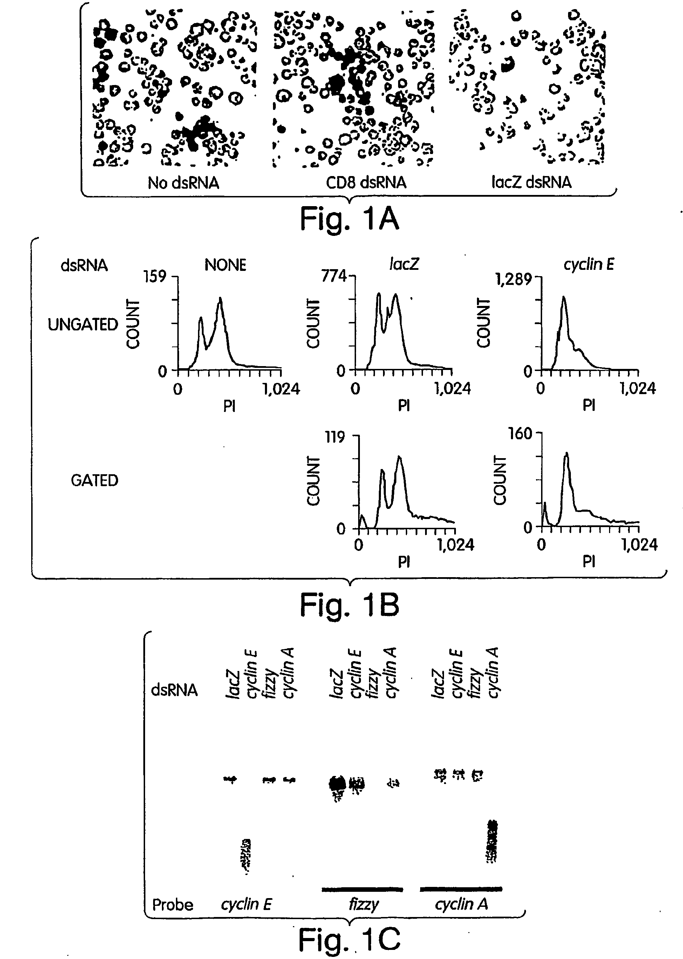

Figure 1: RNAi in S2 cells. (**a**) *Drosophila* S2 cells were transfected with a plasmid that directs *lacZ* expression from the copia promoter in combination with dsRNAs corresponding to either human CD8 or lacZ, or with no dsRNA, as indicated. (**b**) S2 cells were co-transfected with a plasmid that directs expression of a GFP-US9 fusion protein and dsRNAs of either *lacZ* or *cyclin E,* as indicated. Upper panels show FACS profiles of the bulk population. Lower panels show FACS profiles from GFP-positive cells. (**c**) Total RNA was extracted from cells transfected with /*acZ, cyclin E, fizzy* or *cyclin A* dsRNAs, as indicated. Northern blots were hybridized with sequences not present in the transfected dsRNAs.

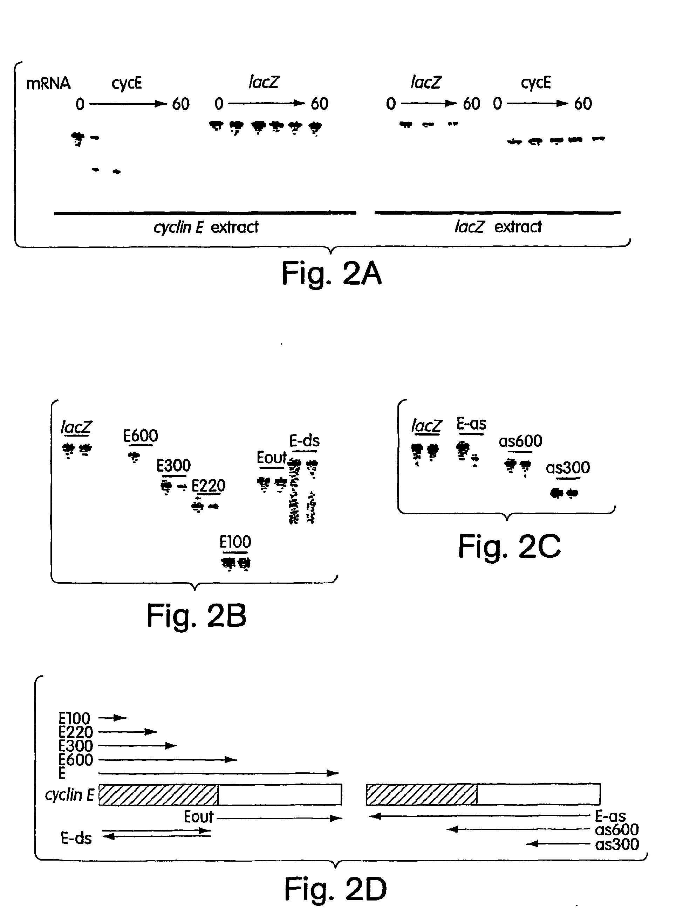

Figure 2: RNAi *in vitro.* (**a**) Transcripts corresponding to either the first 600 nucleotides of *Drosophila cyclin E* (E600) or the first 800 nucleotides of *lacZ* (Z800) were incubated in lysates derived from cells that had been transfected with either /*acZ* or *cyclin E* (cycE) dsRNAs, as indicated. Time points were 0, 10, 20, 30, 40 and 60 min for *cyclin E* and 0, 10, 20, 30 and 60 min for *lacZ.* (**b**) Transcripts were incubated in an extract of S2 cells that had been transfected with *cyclin E* dsRNA (cross-hatched box, below). Transcripts corresponded to the first 800 nucleotides of *lacZ* or the first 600, 300, 220 or 100 nucleotides of *cyclin E,* as indicated. Eout is a transcript derived from the portion of the *cyclin E* cDNA not contained within the transfected dsRNA. E-ds is identical to the dsRNA that had been transfected into S2 cells. Time points were 0 and 30 min. (**c**) Synthetic transcripts complementary to the complete *cyclin E* cDNA (Eas) or the final 600 nucleotides (Eas600) or 300 nucleotides (Eas300) were incubated in extract for 0 or 30 min.

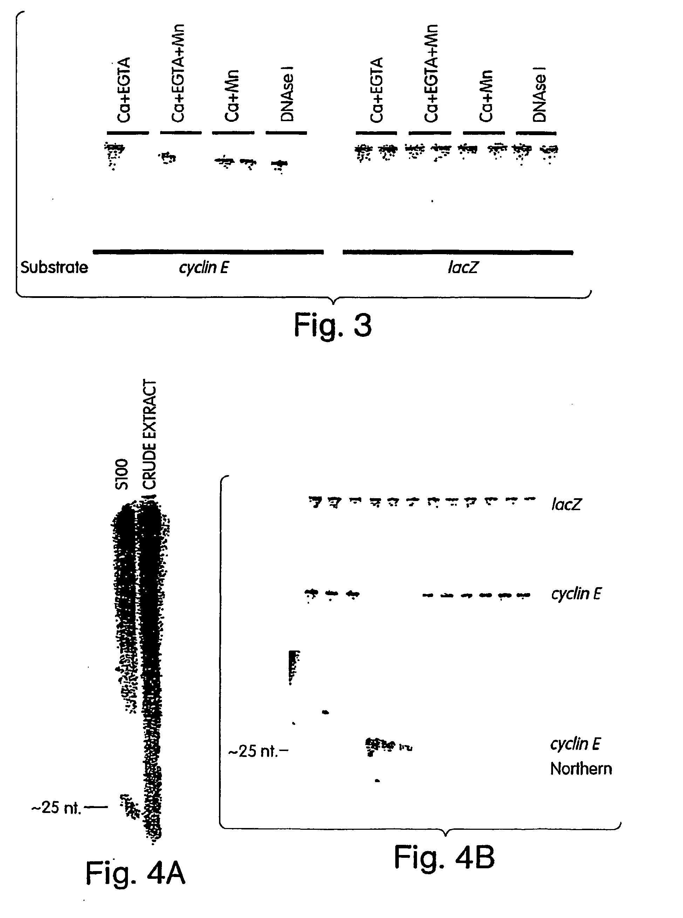

Figure 3: Substrate requirements of the RISC. Extracts were prepared from cells transfected with *cyclin E* dsRNA. Aliquots were incubated for 30 min at 30 °C before the addition of either the *cyclin E* (E600) or *lacZ* (Z800) substrate. Individual 20 µl aliquots, as indicated, were pre-incubated with 1 mM CaCl₂ and 5 mM EGTA, 1 mM CaCl₂, 5 mM EGTA and 60 U of micrococcal nuclease, 1 mM CaCl₂ and 60 U of micrococcal nuclease or 10 U of DNase I (Promega) and 5 mM EGTA. After the 30 min pre-incubation, EGTA was added to those samples that lacked it. Yeast tRNA (1 µg) was added to all samples. Time points were at 0 and 30 min.
Figure 4: The RISC contains a potential guide RNA. (**a**) Northern blots of RNA from either a crude lysate or the S 100 fraction (containing the soluble nuclease activity, see Methods) were hybridized to a riboprobe derived from the sense strand of the *cyclin E* mRNA. (**b**) Soluble *cyclin-E*-specific nuclease activity was fractionated as described in Methods. Fractions from the anion-exchange resin were incubated with the lacZ, control substrate (upper panel) or the cyclin E substrate (centre panel). Lower panel, RNA from each fraction was analysed by northern blotting with a uniformly labelled transcript derived from sense strand of the *cyclin E* cDNA. DNA oligonucleotides were used as size markers.

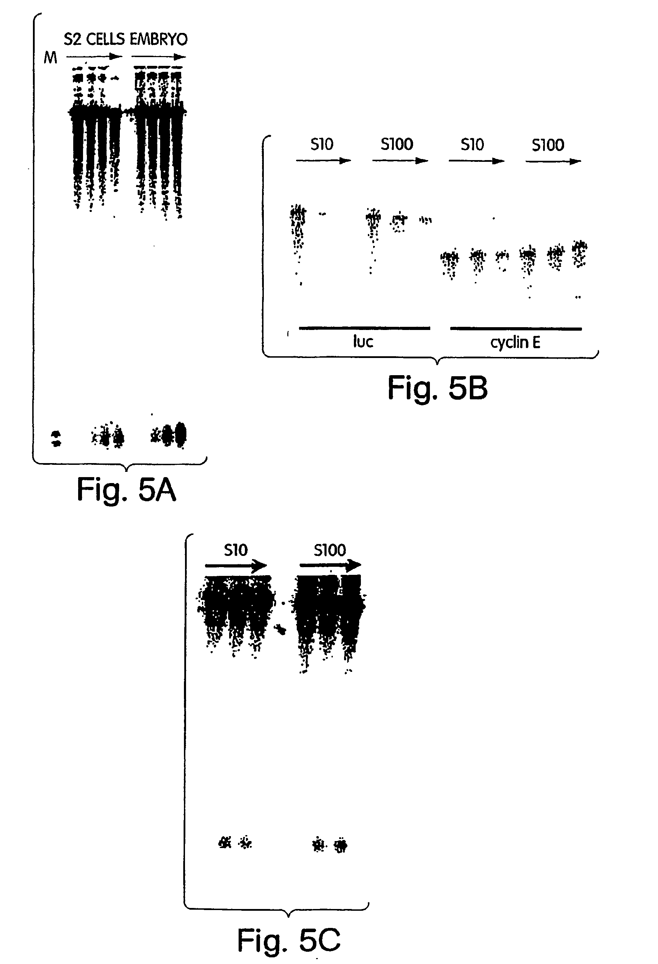

Figure 5: Generation of 22mers and degradation of mRNA are carried out by distinct enzymatic complexes. (**a**) Extracts prepared either from 0-12 hour *Drosophila* embryos or *Drosophila* S2 cells (see Methods) were incubated for 0, 15, 30, or 60 minutes (left to right) with a uniformly-labeled double-stranded RNA corresponding to the first 500 nucleotides of the *Drosophila cyclin E* coding region. M indicates a marker prepared by *in vitro* transcription of a synthetic template. The template was designed to yield a 22 nucleotide transcript. The doublet most probably results from improper initiation at the +1 position. (**b**) Whole-cell extracts were prepared from S2 cells that had been transfected with a dsRNA corresponding to the first 500 nt. of the luciferase coding region. S10 extracts were spun at 30,000xg for 20 minutes which represents our standard RISC extract. S100 extracts were prepared by further centrifugation of S10 extracts for 60 minutes at 10,000xg. Assays for mRNA degradation were carried out as described previously for 0, 30 or 60 minutes (left to right in each set) with either a single-stranded luciferase mRNA or a single-stranded cyclin E mRNA, as indicated. (**c**) S10 or S100 extracts were incubated with cyclin E dsRNAs for 0, 60 or 120 minutes (L to R).

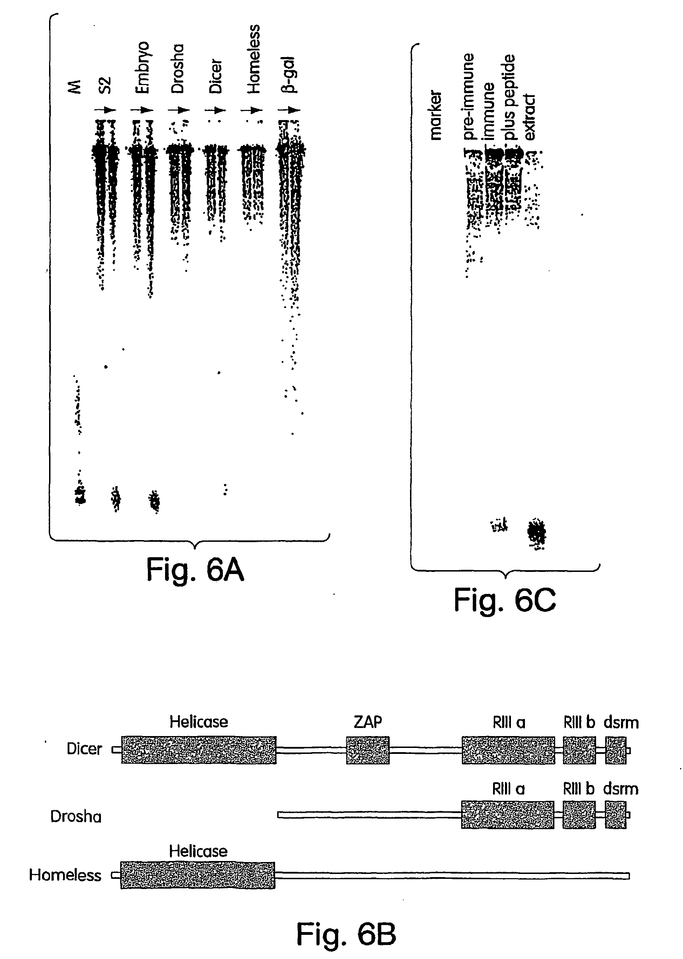

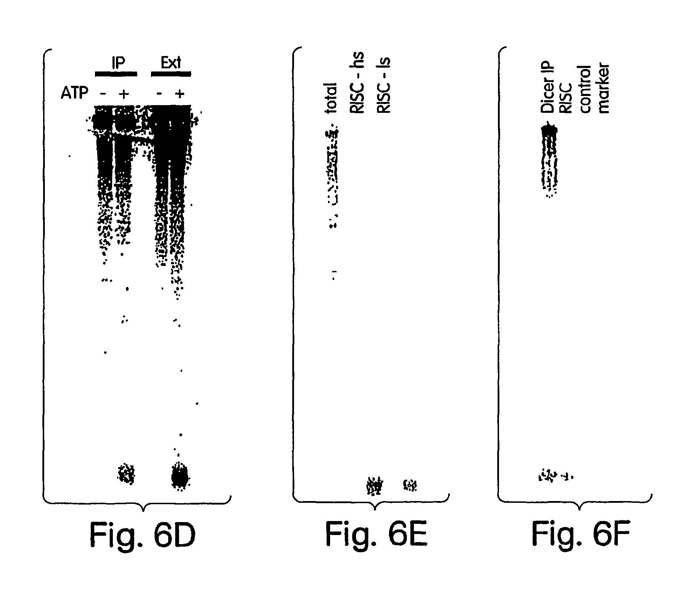

Figure 6: Production of 22mers by recombinant CG4792/Dicer. (**a**) *Drosophila* S2 cells were transfected with plasmids that direct the expression of T7-epitope tagged versions of Drosha, CG4792/Dicer-1 and Homeless. Tagged proteins were purified from cell lysates by immunoprecipitation and were incubated with *cyclin E* dsRNA. For comparison, reactions were also performed in *Drosophila* embryo and S2 cell extracts. As a negative control, immunoprecipitates were prepared from cells transfected with a β-galactosidase expression vector. Pairs of lanes show reactions performed for 0 or 60 minutes. The synthetic marker (**M**) is as described in the legend to Figure 1. (**b**) Diagrammatic representations of the domain structures of CG4792/Dicer-1, Drosha and Homeless are shown. (**c**) Immunoprecipitates were prepared from detergent lysates of S2 cells using an antiserum raised against the C-terminal 8 amino acids of Drosophila Dicer-1 (CG4792). As controls, similar preparations were made with a pre-immune serum and with an immune serum that had been pre-incubated with an excess of antigenic peptide. Cleavage reactions in which each of these precipitates was incubated with an ∼500 nt. fragment of Drosophila cyclin E are shown. For comparsion, an incubation of the substrate in Drosophila embryo extract was electrophoresed in parallel. (**d**) Dicer immunoprecipitates were incubated with dsRNA substrates in the presence or absence of ATP. For comparison, the same substrate was incubated with S2 extracts that either contained added ATP or that were depleted of ATP using glucose and hexokinase (see methods). (**e**) Drosophila S2 cells were transfected with uniformly, ³²P-labelled dsRNA corresponding to the first 500 nt. of GFP. RISC complex was affinity purified using a histidine-tagged version of *Drosophila* Ago-2, a recently identified component of the RISC complex (Hammond et al., in prep). RISC was isolated either under conditions in which it remains ribosome associated (ls, low salt) or under conditions that extract it from the ribosome in a soluble form (hs, high salt). For comparison, the spectrum of labelled RNAs in the total lysate is shown. (**f**) Guide RNAs produced by incubation of dsRNA with a Dicer immunoprecipitate are compared to guide RNAs present in an affinity-purified RISC complex. These precisely comigrate on a gel that has single-nucleotide resolution. The lane labelled control is an affinity selection for RISC from a cell that had been transfected with labeled dsRNA but not with the epitope-tagged *Drosophila* Ago-2.

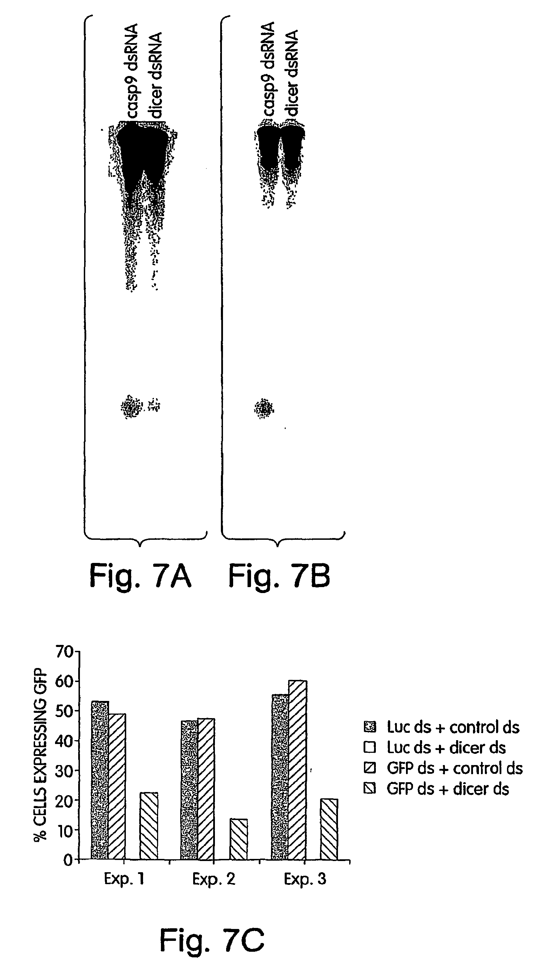

Figure 7: Dicer participates in RNAi. (**a**) Drosophila S2 cells were transfected with dsRNAs corresponding to the two Drosophila Dicers (CG4792 and CG6493) or with a control dsRNA corresponding to murine caspase 9. Cytoplasmic extracts of these cells were tested for Dicer activity. Transfection with Dicer dsRNA reduced activity in lysates by 7.4-fold. (**b**) The Dicer-1 antiserum (CG4792) was used to prepare immunoprecipitates from S2 cells that had been treated as described above. Dicer dsRNA reduced the activity of Dicer-1 in this assay by 6.2-fold. (**c**) Cells that had been transfected two days previously with either mouse caspase 9 dsRNA or with Dicer dsRNA were cotransfected with a GFP expression plasmid and either control, luciferase dsRNA or GFP dsRNA. Three independent experiments were quantified by FACS. A comparison of the relative percentage of GFP-positive cells is shown for control (GFP plasmid plus luciferase dsRNA) or silenced (GFP plamsid plus GFP dsRNA) populations in cells that had previously been transfected with either control (caspase 9) or Dicer dsRNAs.

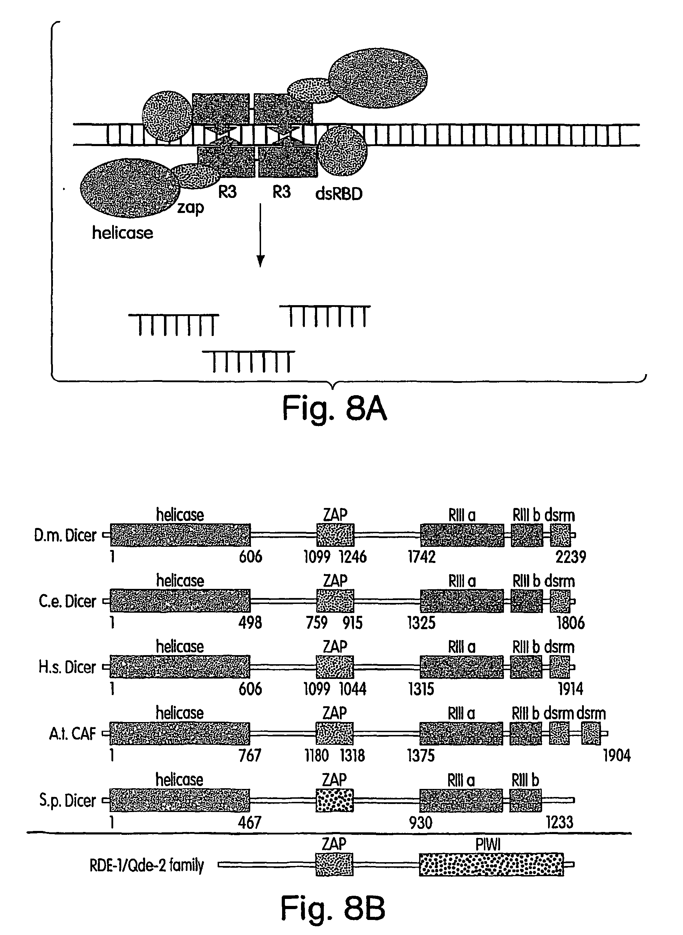

Figure 8: Dicer is an evolutionarily conserved ribonuclease. (**a**) A model for production of 22mers by Dicer. Based upon the proposed mechanism of action of Ribonuclease III, we propose that Dicer acts on its substrate as a dimer. The positioning of the two ribonuclease domains (RIIIa and RIIIb) within the enzyme would thus determine the size of the cleavage product. An equally plausible alternative model could be derived in which the RIIIa and RIIIb domains of each Dicer enzyme would cleave in concert at a single position. In this model, the size of the cleavage product would be determined by interaction between two neighboring Dicer enzymes. (**b**) Comparison of the domain structures of potential Dicer homologs in various organisms (*Drosophila -* CG4792, CG6493, *C*. *elegans -* K12H4.8, *Arabidopsis -* CARPEL FACTORY, T25K16.4, AC012328_1, human Helicase-MOI and *S*. *pombe -* YC9A_SCHPO). The ZAP domains were identified both by analysis of individual sequences with Pfam and by Psi-blast searches. The ZAP domain in the putative *S*. *pombe* Dicer is not detected by PFAM but is identified by Psi-Blast and is thus shown in a different color. For comparison, a domain structure of the RDE1/QDE2/ARGONAUTE family is shown. It should be noted that the ZAP domains are more similar within each of the Dicer and ARGONAUTE families than they are between the two groups. (**c**) An alignment of the ZAP domains in selected Dicer and Argonaute family members is shown. The alignment was produced using ClustalW.

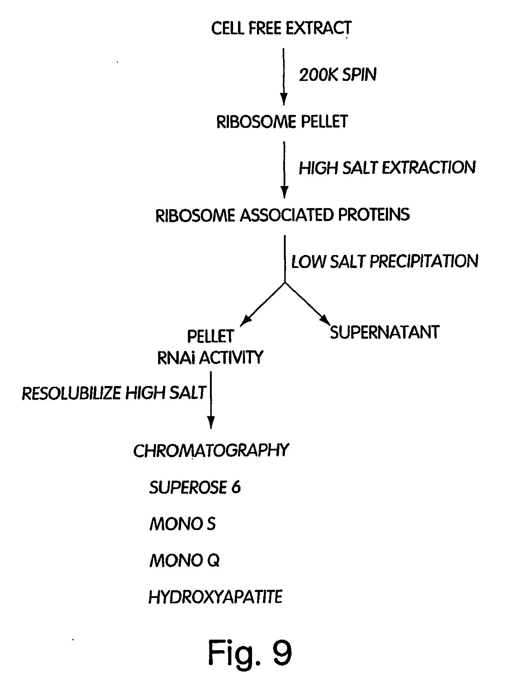

Figure 9: Purification strategy for RISC. (second step in RNAi model).

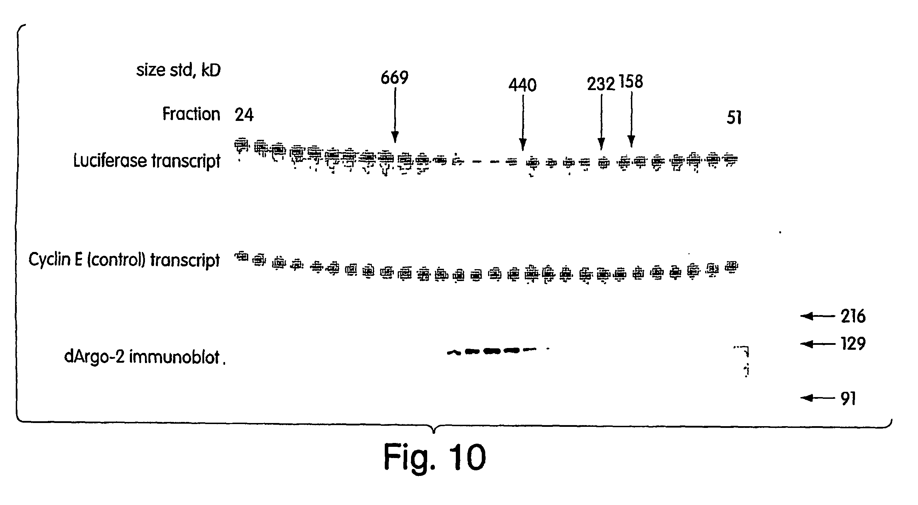

Figure 10: Fractionation of RISC activity over sizing column. Activity fractionates as 500 KDa complex. Also, antibody to *Drosophila* argonaute 2 cofractionates with activity.

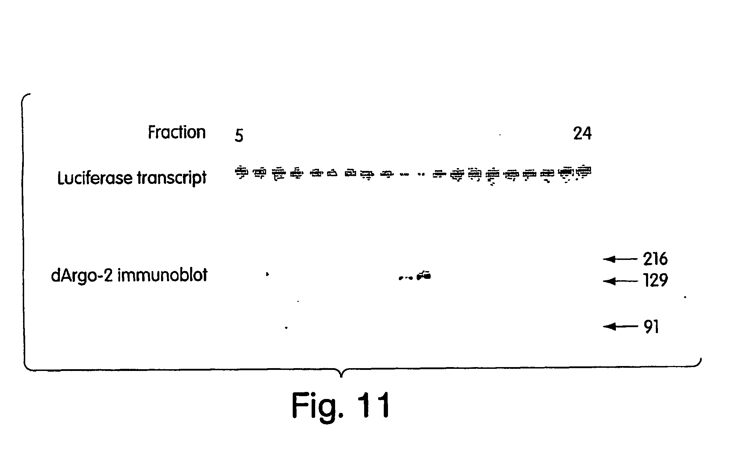

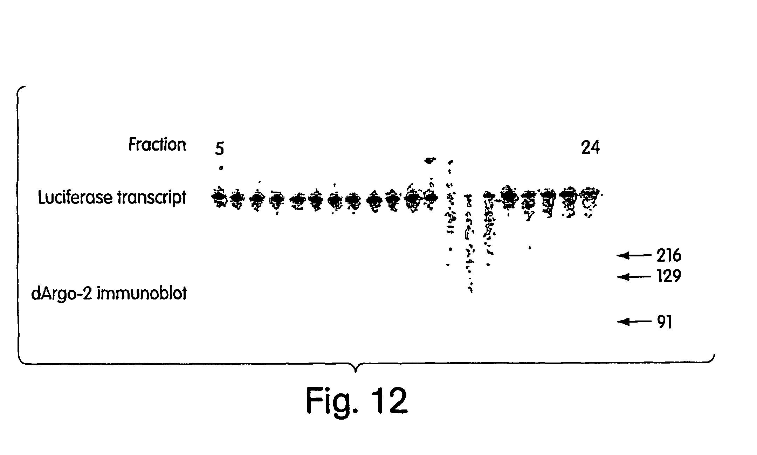

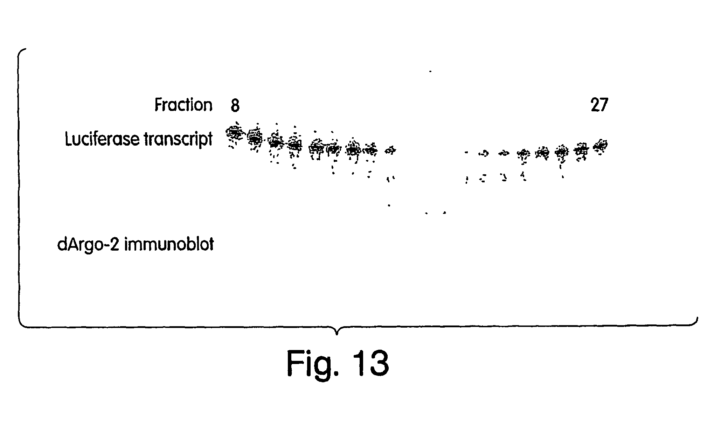

Figures 11-13: Fractionation of RISC over monoS, monoQ, Hydroxyapatite columns. *Drosophila* argonaute 2 protein also cofactionates.

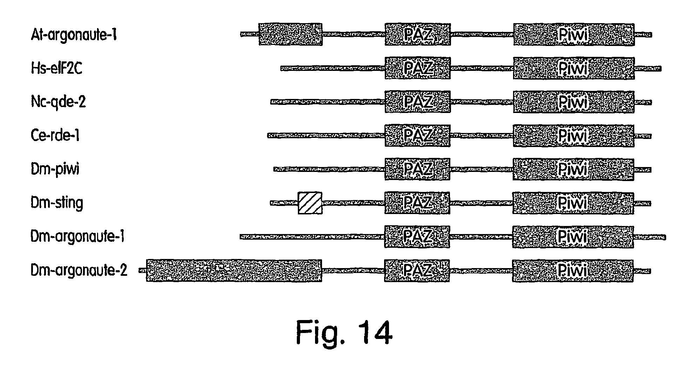

Figure 14: Alignment of *Drosophila* argonaute 2 with other family members.

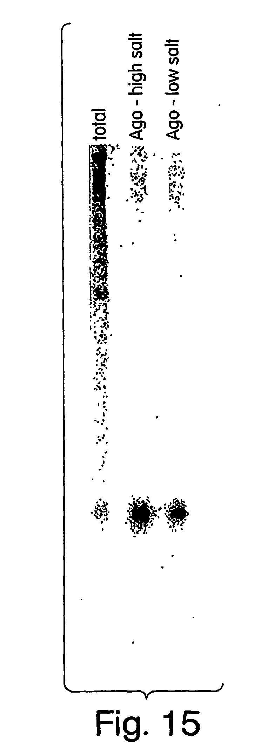

Figure 15: Confirmation of *Drosophila* argonaute 2. S2 cells were transfected with labeled dsRNA and His tagged argonaute. Argonaute was isolated on nickel agarose and RNA component was identified on 15% acrylamide gel.

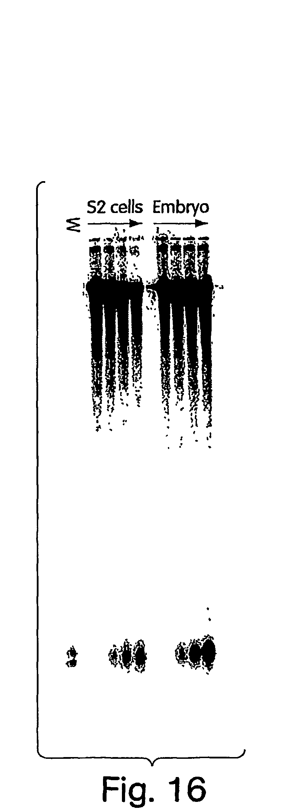

Figure 16: S2 cell and embryo extracts were assayed for 22mer generating activity.

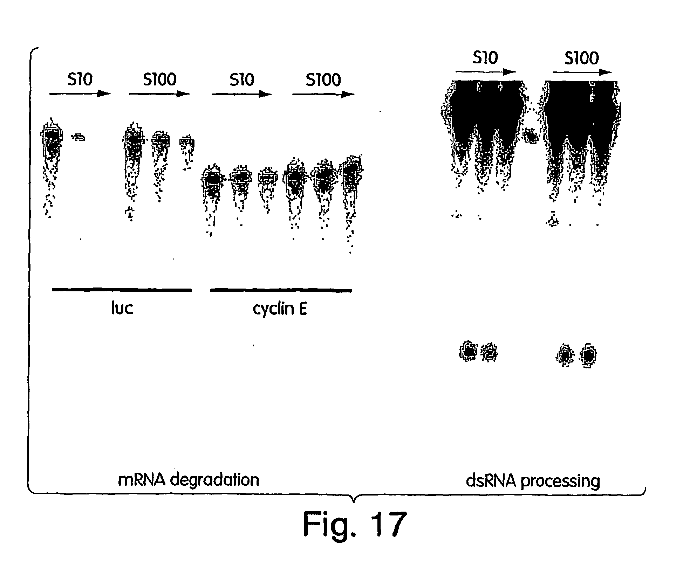

Figure 17: RISC can be separated from 22mer generating activity (dicer). Spinning extracts (S100) can clear RISC activity from supernatant (left panel) however, S100 spins still contain dicer activity (right panel).

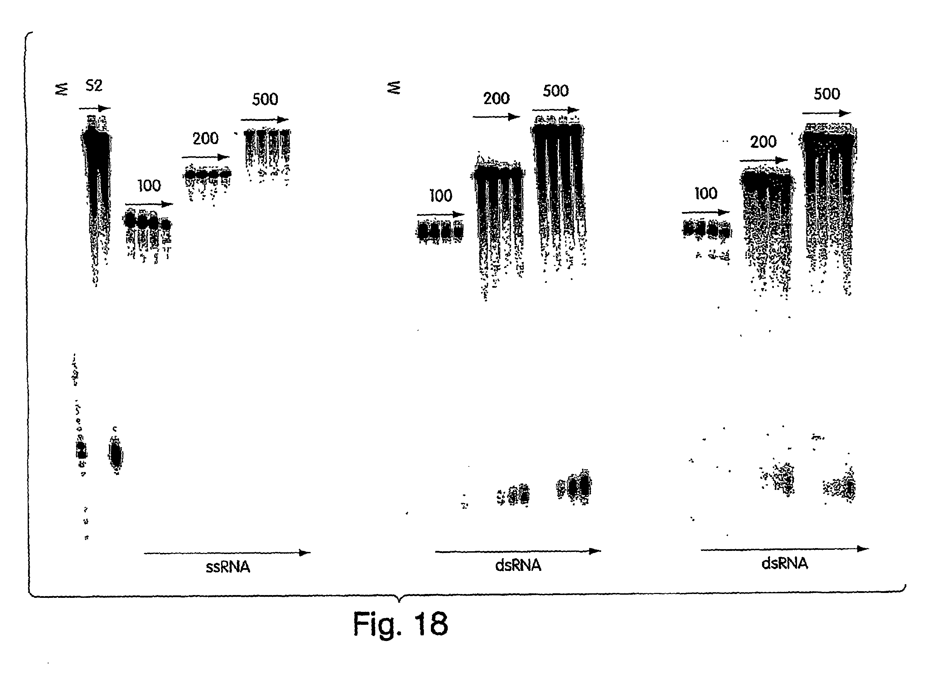

Figure 18: Dicer is specific for dsRNA and prefers longer substrates.

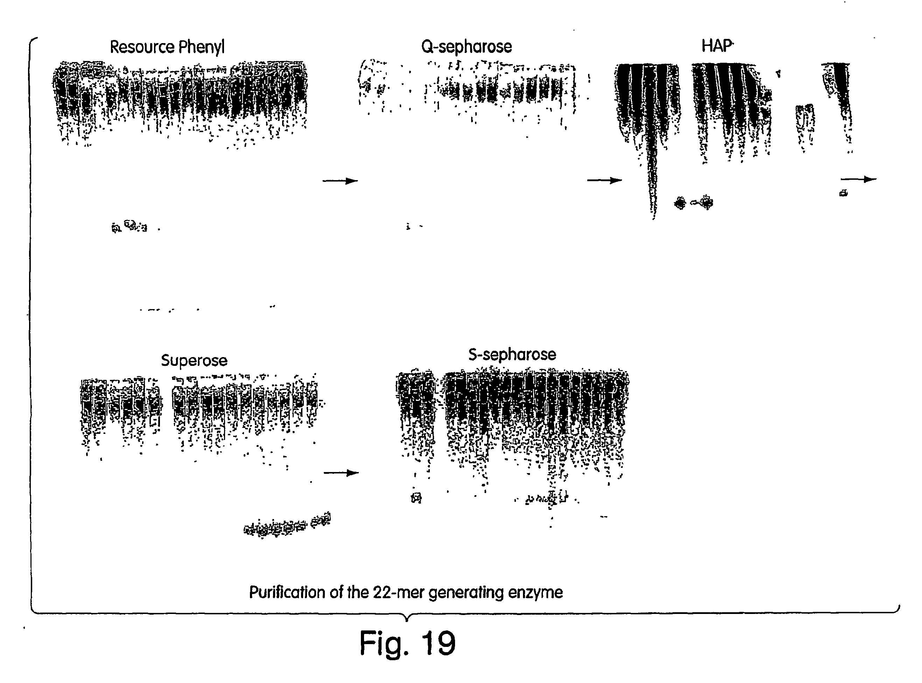

Figure 19: Dicer was fractionated over several columns.

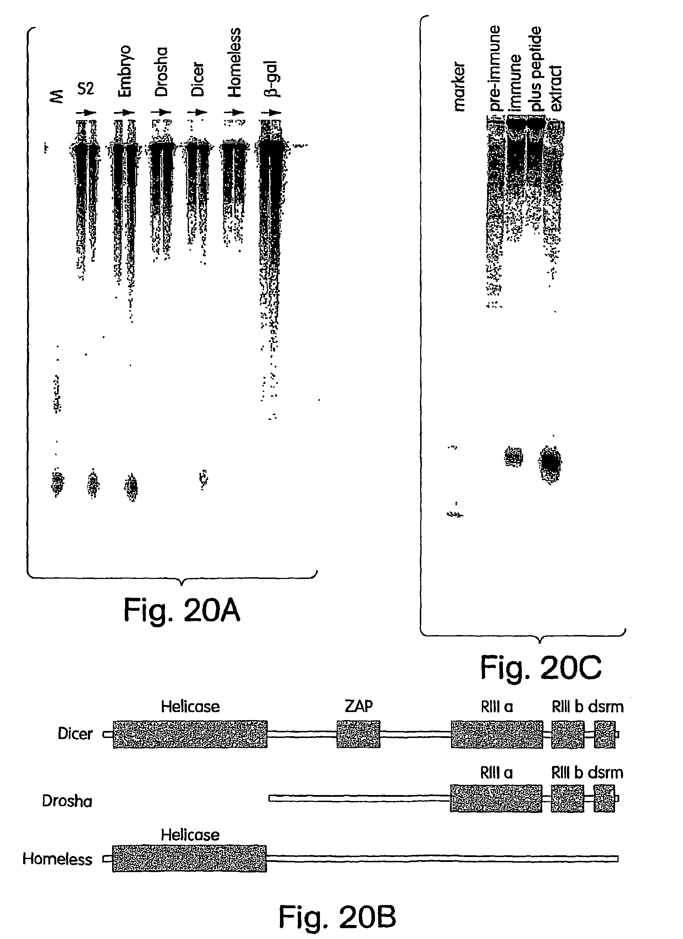

Figure 20: Identification of dicer as enzyme which can process dsRNA into 22mers. Various RNaseIII family members were expressed with n terminal tags, immunoprecipitated, and assayed for 22mer generating activity ( left panel). In right panel, antibodies to dicer could also precipitate 22mer generating activity.

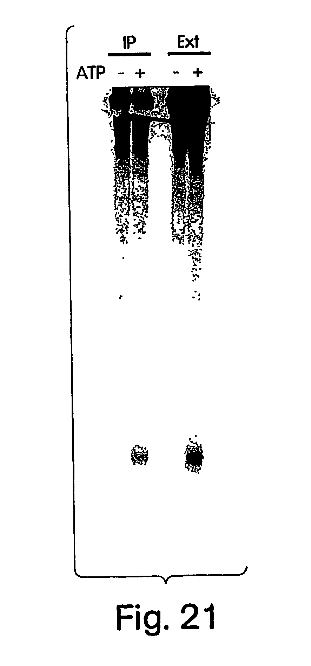

Figure 21: Dicer requires ATP.

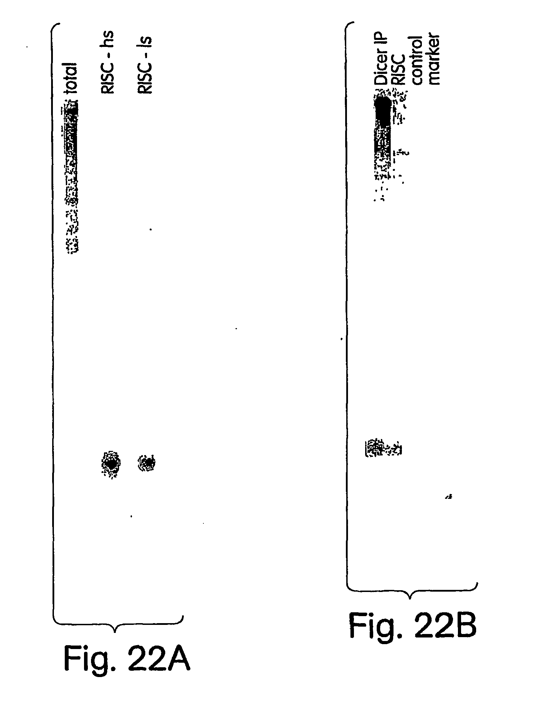

Figure 22: Dicer produces RNAs that are the same size as RNAs present in RISC.

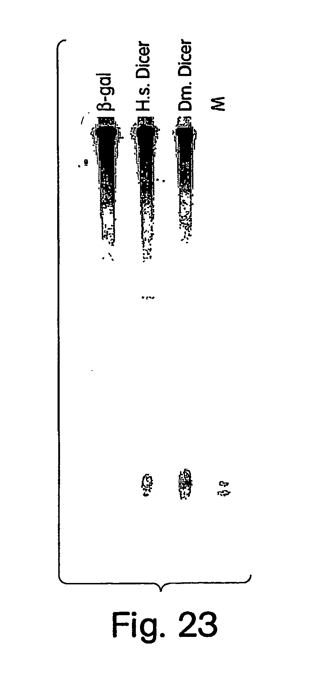

Figure 23: Human dicer homolog when expressed and immunoprecipitated has 22mer generating activity.
Figure 24: Sequence of *Drosophila* argonaute 2. Peptides identified by microsequencing are shown in underline.

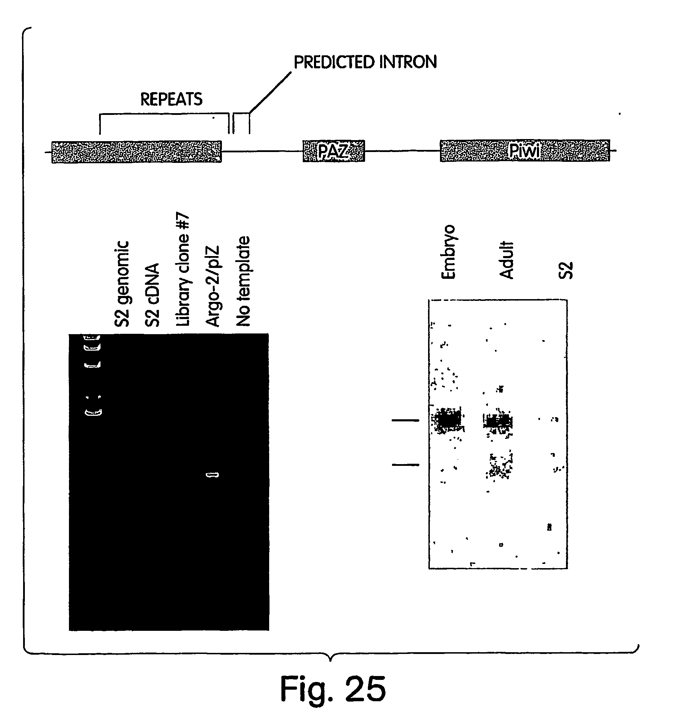

Figure 25: Molecular charaterization of *Drosophila* argonaute 2. The presence of an intron in coding sequence was determined by northern blotting using intron probe. This results in a different 5' reading frame then the published genome seqeunce. Number of polyglutamine repeats was determined by genomic PCR.

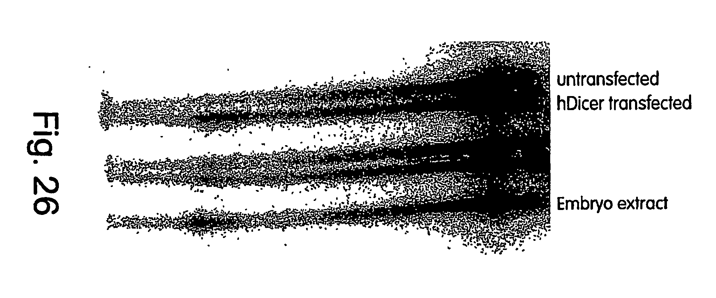

Figure 26: Dicer activity can be created in human cells by expression of human dicer gene. Host cell was 293. Crude extracts had dicer activity, while activity was absent from untransfected cells. Activity is not dissimilar to that seen in drosophila embryo extracts.

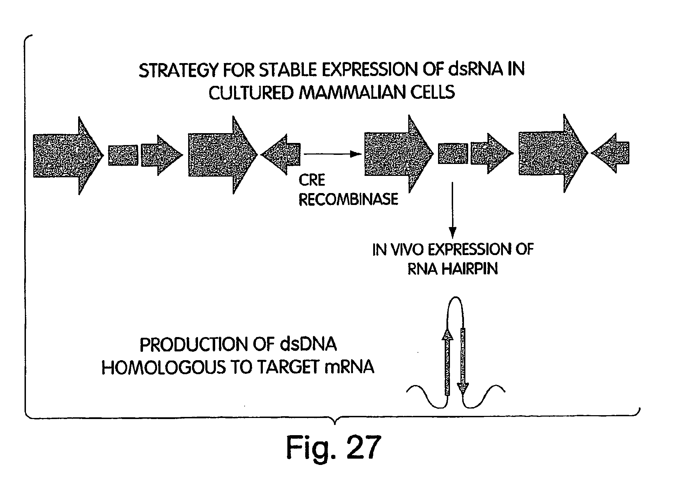

Figure 27: A ∼500 nt. fragment of the gene that is to be silenced (X) is inserted into the modified vector as a stable direct repeat using standard cloning procedures. Treatment with commercially available cre recombinase reverses sequences within the IoxP sites (L) to create an inverted repeat. This can be stably maintained and amplified in an sbc mutant bacterial strain (DL759). Transcription in vivo from the promoter of choice (P) yields a hairpin RNA that causes silencing. A zeocin resistance marker is included to insure maintenance of the direct and inverted repeat structures; however this is non-essential in vivo and could be removed by pre-mRNA splicing if desired. (Smith et al. (2000) Nature 407: 319-20).

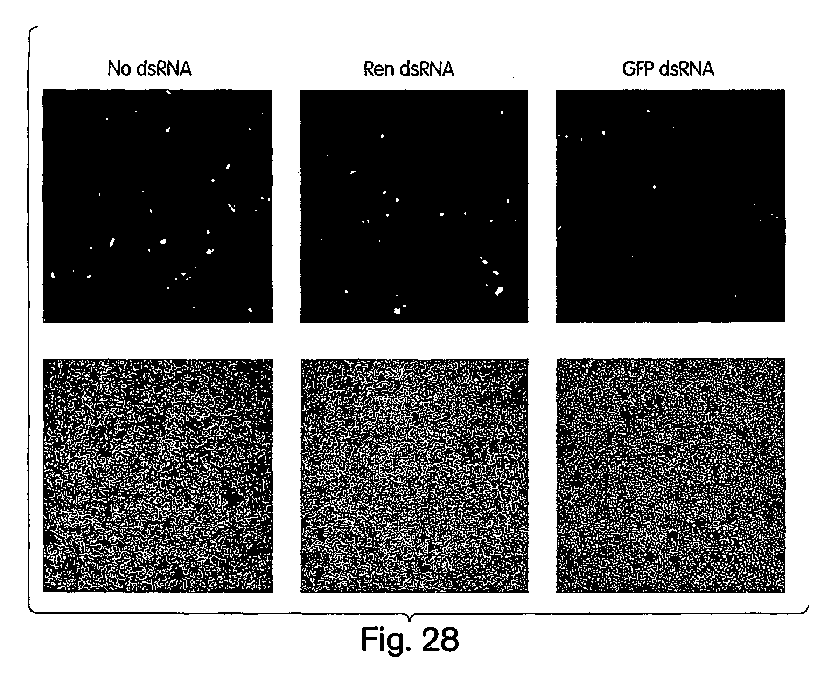

Figure 28: RNAi in P19 embryonal carcinoma cells. Ten-centimeter plates of P 19 cells were transfected by using 5 µg of GFP plasmid and 40 µg of the indicated dsRNA (or no RNA). Cells were photographed by fluorescent (tope panel) and phase-contrast microscopy (bottom panel) at 72 h after transfection; silencing was also clearly evident at 48 h posttransfection.

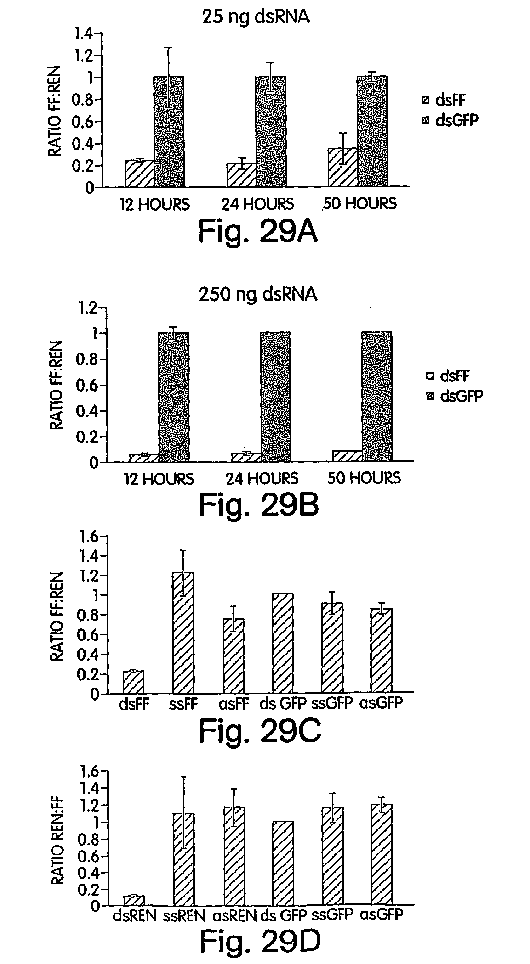

Figure 29: RNAi of firefly and *Renilla* luciferase in P19 cells. (**A** and **B**) P19 cells were transfected with plasmids that direct the expression of firefly and *Renilla* luciferase and dsRNA 500 mers (25 or 250 ng, as indicated in A and B, respectively), that were either homologous to the firefly luciferase mRNA (dsFF) or nonhomologous (dsGFP). Luciferase activities were assayed at various times after transfection, as indicated. Ratios of firefly to *Renilia* activity are normalized to dsGFP controls. (**C** and **D**) P19 cells in 12-well culture dishes (2 ml of media) were transfected with 0.25 µg of a 9:1 mix of pGL3-Control and pRL-SV40 as well as 2 µg of the indicated RNA. Extracts were prepared 9 h after transfection. (**C**) Ratio of firefly to *Renilla* luciferase is shown. (**D**) Ratio of *Renilia* to firefly luciferase is shown. Values are normalized to dsGFP. The average of three independent experiments is shown; error bars indicate standard deviation.

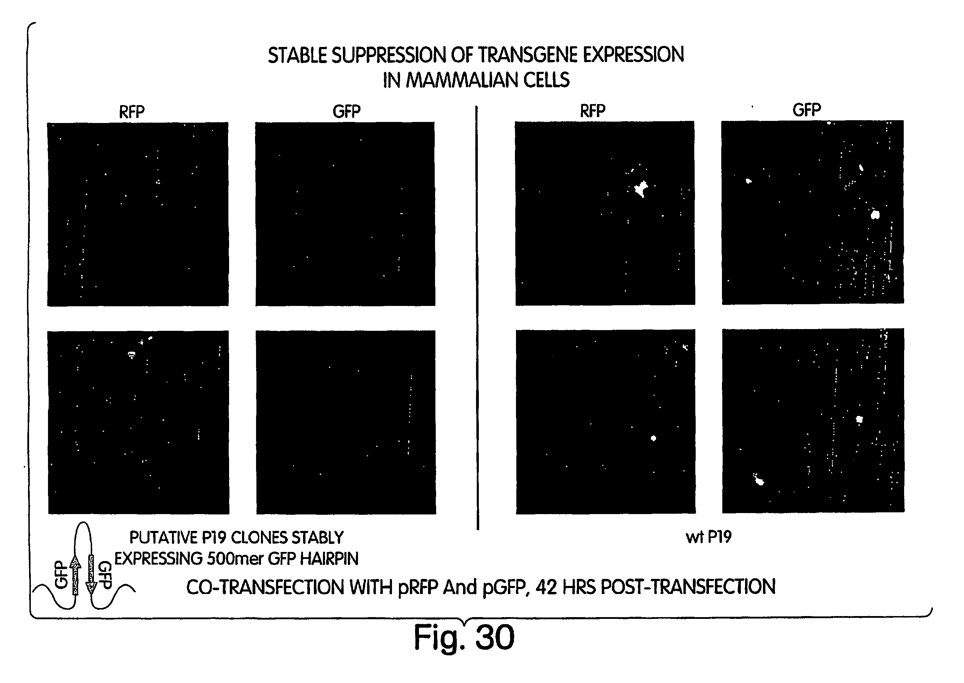

Figure 30: The panels at the right show expression of either RFP or GFP following transient transfection into wild type P19 cells. The panels at the left demonstrate the specific suppression of GFP expression in P 19 clones which stably express a 500 nt double stranded GFP hairpin. P19 clones which stably express the double stranded GFP hairpin were transiently transfected with RFP or GFP, and expression of RFP or GFP was assessed by visual inspection.

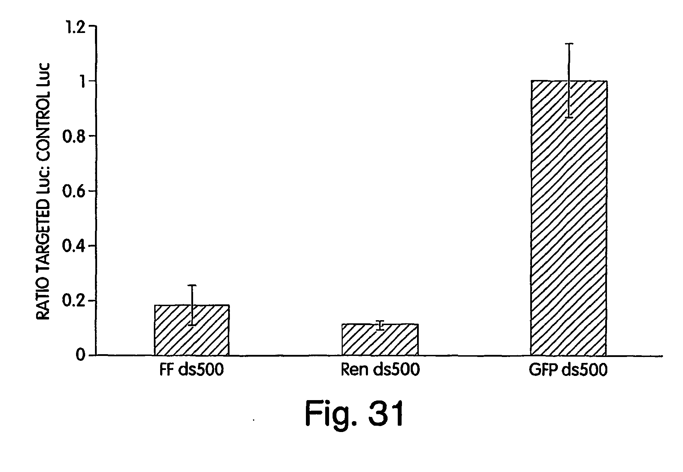

Figure 31: Specific silencing of luciferase expression by dsRNA in murine embryonic stem cells. Mouse embryonic stem cells in 12-well culture dishes (1 ml of media) were transfected with 1.5 µg of dsRNA along with 0.25 µg of a 10:1 mixture of the reporter plasmids pGL3-Control and pRL-SV40. Extracts were prepared and assayed 20 h after transfection. The ratio of firefly to *Renilla* luciferase expression is shown for FF ds500; the ratio of *Renilla* to firefly is shown for Ren ds500. Both are normalized to ratios from the dsGFP transfection. The average of three independent experiments is shown; error bars indicate standard deviation.

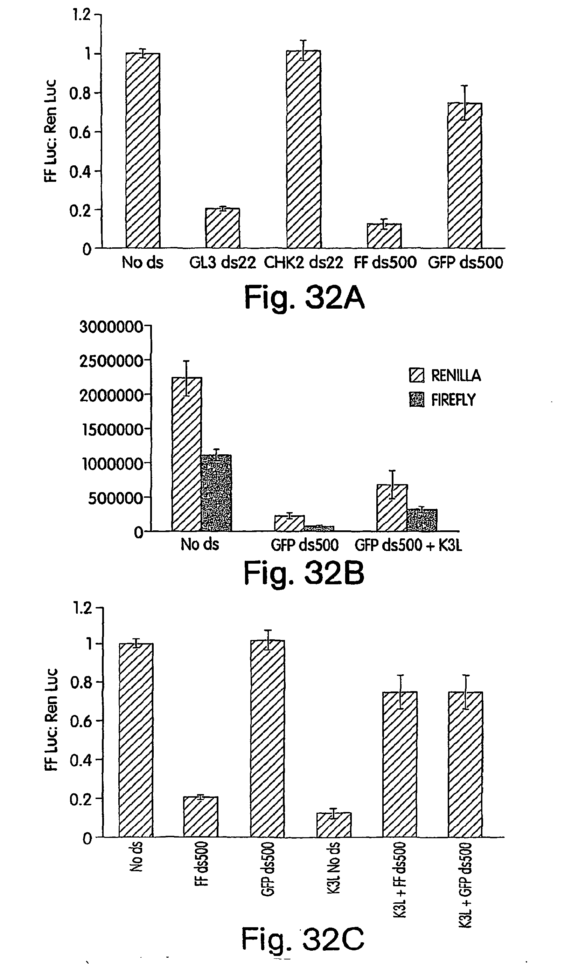

Figure 32: RNAi in C2C 12 murine myoblast cells. (**A**) Mouse C2C12 cells in 12-well culture dishes (1 ml of media) were transfected with 1 µg of the indicated dsRNA along with 0.250 µg of the reporter plasmids pGL3-Control and pRL-SV40. Extracts were prepared and assayed 24 h after transfection. The ratio of firefly to *Renilla* luciferase expression is shown; values are normalized to ratios from the no dsRNA control. The average of three independent experiments is shown; error bars indicate standard deviation. (**B**) C2C12 cells cotransfected with 1 µg of either plasmid alone or a plasmid containing a hyperactive mutant of vaccinia virus K3L (Kawagishi-Kobayashi et al. 2000, Virology 276: 424-434). The absolute counts of *Renilla* and firefly luciferase activity are shown. (**C**) The ratios of firefly/*Renilla* activity from **B**, normalized to no dsRNA controls.

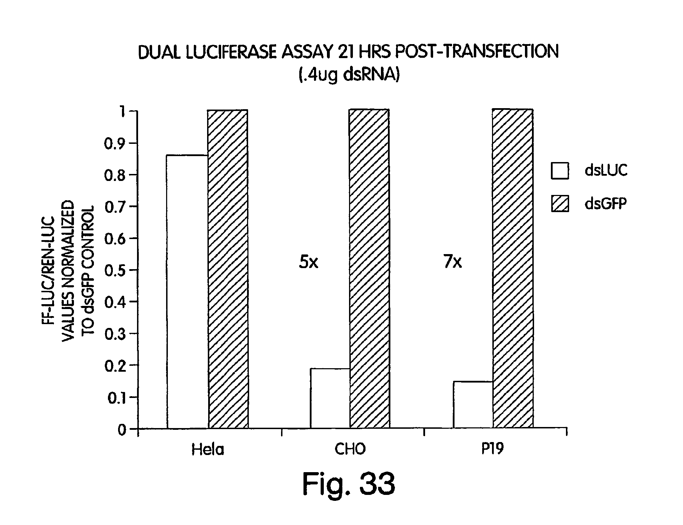

Figure 33: Hela, Chinese hamster ovary, and P19 (pluripotent, mouse embryonic carcinoma) cell lines transfected with plasmids expressing Photinus pyralis (firefly) and Renilla reniformis (sea pansy) luciferases and with dsRNA 500mers (400ng), homologous to either firefly luciferase mRNA (dsLUC) or non-homologous (dsGFP). Dual luciferase assays were carried out using an Analytical Scientific Instruments model 3010 Luminometer. In this assay Renilla luciferase serves as an internal control for dsRNA-specific suppression of firefly luciferase activity. These data demonstrate that 500mer dsRNA can specifically suppress cognate gene expression in vivo.

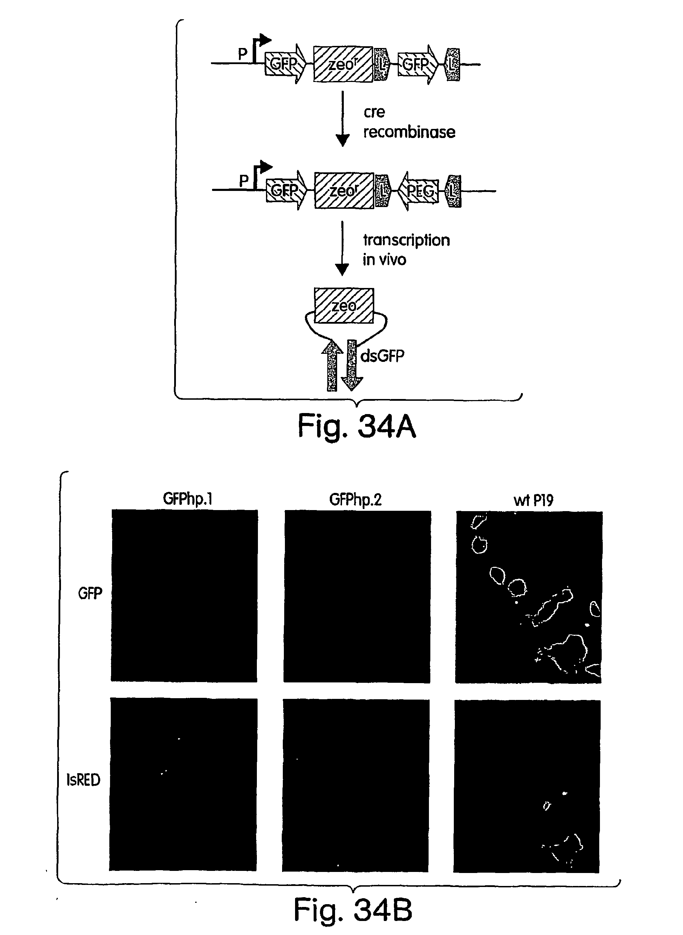

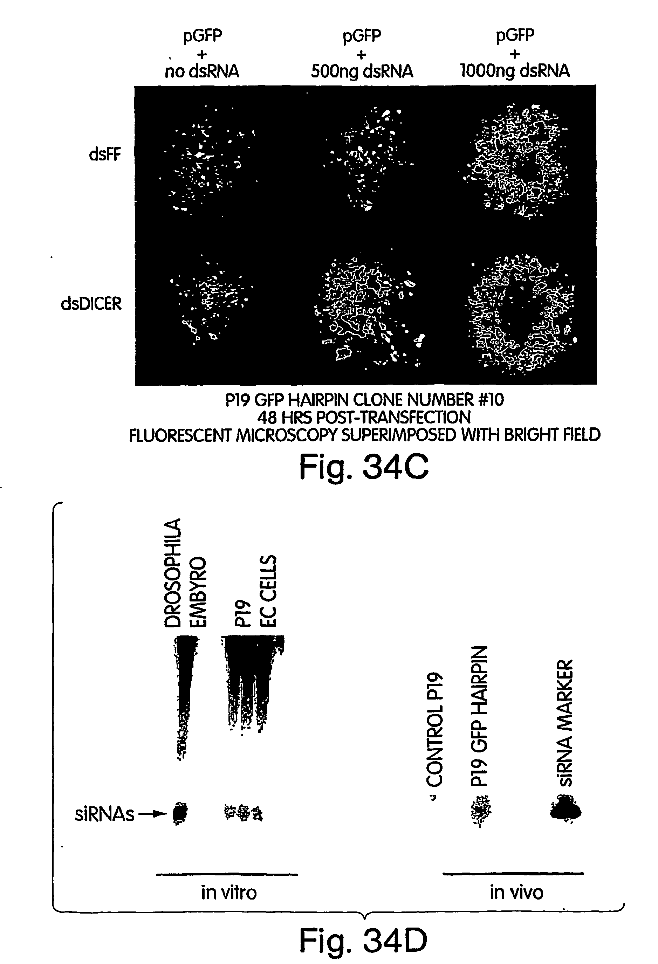

Figure 34: Expression of a hairpin RNA produces P 19 EC cell lines that stably silence GFP. (**A**) A cartoon of the FLIP cassette used to construct the GFP hairpin. GFP represents the first 500 coding base pairs of EGFP. Zeo, zeocin resistance gene; L, Lox; P, the cytomegalovirus promoter in the expression plasmid pcDNA3. Homologous GFP fragments are first cloned as direct repeats into the FLIP cassette. To create inverted repeats for hairpin production, the second repeat is flipped by using Cre recombinase. When transcribed, the inverted repeat forms a GFP dsRNA with a hairpin loop. (**B**) P19 cell lines stably expressing the GFP hairpin plasmid, GFPhp.1 (clone 10) and GFPhp.2 (clone 12), along with wt P19 were transfected with 0.25 µg each of GFP and RFP reporter genes. Fluorescence micrographs were taken by using filters appropriate for GFP and RFP. Magnification is 200×. (**C**) P 19 GFPhp.1 cells were transfected with pEGFP and 0, 0.5, or 1 µg of Dicer or firefly dsRNA. Fluorescence micrographs were taken at 48 h posttransfection and are superimposed with bright field images to reveal non-GFP expressing cells. Magnification is 100×. (**D**) *In vitro* and *in vivo* processing of dsRNA in P19 cells. *In vitro* Dicer assays were performed on S2 cells and three independently prepared P19 extracts by using ³²P-labeled dsRNA (30 °C for 30 min). A Northern blot of RNA extracted from control and GFPhp.1 P19 cells shows the production of ≈22mer RNA species in hairpin-expressing cells but not in control cells. Blots were probed with a ³²P-labeled "sense" GFP transcript.

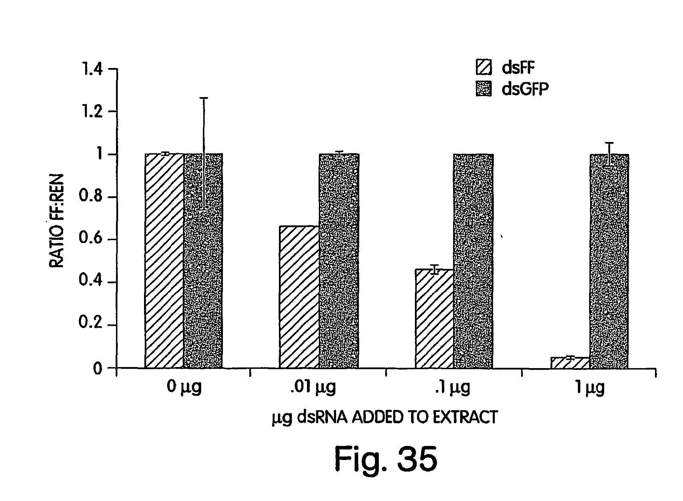

Figure 35: dsRNA induces silencing at the posttranscriptional level. P19 cell extracts were used for *in vitro* translation of firefly and *Renilla* luciferase mRNA (100 ng each). Translation reactions were programmed with various amounts of dsRNA 500mers, either homologous to firefly luciferase mRNA (dsLUC) or nonhomologous (dsGFP). Luciferase assays were carried out after a 1 h incubation at 30 °C. Ratios of firefly to *Renilla* activity are normalized to no dsRNA controls. Standard deviations from the mean are shown.

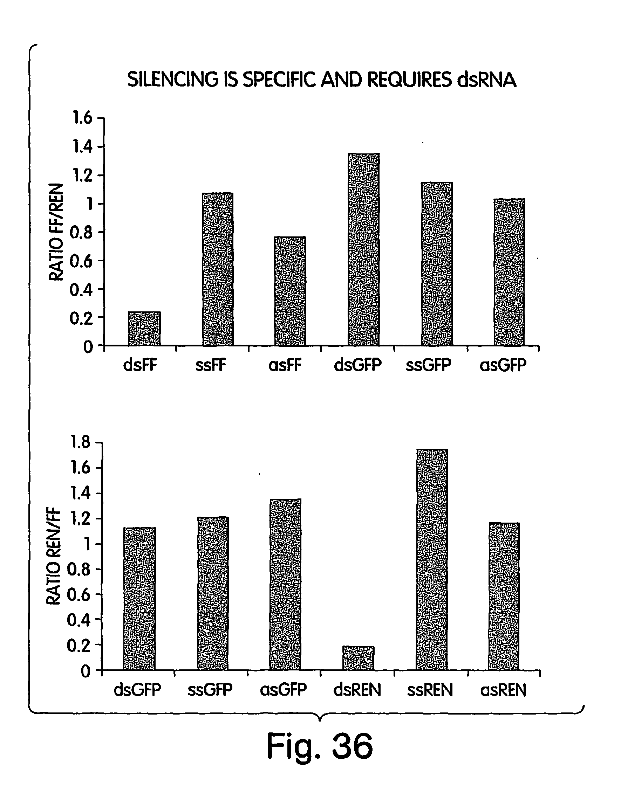

Figure 36: S10 fractions from P19 cell lysates were used for in vitro translations of mRNA coding for Photinus pyralis (firefly) and Renilla reniformis (sea pansy) luciferases. Translation reactions were programmed with dsRNA, ssRNA, or asRNA 500mers, either complementary to firefly luciferase mRNA (dsFF, ssFF, or asFF), complementary to Renilla luciferase (dsREN, ssREN, or asREN) or non-complementary (dsGFP). Reactions were carried out at 30 °C for 1 hour, after a 30 min preincubation with dsRNA, ssRNA,or asRNA. Dual luciferase assays were carried out using an Analytical Scientific Instruments model 3010 Luminometer. On the left, Renilla luciferase serves as an internal control for dsRNA-specific suppression of firefly luciferase activity. On the right, firefly luciferase serves as an internal control for dsRNA-specific suppression of Renilla luciferase activity. These data demonstrate that 500mer double-stranded RNA (dsRNA) but not single-stranded (ssRNA) or anti-sense RNA (asRNA) suppresses cognate gene expression in vitro in a manner consistent with post-transcriptional gene silencing.

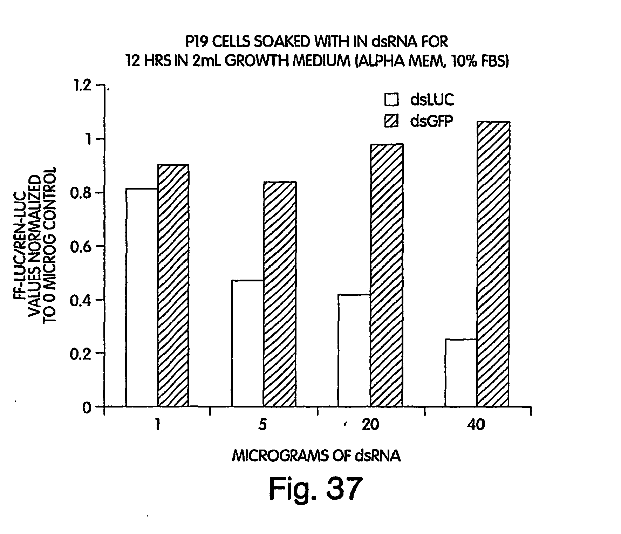

Figure 37: P19 cells were grown in 6-well tissue culture plates to approximately 60% confluence. Various amounts of dsRNA, either homologous to firefly luciferase mRNA (dsLUC) or non-homologous (dsGFP), were added to each well and incubated for 12hrs under normal tissue culture conditions. Cells were then transfected with plasmids expressing Photinus pyralis (firefly) and Renilla reniformis (sea pansy) luciferases and with dsRNA 500mers (500ng). Dual luciferase assays were carried out 12 hrs post-transfection using an Analytical Scientific Instruments model 3010 Luminometer. In this assay Renilla luciferase serves as an internal control for dsRNA-specific suppression of firefly luciferase activity. These data show that 500mer dsRNA can specifically suppress cognate gene expression in vivo without transfection under normal tissue culture conditions.

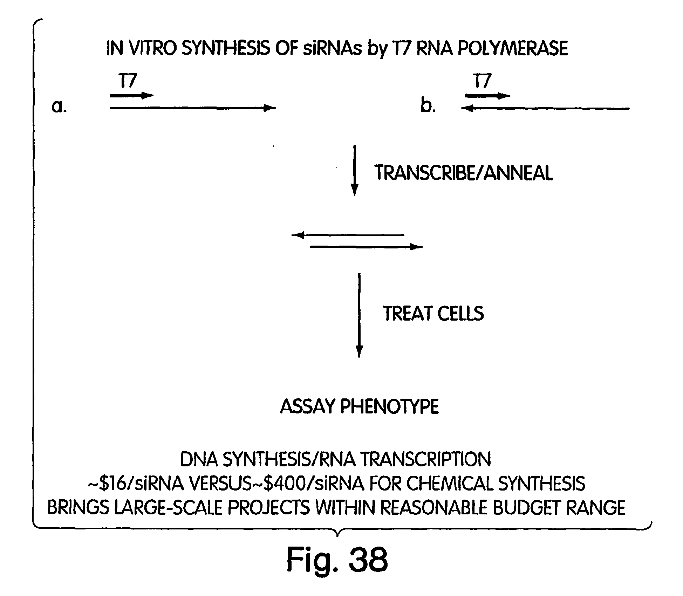

Figure 38: Previous methods for generating siRNAs required costly chemical synthesis. The invention provides an in vitro method for synthesizing siRNAs using standard RNA transcription reactions.

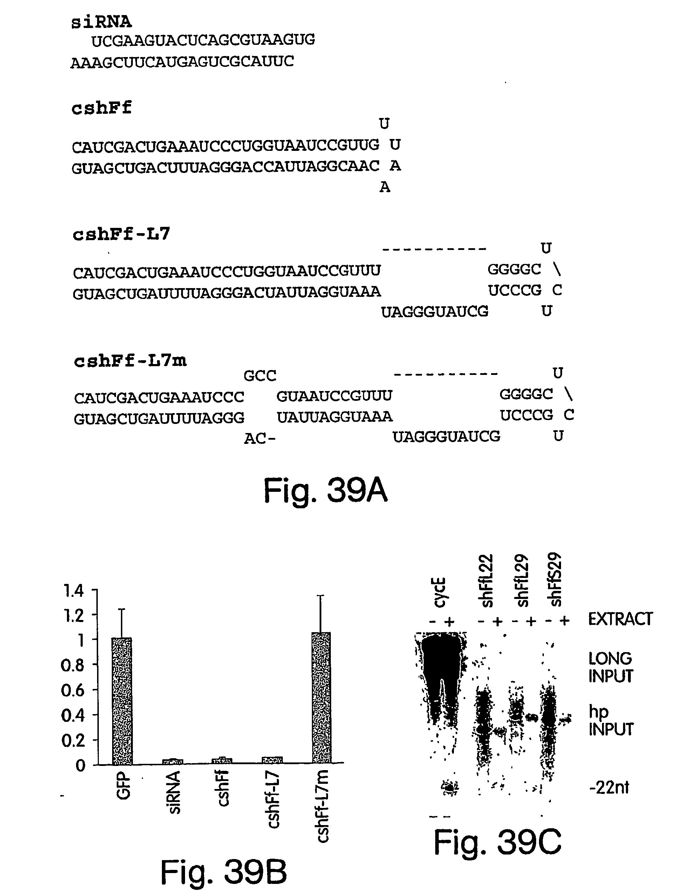

Figure 39: Short hairpins suppress gene expression in *Drosophila* S2 cells. (**A**) Sequences and predicted secondary structure of representative chemically synthesized RNAs. Sequences correspond to positions 112-134 (siRNA) and 463-491 (shRNAs) of Firefly luciferase carried on pGL3-Control. An siRNA targeted to position 463-485 of the luciferase sequence was virtually identical to the 112-134 siRNA in suppressing expression, but is not shown. (**B**) Exogenously supplied short hairpins suppress expression of the targeted Firefly luciferase gene in vivo. Six-well plates of S2 cells were transfected with 250 ng/well of plasmids that direct the expression of firefly and *Renilla* luciferase and 500 ng/well of the indicated RNA. Luciferase activities were assayed 48 h after transfection. Ratios of firefly to *Renilla* luciferase activity were normalized to a control transfected with an siRNA directed at the green fluorescent protein (GFP). The average of three independent experiments is shown; error bars indicate standard deviation. (**C**) Short hairpins are processed by the *Drosophila* Dicer enzyme. T7 transcribed hairpins shFfL22, shFfL29, and shFfS29 were incubated with (+) and without (-) 0-2-h *Drosophila* embryo extracts. Those incubated with extract produced ∼22-nt siRNAs, consistent with the ability of these hairpins to induce RNA interference. A long dsRNA input (cyclin E 500-mer) was used as a control. Cleavage reactions were performed as described in Bernstein et al., 2001, Nature, 409:363-366.

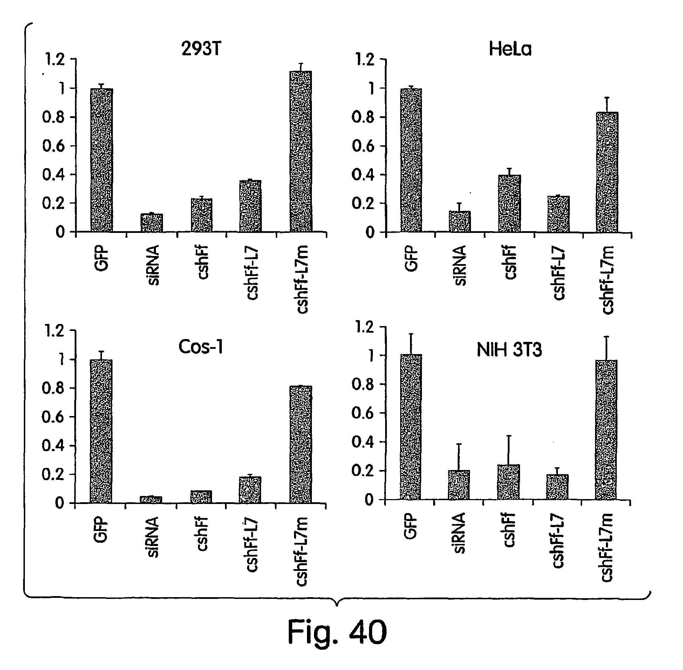

Figure 40: Short hairpins function in mammalian cells. HEK 293T, HeLa, COS-1, and NIH 3T3 cells were transfected with plasmids and RNAs as in Figure 1 and subjected to dual luciferase assays 48 h posmansfection. The ratios of firefly to *Renilla* luciferase activity are normalized to a control transfected with an siRNA directed at the green fluorescent protein (GFP). The average of three independent experiments is shown; error bars indicate standard deviation.

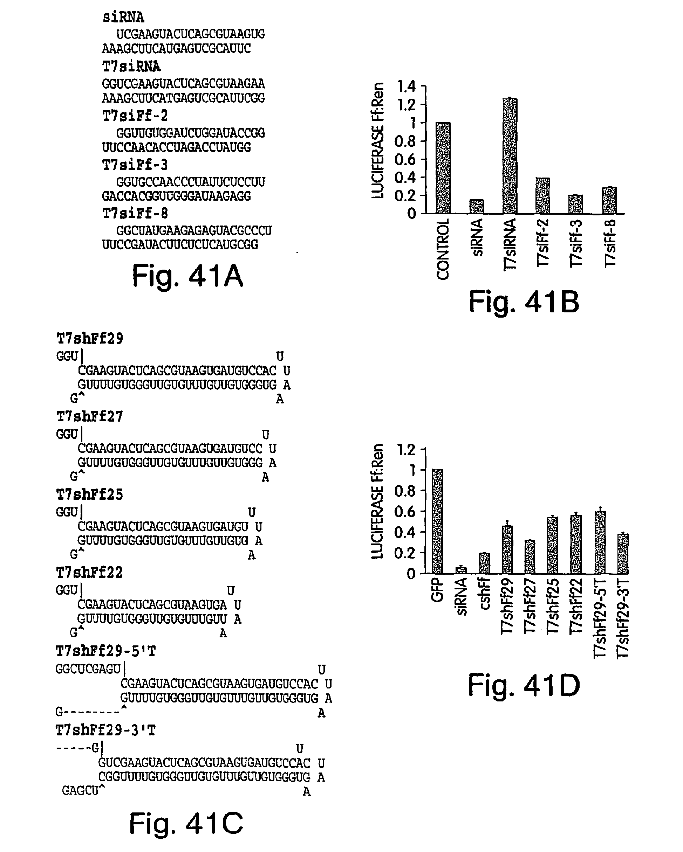

Figure 41: siRNAs and short hairpins transcribed in vitro suppress gene expression in mammalian cells. (**A**) Sequences and predicted secondary structure of representative in vitro transcribed siRNAs. Sequences correspond to positions 112-134 (siRNA) and 463-491 (shRNAs) of firefly luciferase carried on pGL3-Control. (**B**) In vitro transcribed siRNAs suppress expression of the targeted firefly luciferase gene in vivo. HEK 293T cells were transfected with plasmids as in Figure 2. The presence of non-base-paired guanosine residues at the 5' end of siRNAs significantly alters the predicted end structure and abolishes siRNA activity. (**C**) Sequences and predicted secondary structure of representative in vitro transcribed shRNAs. Sequences correspond to positions 112-141 of firefly luciferase carried on pGL3-Control. (**D**) Short hairpins transcribed in vitro suppress expression of the targeted firefly luciferase gene in vivo. HEK 293T cells were transfected with plasmids as in Figure 2.

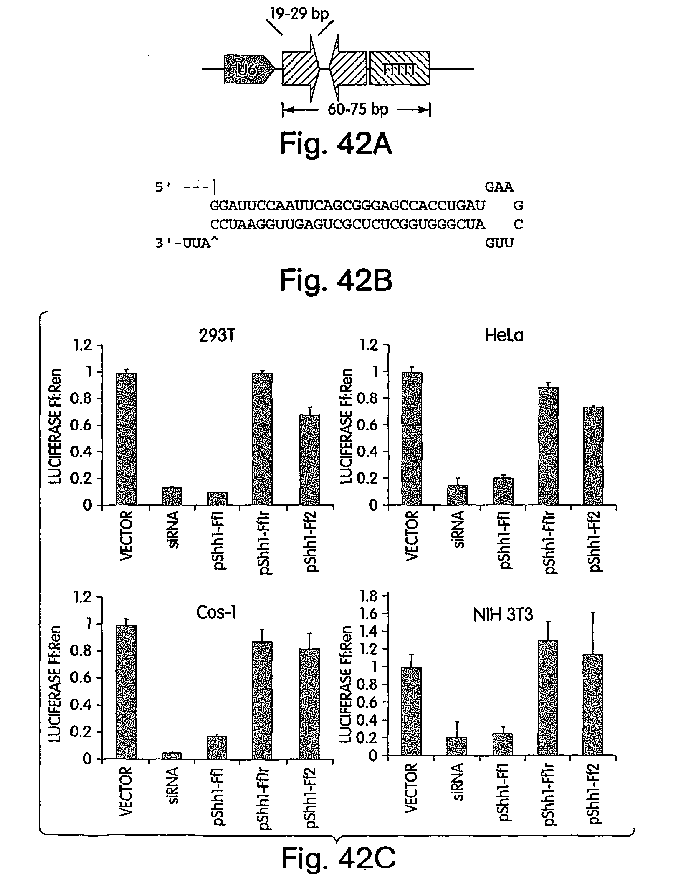

Figure 42: Transcription of functional shRNAs in vivo. (**A**) Schematic of the pShh1 vector. Sequences encoding shRNAs with between 19 and 29 bases of homology to the targeted gene are synthesized as 60-75-bp double-stranded DNA oligonucleotides and ligated into an *Eco*RV site immediately downstream of the U6 promoter. (**B**) Sequence and predicted secondary structure of the Ff1 hairpin. (**C**) An shRNA expressed from the pShh1 vector suppresses luciferase expression in mammalian cells. HEK 293T, HeLa, COS-1, and NIH 3T3 cells were transfected with reporter plasmids as in Figure 1, and pShh1 vector, firefly siRNA, or pShh1 firefly shRNA constructs as indicated. The ratios of firefly to *Renilia* luciferase activity were determined 48 h after transfection and represent the average of three independent experiments; error bars indicate standard deviation.

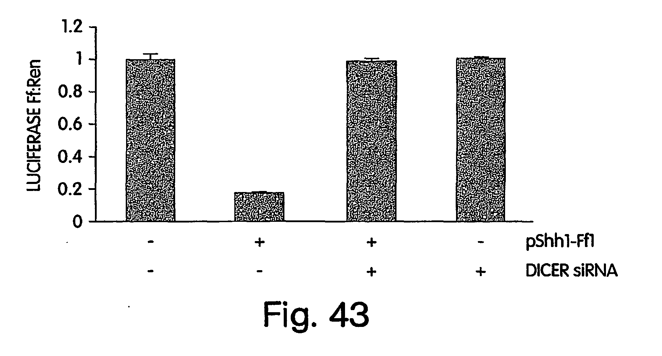

Figure 43: Dicer is required for shRNA-mediated gene silencing. HEK 293T cells were transfected with luciferase reporter plasmids as well as pShh1-Ff1 and an siRNA targeting human Dicer either alone or in combination, as indicated. The Dicer siRNA sequence (TCA ACC AGC CAC TGC TGG A) corresponds to coordinates 3137-3155 of the human *Dicer* sequence. The ratios of firefly to *Renilla* luciferase activity were determined 26 h after transfection and represent the average of three independent experiments; error bars indicate standard deviation.

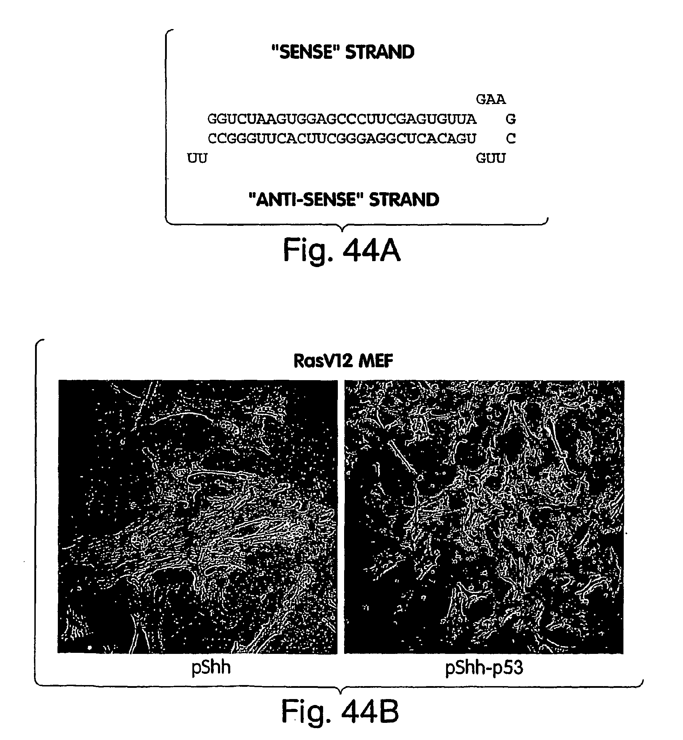

Figure 44: Stable shRNA-mediated gene silencing of an endogenous gene. (**A**) Sequence and predicted secondary structure of the *p53* hairpin. The 5' shRNA stem contains a 27-nt sequence derived from mouse *p53* (nucleotides 166-192), whereas the 3' stem harbors the complimentary antisense sequence. (**B**) Senescence bypass in primary mouse embryo fibroblasts (MEFs) expressing an shRNA targeted at *p53.* Wild-type MEFs, passage 5, were transfected with pBabe-RasV12 with control plasmid or with p53hp (5 µg each with FuGENE; Roche). Two days after transfection, cells were trypsinized, counted, and plated at a density of 1 × 10⁵/10-cm plate in media containing 2.0 µg/mL of puromycin. Control cells cease proliferation and show a senescent morphology (*left* panel). Cells expressing the *p53* hairpin continue to grow (*right* panel). Photos were taken 14 d posttransfection.

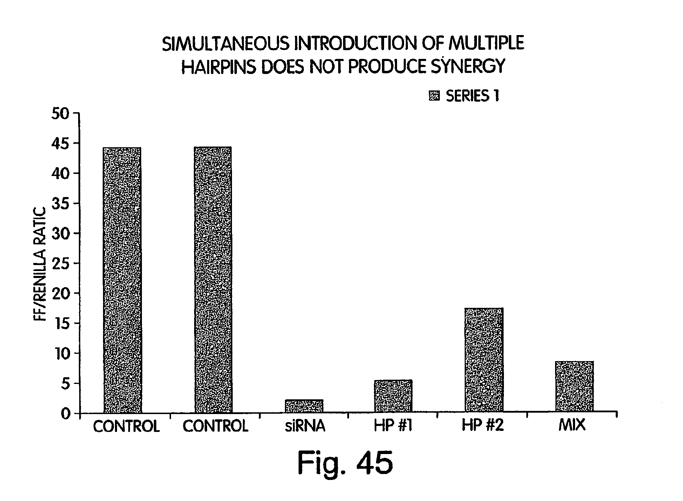

Figure 45: A mixture of two short hairpins, both corresponding to firefly luciferase, does not result in a synergistic suppression of gene expression. Suppression of firefly luciferase gene expression resulting from transfection of a mixture of two different short hairpins (HP #1 and HP #2) was examined. The mixture of HP #1 and HP #2 did not have a more robust effect on the suppression of firefly luciferase gene expression than expression of HP #1 alone.

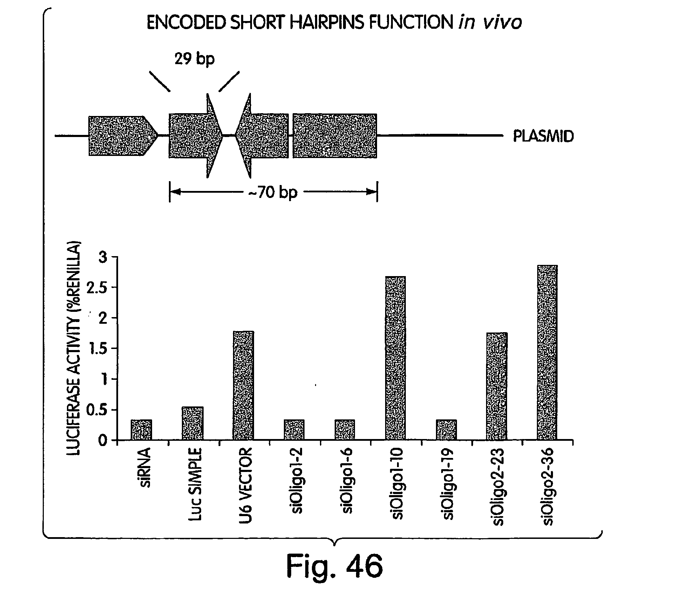

Figure 46: Encoded short hairpins specifically suppress gene expression in vivo. DNA oligonucleotides encoding 29 nucleotide hairpins corresponding to firefly luciferase were inserted into a vector containing the U6 promoter. Three independent constructs were examined for their ability to specifically suppress firefly luciferase gene expression in 293T cells. siOligo1-2, siOligo1-6, and siOligo1-19 (construct in the correct orientation) each suppressed gene expression as effectively as siRNA. In contrast, siOligo1-10 (construct in the incorrect orientation) did not suppress gene expression. An independent construct targeted to a different portion of the firefly luciferase gene did not effectively suppress gene expression in either orientation (siOligo2-23, siOligo2-36).

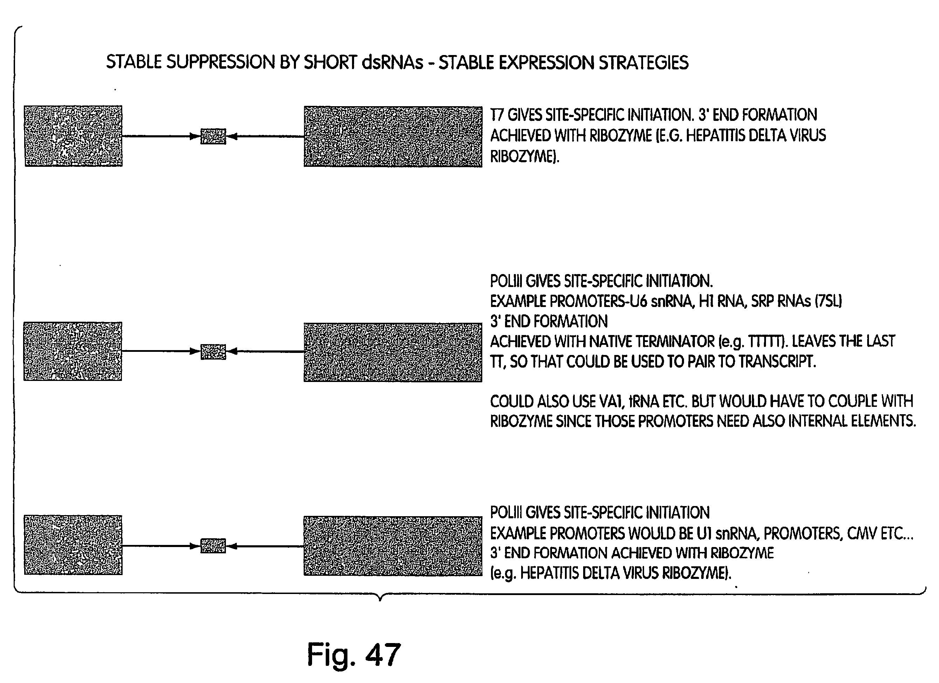

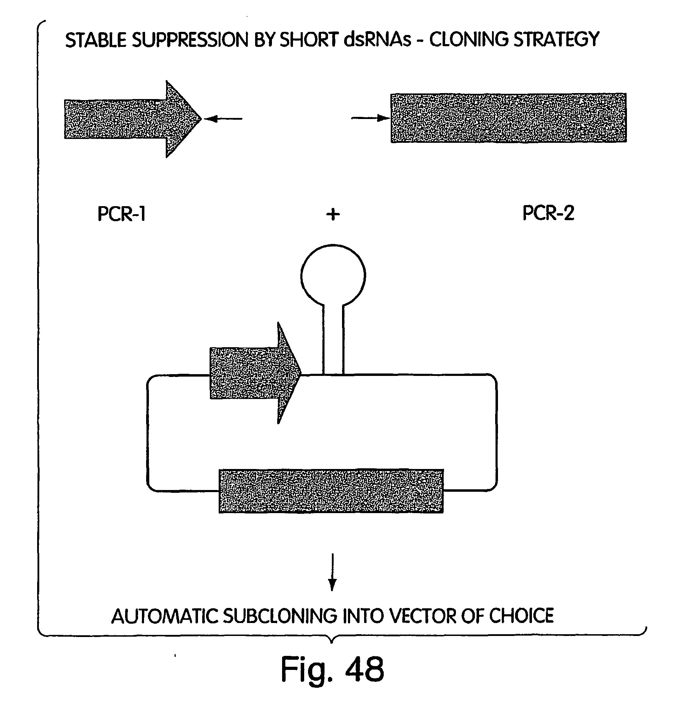

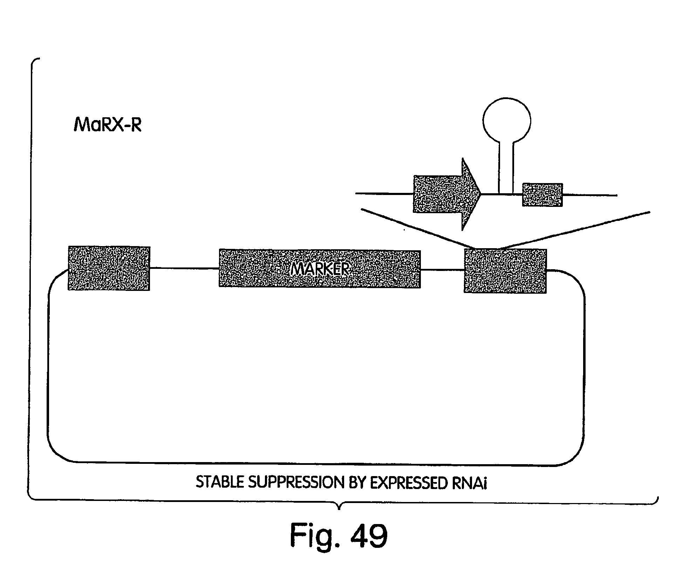

Figures 47-49: Strategies for stable expression of short dsRNAs.

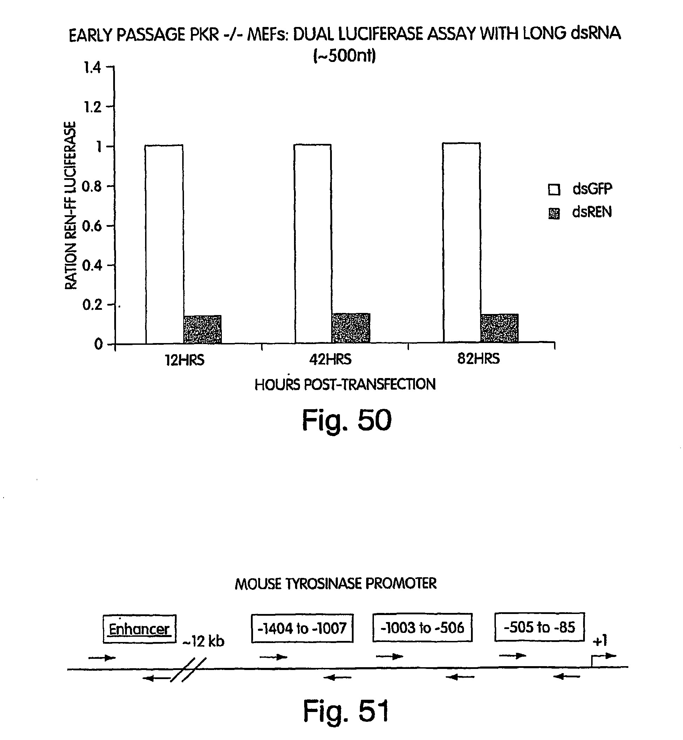

Figure 50: Dual luciferase assays were performed as described in detail in figures 28-35, however the cells used in these experiments were PKR-/- murine embryonic fibroblasts (MEFs). Briefly, RNAi using long dsRNAs typically envokes a non-specific response in MEFs (due to PKR activity). To evaluate the effect of long dsRNA constructs to specifically inhibit gene expression in MEFs, RNAi was examined in PKR -/- MEFs. Such cells do not respond to dsRNA with a non-specific response. The data summarized in this figure demonstrates that in the absence of the non-specific PKR response, long dsRNA constructs specifically suppress gene expression in MEFs.
Figure 51: Is a schematic representation of the mouse tyrosinase promoter. Primers were used to amplify three separate regions in the proximal promoter, or to amplify sequence corresponding to an enhancer located approximately 12 kb upstream.

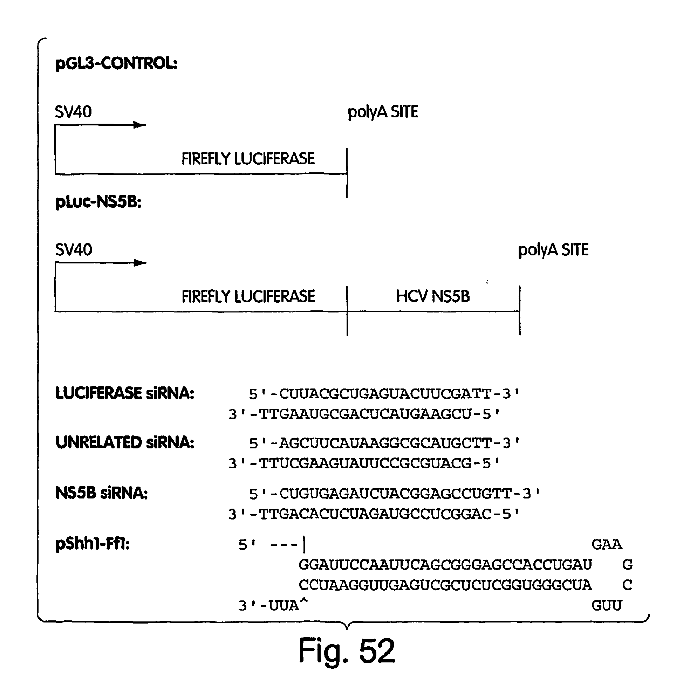

Figure 52: Reporter expression plasmids and siRNA sequences used in Figures X and Y. PGL-3-Control and Pluc -NS5B are the expression plasmids used for transfection into mouse liver. The nucleotide sequences of the siRNAs used in the study are shown underneath.

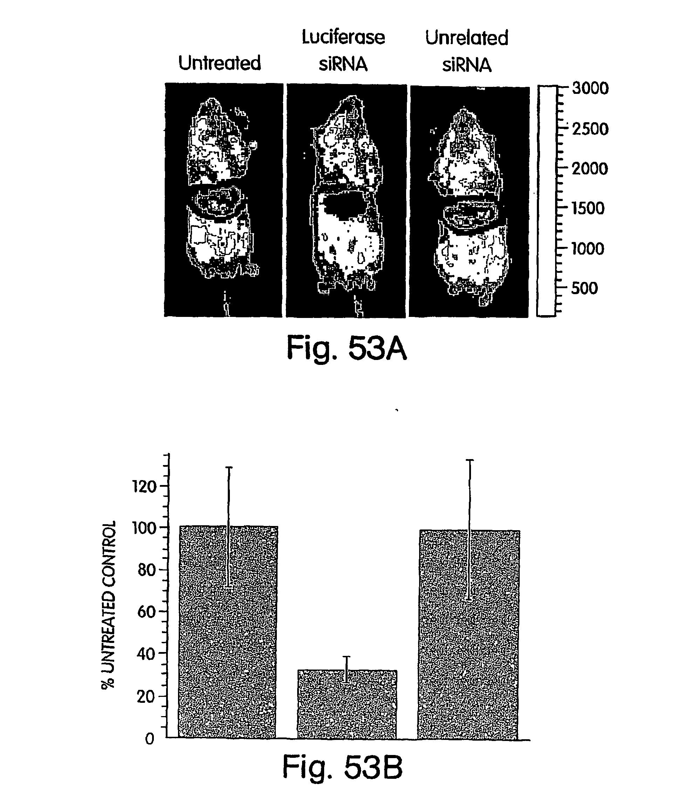

Figure 53: RNA interference in adult mice using siRNAs. (**a**) Representative images of light emitted from mice co-transfected with the luciferase plasmid pGL3-control and either no siRNA, luciferase siRNA or unrelated siRNA. A pseudocolour image representing intensity of emitted light (red, most intense; blue, least intense) superimposed on a greyscale reference image (for orientation) shows that RNAi functions in adult mice. Annealed 21-nucleotide siRNAs (40 µg; Dharmacon) were co-injected into the livers of mice with 2 µg pGL3-control DNA (Promega) and 800 units of RNasin (Promega) in 1.8 ml PBS buffer in 5-7 s. After 72 h, mice were anaesthetized and given 3 mg luciferin intraperitoneally 15 min before imaging. (**b**) siRNA results (six mice per group) from a representative experiment. Mice receiving luciferase siRNA emitted significantly less light than reporter-alone controls (one-way ANOVA with post hoc Fisher's test). Results for reporter alone and unrelated siRNA were statistically similar. Animals were treated according to the US National Institutes of Health's guidelines for animal care and the guidelines of Stanford University.

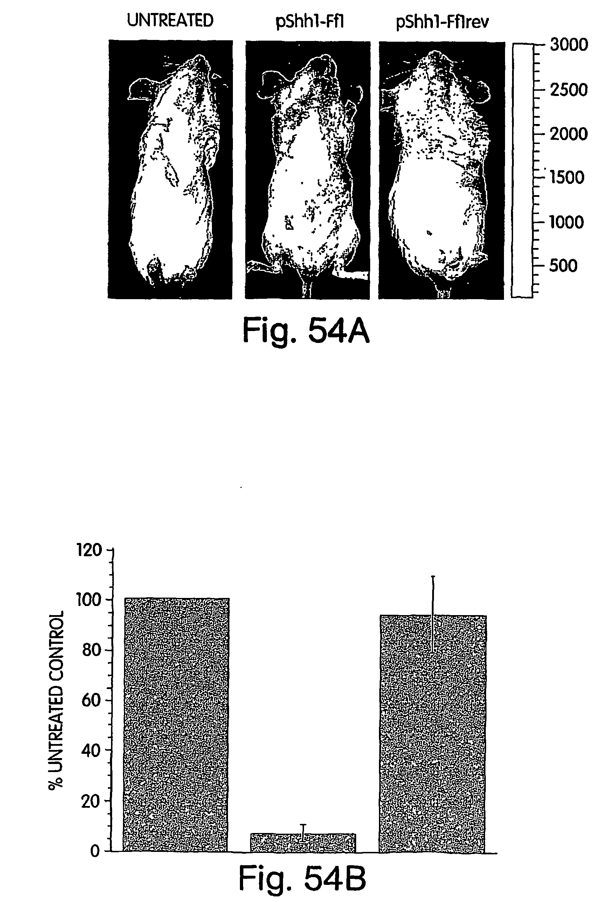

Figure 54: RNA interference in adult mice using shRNAs. (**a**) Representative images of light emitted from mice co-transfected with the luciferase plasmid control, pShh1-Ff1, and pShh1-Ff1rev. pShh1-Ff1, but not pShh1-Ff1rev, reduced luciferase expression in mice relative to the reporter-alone control. pShh1-Ff1 or pShh1-rev (10 µg) were co-injected with 2 µg pGL3-control in 1.8 ml PBS buffer. (**b**) Average of three independent shRNA experiments (n = 5). Average values for the reporter-alone group are designated as 100% in each of the three experiments. Animals were treated according to the US National Institutes of Health's guidelines for animal care and the guidelines of Stanford University.

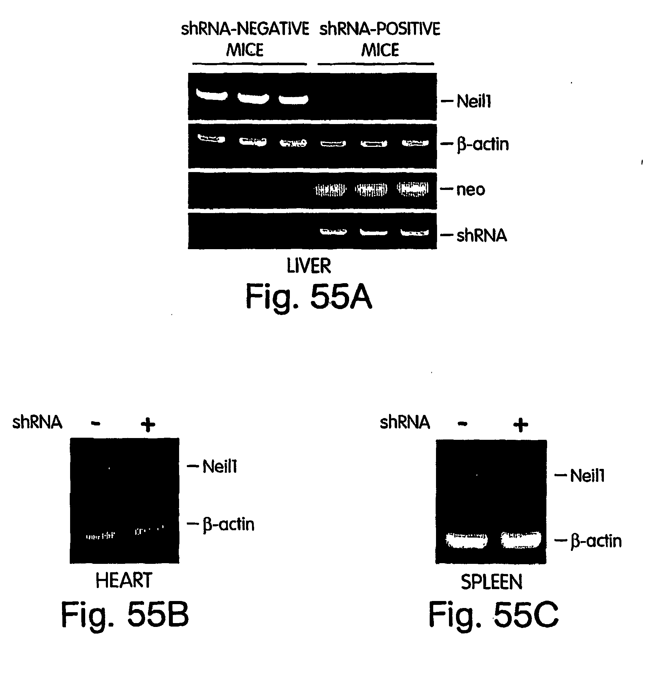

Figure 55: Heritable repression of Neill expression by RNAi in several tissues. (**a**) Expression of Neill mRNA in the livers of three mice containing the Neill shRNA transgene (shRNA-positive) or three siblings lacking the transgene (shRNA-negative) was assayed by RT-PCR (top row is Neill). An RT-PCR of β-actin was done to ensure that equal quantities of mRNAs were tested for each mouse (second row). Expression of the neomycin resistance gene (neo), carried on the shRNA vector, was tested similarly (third row). Finally, the mice were genotyped using genomic DNA that was PCR-amplified with vector-specific primers (bottom row). (**b**) Similar studies were performed in the heart. (**c**) Similar studies were performed in the spleen. Animal procedures have been approved by the SUNY, Stony Brook Institutional Animal Care and Use Committee (IACUC).

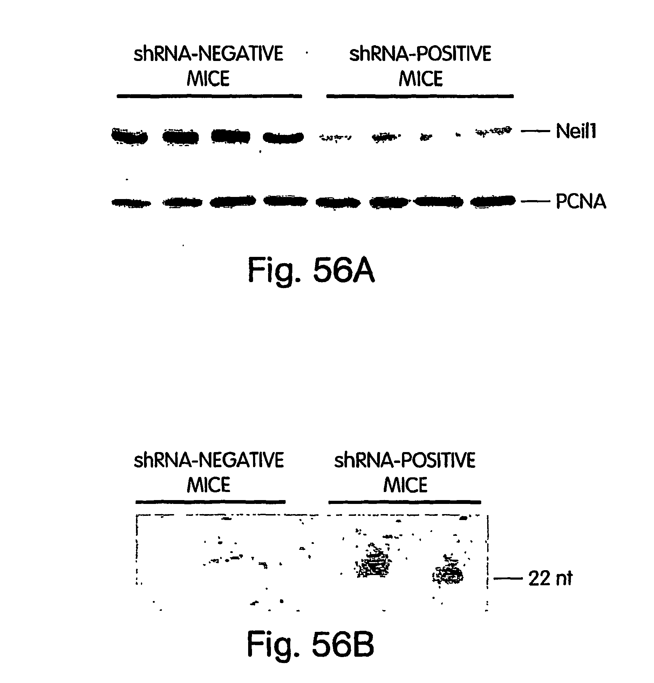

Figure 56: Reduction in Neill protein correlates with the presence of siRNAs. (**a**) Expression of Neil 1 protein was examined in protein extracts from the livers of mice carrying the shRNA transgene (shRNA-positive) or siblings lacking the transgene (shRNA-negative) by western blotting with Neill-specific antiserum. A western blot for PCNA was used to standardize loading. (**b**) The presence of siRNAs in RNA derived from the livers of transgenic mice as assayed by northern blotting using a 300 nt probe, part of which was complementary to the shRNA sequence. We note siRNAs only in mice transgenic for the shRNA expression cassette.

### Detailed Description of Certain Preferred Embodiments

It is to be appreciated that some aspects of the following embodiments are provided for background reference and do not form part of the invention.

### I. Overview

The present invention provides methods for attenuating gene expression in a cell using gene-targeted double stranded RNA (dsRNA). The dsRNA contains a nucleotide sequence that hybridizes under physiologic conditions of the cell to the nucleotide sequence of at least a portion of the gene to be inhibited (the "target" gene). The nucleotide sequence hybridizes to the coding sequence of the target gene.

A significant aspect to certain embodiments of the present invention relates to the demonstration in the present application that RNAi can in fact be accomplished both in cultured mammalian cells and in whole organisms. This had not been previously described in the art.

Another salient feature of the present invention concerns the ability to carry out RNAi in higher eukaryotes, particularly in non-oocytic cells of mammals, e.g., cells from adult mammals as an example.

As described in further detail below, the present invention(s) are based on the discovery that the RNAi phenomenum is mediated by a set of enzyme activities, including an essential RNA component, that are evolutionarily conserved in eukaryotes ranging from plants to mammals.

One enzyme contains an essential RNA component. After partial purification, a multi-component nuclease (herein "RISC nuclease") co-fractionates with a discrete, 22-nucleotide RNA species which may confer specificity to the nuclease through homology to the substrate mRNAs. The short RNA molecules are generated by a processing reaction from the longer input dsRNA. Without wishing to be bound by any particular theory, these 22mer guide RNAs may serve as guide sequences that instruct the RISC nuclease to destroy specific mRNAs corresponding to the dsRNA sequences.

As illustrated, double stranded forms of the 22-mer guide RNA can be sufficient in length to induce sequence-dependent dsRNA inhibition of gene expression. In the illustrated example, dsRNA contructs are administered to cells having a recombinant luciferase reporter gene. In the control cell, e.g., no exogeneously added RNA, the level of expression of the luciferase reporter is normalized to be the value of "1". As illustrated, both long (500-mer) and short (22-mer) dsRNA constructs complementary to the luciferase gene could inhibit expression of that gene product relative to the control cell. On the other hand, similarly sized dsRNA complementary to the coding sequence for another protein, green fluorescence protein (GFP), did not significantly effect the expression of luciferase -indicating that the inhibitory phenomena was in each case sequence-dependent. Likewise, single stranded 22-mers of luciferase did not inhibit expression of that gene - indicating that the inhibitory phenomena is double stranded-dependent.

The appended examples also identify an enzyme, Dicer, that can produce the putative guide RNAs. Dicer is a member of the RNAse III family of nucleases that specifically cleave dsRNA and is evolutionarily conserved in worms, flies, plants, fungi and, as described herein, mammals. The enzyme has a distinctive structure which includes a helicase domain and dual RNAse III motifs. Dicer also contains a region of homology to the RDEI/QDE2/ARGONAUTE family, which have been genetically linked to RNAi in lower eukaryotes. Indeed, activation of, or overexpression of Dicer may be sufficient in many cases to permit RNA interference in otherwise non-receptive cells, such as cultured eukaryotic cells, or mammalian (non-oocytic) cells in culture or in whole organisms.

In certain embodiments, the cells can be treated with an agent(s) that inhibits the general double-stranded RNA response(s) by the host cells, such as may give rise to sequence-independent apoptosis. For instance, the cells can be treated with agents that inhibit the dsRNA-dependent protein kinase known as PKR (protein kinase RNA-activated). Double stranded RNAs in mammalian cells typically activate protein kinase PKR and lead to apoptosis. The mechanism of action of PKR includes phosphorylation and inactivation of eIF2α (Fire (1999) Trends Genet 15: 358). It has also been reported that induction of NF-κB by PKR is involved in apoptosis commitment and this process is mediated through activation of the IKK complex. This sequence-independent response may reflect a form of primitive immune response, since the presence of dsRNA is a common feature of many viral lifecycles.

As described herein, Applicants have demonstrated that the PKR response can be overcome in favor of the sequence-specific RNAi response. However, in certain instances, it may be desirable to treat the cells with agents which inhibit expression of PKR, cause its destruction, and/or inhibit the kinase activity of PKR, and such methods are specifically contemplated for use in the present invention. Likewise, overexpression of agents which ectopically activate eIF2α can be used. Other agents which can be used to suppress the PKR response include inhibitors of IKK phosphorylation of IκB, inhibitors of IκB ubiquitination, inhibitors of 1κB degradation, inhibitors of NF-κB nuclear translocation, and inhibitors of NF-κB interaction with κB response elements.

Other inhibitors of sequence-independent dsRNA response in cells include the gene product of the vaccinia virus E3L. The E3L gene product contains two distinct domains. A conserved carboxy-terminal domain has been shown to bind double-stranded RNA (dsRNA) and inhibit the antiviral dsRNA response by cells. Expression of at least that portion of the E3L gene in the host cell, or the use of polypeptide or peptidomimetics thereof, can be used to suppress the general dsRNA response. Caspase inhibitors sensitize cells to killing by double-stranded RNA. Accordingly, ectopic expression or activation of caspases in the host cell can be used to suppress the general dsRNA response.

In other embodiments, the subject method is carried out in cells which have little or no general response to double stranded RNA, e.g., have no PKR-dependent dsRNA response, at least under the culture conditions. As illustrated in Figures 28-32, CHO and P19 cells can be used without having to inhibit PKR or other general dsRNA responses.

Thus, the present invention provides a process and compositions for inhibiting expression of a target gene in a mammalian cell. In certain embodiments, the process comprises introduction of RNA (the "dsRNA construct") with partial or fully double-stranded character into the cell or into the extracellular environment. Inhibition is specific in that a nucleotide sequence from a portion of the target gene is chosen to produce the dsRNA construct. The dsRNA may be identical or similar to the coding sequence of the target gene. In preferred application, the method utilizes a cell in which Dicer and/or Argonaute activities are recombinantly expressed or otherwise ectopically activated. This process can be (1) effective in attenuating gene expression, (2) specific to the targeted gene, and (3) general in allowing inhibition of many different types of target gene.

### II. Definitions

For convenience, certain terms employed in the specification, examples, and appended claims are collected here.

As used herein, the term "vector," refers to a nucleic acid molecule capable of transporting another nucleic acid to which it has been linked. The vector is a genomic integrated vector, or "integrated vector", which can become integrated into the chromsomal DNA of the host cell. Vectors capable of directing the expression of genes to which they are operatively linked are referred to herein as "expression vectors". In the present specification, "plasmid" and "vector" are used interchangeably unless otherwise clear from the context.

As used herein, the term "nucleic acid" refers to polynucleotides such as deoxyribonucleic acid (DNA), and, where appropriate, ribonucleic acid (RNA). The term should also be understood to include, as applicable to the embodiment being described, single-stranded (such as sense or antisense) and double-stranded polynucleotides.

As used herein, the term "gene" or "recombinant gene" refers to a nucleic acid comprising an open reading frame encoding a polypeptide of the present invention, including both exon and (optionally) intron sequences. The nucleic acid may also optionally include non-coding sequences such as promoter or enhancer sequences. A "recombinant gene" refers to nucleic acid encoding such regulatory polypeptides, that may optionally include intion sequences that are derived from chromosomal DNA. The term "intron" refers to a DNA sequence present in a given gene that is not translated into protein and is generally found between exons.

A "protein coding sequence" or a sequence that "encodes" a particular polypeptide or peptide, is a nucleic acid sequence that is transcribed (in the case of DNA) and is translated (in the case of mRNA) into a polypeptide in vitro or in vivo when placed under the control of appropriate regulatory sequences. The boundaries of the coding sequence are determined by a start codon at the 5' (amino) terminus and a translation stop codon at the 3' (carboxy) terminus. A coding sequence can include, but is not limited to, cDNA from procaryotic or eukaryotic mRNA, genomic DNA sequences from procaryotic or eukaryotic DNA, and even synthetic DNA sequences. A transcription termination sequence will usually be located 3' to the coding sequence.

Likewise, "encodes", unless evident from its context, will be meant to include DNA sequences that encode a polypeptide, as the term is typically used, as well as DNA sequences that are transcribed into inhibitory antisense molecules.

The term "loss-of-function", as it refers to genes inhibited by the subject RNAi method, refers to a diminishment in the level of expression of a gene(s) in the presence of one or more dsRNA construct(s) when compared to the level in the absense of such dsRNA construct(s).

The term "expression" with respect to a gene sequence refers to transcription of the gene and, as appropriate, translation of the resulting mRNA transcript to a protein. Thus, as will be clear from the context, expression of a protein coding sequence results from transcription and translation of the coding sequence.

"Cells," "host cells" or "recombinant host cells" are terms used interchangeably herein. It is understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

The term "cultured cells" refers to cells suspended in culture, e.g., dispersed in culture or in the form tissue. It does not, however, include oocytes or whole embryos (including blastocysts and the like) which may be provided in culture. In certain embodiments, the cultured cells are adults cells, e.g., non-embryonic.

By "recombinant virus" is meant a virus that has been genetically altered, e.g., by the addition or insertion of a heterologous nucleic acid construct into the particle.

As used herein, the terms "transduction" and "transfection" are art recognized and mean the introduction of a nucleic acid, e.g., an expression vector, into a recipient cell by nucleic acid-mediated gene transfer. "Transformation", as used herein, refers to a process in which a cell's genotype is changed as a result of the cellular uptake of exogenous DNA or RNA, and, for example, the transformed cell expresses a dsRNA contruct.

"Transient transfection," (for background purposes) refers to cases where exogenous DNA does not integrate into the genome of a transfected cell, e.g., where episomal DNA is transcribed into mRNA and translated into protein.

A cell has been "stably transfected" with a nucleic acid construct when the nucleic acid construct is capable of being inherited by daughter cells.

As used herein, a "reporter gene construct" is a nucleic acid that includes a "reporter gene" operatively linked to at least one transcriptional regulatory sequence. Transcription of the reporter gene is controlled by these sequences to which they are linked. The activity of at least one or more of these control sequences can be directly or indirectly regulated by the target receptor protein. Exemplary transcriptional control sequences are promoter sequences. A reporter gene is meant to include a promoter-reporter gene construct that is heterologously expressed in a cell.

As used herein, "transformed cells" refers to cells that have spontaneously converted to a state of unrestrained growth, i.e., they have acquired the ability to grow through an indefinite number of divisions in culture. Transformed cells may be characterized by such terms as neoplastic, anaplastic and/or hyperplastic, with respect to their loss of growth control. For purposes of this invention, the terms "transformed phenotype of malignant mammalian cells" and "transformed phenotype" are intended to encompass, but not be limited to, any of the following phenotypic traits associated with cellular transformation of mammalian cells: immortalization, morphological or growth transformation, and tumorigenicity, as detected by prolonged growth in cell culture, growth in semi-solid media, or tumorigenic growth in immuno-incompetent or syngeneic animals.

As used herein, "proliferating" and "proliferation" refer to cells undergoing mitosis.

As used herein, "immortalized cells" refers to cells that have been altered via chemical, genetic, and/or recombinant means such that the cells have the ability to grow through an indefinite number of divisions in culture.

The "growth state" of a cell refers to the rate of proliferation of the cell and the state of differentiation of the cell.

"MHC antigen", as used herein, refers to a protein product of one or more MHC genes; the term includes fragments or analogs of products of MHC genes which can evoke an immune response in a recipient organism. Examples of MHC antigens include the products (and fragments or analogs thereof) of the human MHC genes, i.e., the HLA genes.

The term "histocompatibility" refers to the similarity of tissue between different individuals. The level of histocompatibility describes how well matched the patient and donor are. The major histocompatibility determinants are the human leukocyte antigens (HLA). HLA typing is performed between the potential marrow donor and the potential transplant recipient to determine how close a HLA match the two are. The closer the match the less the donated marrow and the patient's body will react against each other.

The term "human leukocyte antigens" or "HLA", refers to proteins (antigens) found on the surface of white blood cells and other tissues that are used to match donor and patient. For instances, a patient and potential donor may have their white blood cells tested for such HLA antigens as, HLA-A, B and DR. Each individual has two sets of these antigens, one set inherited from each parent. For this reason, it is much more likely for a brother or sister to match the patient than an unrelated individual, and much more likely for persons of the same racial and ethnic backgrounds to match each other.

### III. Exemplary Embodiments of Isolation Method

One aspect of the invention provides a method for potentiating RNAi by induction or ectopic activation of an RNAi enzyme in a cell in vivo or in vitro) or cell-free mixtures. In preferred embodiments, the RNAi activity is activated or added to a mammalian cell, e.g., a human cell, which cell may be provided in vitro or as part of a whole organism. In other embodiments, the subject method is carried out using eukaryotic cells generally (except for oocytes) in culture. For instance, the Dicer enzyme may be activated by virtue of being recombinantly expressed or it may be activated by use of an agent which (i) induces expression of the endogenous gene, (ii) stabilizes the protein from degradation, and/or (iii) allosterically modifies the enzyme to increase its activity (by altering its Kcat, Km or both).

### A. Dicer and Argonaut Activities

In certain embodiments, at least one of the activated RNAi enzymes is Dicer, or a homolog thereof. In certain preferred embodiments, the present method provides for ectopic activation of Dicer. As used herein, the term "Dicer" refers to a protein which (a) mediates an RNAi response and (b) has an amino acid sequence at least 50 percent identical, and more preferably at least 75, 85, 90 or 95 percent identical to SEQ ID No. 2 or 4, and/or which can be encoded by a nucleic acid which hybridizes under wash conditions of 2 x SSC at 22°C, and more preferably 0.2 x SSC at 65°C, to a nucleotide represented by SEQ ID No. 1 or 3. Accordingly, the method may comprise introducing a dsRNA contruct into a cell in which Dicer has been recombinantly expressed or otherwise ectopically activated.

In certain embodiment, at least one of the activated RNAi enzymes is Argonaut, or a homolog thereof. In certain preferred embodiments, the present method provides for ectopic activation of Argonaut. As used herein, the term "Argonaut" refers to a protein which (a) mediates an RNAi response and (b) has an amino acid sequence at least 50 percent identical, and more preferably at least 75, 85, 90 or 95 percent identical to the amino acid sequence shown in Figure 24. Accordingly, the method may comprise introducing a dsRNA contruct into a cell in which Argonaut has been recombinantly expressed or otherwise ectopically activated.

This invention also provides expression vectors containing a nucleic acid encoding a Dicer or Argonaut polypeptide, operably linked to at least one transcriptional regulatory sequence. Operably linked is intended to mean that the nucleotide sequence is linked to a regulatory sequence in a manner which allows expression of the nucleotide sequence. Regulatory sequences are art-recognized and are selected to direct expression of the subject Dicer or Argonaut proteins. Accordingly, the term transcriptional regulatory sequence includes promoters, enhancers and other expression control elements. Such regulatory sequences are described in Goeddel; Gene Expression Technology: Methods in Enzymology 185, Academic Press, San Diego, CA (1990). For instance, any of a wide variety of expression control sequences, sequences that control the expression of a DNA sequence when operatively linked to it, may be used in these vectors to express DNA sequences encoding Dicer or Argonaut polypeptides of this invention. Such useful expression control sequences, include, for example, a viral LTR, such as the LTR of the Moloney murine leukemia virus, the early and late promoters of SV40, adenovirus or cytomegalovirus immediate early promoter, the lac system, the trp system, the TAC or TRC system, T7 promoter whose expression is directed by T7 RNA polymerase, the major operator and promoter regions of phage λ, the control regions for fd coat protein, the promoter for 3-phosphoglycerate kinase or other glycolytic enzymes, the promoters of acid phosphatase, e.g., Pho5, the promoters of the yeast α-mating factors, the polyhedron promoter of the baculovirus system and other sequences known to control the expression of genes of prokaryotic or eukaryotic cells or their viruses, and various combinations thereof. It should be understood that the design of the expression vector may depend on such factors as the choice of the host cell to be transformed and/or the type of protein desired to be expressed.

Moreover, the vector's copy number, the ability to control that copy number and the expression of any other proteins encoded by the vector, such as antibiotic markers, should also be considered.

The recombinant Dicer or Argonaut genes can be produced by ligating a nucleic acid encoding a Dicer or Argonaut polypeptide into a vector suitable for expression in either prokaryotic cells, eukaryotic cells, or both. Expression vectors for production of recombinant forms of the subject Dicer or Argonaut polypeptides include plasmids and other vectors. For instance, suitable vectors for the expression of a Dicer or Argonaut polypeptide include plasmids of the types: pBR322-derived plasmids, pEMBL-derived plasmids, pEX-derived plasmids, pBTac-derived plasmids and pUC-derived plasmids for expression in prokaryotic cells, such as E. coli.

A number of vectors exist for the expression of recombinant proteins in yeast. For instance, YEP24, YIP5, YEP51, YEP52, pYES2, and YRP17 are cloning and expression vehicles useful in the introduction of genetic constructs into S. cerevisiae (see, for example, Broach et al. (1983) in Experimental Manipulation of Gene Expression, ed. M. Inouye Academic Press, p. 83). These vectors can replicate in E. coli due the presence of the pBR322 ori, and in S. cerevisiae due to the replication determinant of the yeast 2 micron plasmid. In addition, drug resistance markers such as ampicillin can be used. In an illustrative embodiment, a Dicer or Argonaut polypeptide is produced recombinantly utilizing an expression vector generated by sub-cloning the coding sequence of a Dicer or Argonaut gene.

The preferred mammalian expression vectors contain both prokaryotic sequences, to facilitate the propagation of the vector in bacteria, and one or more eukaryotic transcription units that are expressed in eukaryotic cells. The pcDNAI/amp, pcDNAI/neo, pRc/CMV, pSV2gpt, pSV2neo, pSV2-dhfr, pTk2, pRSVneo, pMSG, pSVT7, pko-neo and pHyg derived vectors are examples of mammalian expression vectors suitable for transfection of eukaryotic cells. Some of these vectors are modified with sequences from bacterial plasmids, such as pBR322, to facilitate replication and drug resistance selection in both prokaryotic and eukaryotic cells. Alternatively, derivatives of viruses such as the bovine papillomavirus (BPV-1), or Epstein-Barr virus (pHEBo, pREP-derived and p205) can be used for transient expression of proteins in eukaryotic cells. The various methods employed in the preparation of the plasmids and transformation of host organisms are well known in the art. For other suitable expression systems for both prokaryotic and eukaryotic cells, as well as general recombinant procedures, see Molecular Cloning A Laboratory Manual, 2nd Ed., ed. by Sambrook, Fritsch and Maniatis (Cold Spring Harbor Laboratory Press: 1989) Chapters 16 and 17.

The subject invention may provide a "gene activation" construct which, by homologous recombination with a genomic DNA, alters the transcriptional regulatory sequences of an endogenous Dicer or Argonaut gene. For instance, the gene activation construct can replace the endogenous promoter of a Dicer or Argonaut gene with a heterologous promoter, e.g., one which causes constitutive expression of the Dicer or Argonaut gene or which causes inducible expression of the gene under conditions different from the normal expression pattern of Dicer or Argonaut. A variety of different formats for the gene activation constructs are available. See, for example, the Transkaryotic Therapies, Inc PCT publications WO93/09222, WO95/31560, WO96/294411, WO95/31560 and WO94/12650.

In preferred embodiments, the nucleotide sequence used as the gene activation construct can be comprised of (1) DNA from some portion of the endogenous Dicer or Argonaut gene (exon sequence, intron sequence, promoter sequences, etc.) which direct recombination and (2) heterologous transcriptional regulatory sequence(s) which is to be operably linked to the coding sequence for the genomic Dicer or Argonaut gene upon recombination of the gene activation construct. For use in generating cultures of Dicer or Argonaut producing cells, the construct may further include a reporter gene to detect the presence of the knockout construct in the cell.

The gene activation construct is inserted into a cell, and integrates with the genomic DNA of the cell in such a position so as to provide the heterologous regulatory sequences in operative association with the native Dicer or Argonaut gene. Such insertion occurs by homologous recombination, i.e., recombination regions of the activation construct that are homologous to the endogenous Dicer or Argonaut gene sequence hybridize to the genomic DNA and recombine with the genomic sequences so that the construct is incorporated into the corresponding position of the genomic DNA.

The terms "recombination region" or "targeting sequence" refer to a segment (i.e., a portion) of a gene activation construct having a sequence that is substantially identical to or substantially complementary to a genomic gene sequence, e.g., including 5' flanking sequences of the genomic gene, and can facilitate homologous recombination between the genomic sequence and the targeting transgene construct.

As used herein, the term "replacement region" refers to a portion of a activation construct which becomes integrated into an endogenous chromosomal location following homologous recombination between a recombination region and a genomic sequence.

The heterologous regulatory sequences, e.g., which are provided in the replacement region, can include one or more of a variety of elements, including: promoters (such as constitutive or inducible promoters), enhancers, negative regulatory elements, locus control regions, transcription factor binding sites, or combinations thereof.

Promoters/enhancers which may be used to control the expression of the targeted gene *in vivo* include, but are not limited to, the cytomegalovirus (CMV) promoter/enhancer (Karasuyama et al. (1989) J. Exp. Med 169: 13), the human β-actin promoter (Gunning et al. (1987) PNAS 84: 4831-4835), the glucocorticoid-inducible promoter present in the mouse mammary tumor virus long terminal repeat (MMTV LTR) (Klessig et al. (1984) Mol. Cell Biol. 4: 1354-1362), the long terminal repeat sequences of Moloney murine leukemia virus (MuLV LTR) (Weiss et al. (1985) RNA Tumor Viruses, Cold Spring Harbor Laboratory, Cold Spring Harbor, New York), the SV40 early or late region promoter (Bernoist et al. (1981) Nature 290: 304-310; Templeton et al. (1984) Mol. Cell Biol. 4: 817; and Sprague et al. (1983) J. Virol. 45: 773), the promoter contained in the 3' long terminal repeat of Rous sarcoma virus (RSV) (Yamamoto et al. (1980) Cell 22: 787-797), the herpes simplex virus (HSV) thymidine kinase promoter/enhancer (Wagner et al. (1981) PNAS 82: 3567-71), and the herpes simplex virus LAT promoter (Wolfe et al. (1992) Nature Genetics 1: 379-384).

In still other embodiments, the replacement region merely deletes a negative transcriptional control element of the native gene, e.g., to activate expression, or ablates a positive control element, e.g., to inhibit expression of the targeted gene.

### B. Cell/Organism

The cell with the target gene may be derived from or contained in any mammal The dsRNA construct may be synthesized either in vivo or in vitro. Endogenous RNA polymerase of the cell may mediate transcription in vivo, or cloned RNA polymerase can be used for transcription in vivo or in vitro. For generating double stranded transcripts from a transgene in vivo, a regulatory region may be used to transcribe the RNA strand (or strands). Furthermore, dsRNA can be generated by transcribing an RNA strand which forms a hairpin, thus producing a dsRNA.

Genetic manipulation becomes possible in organisms that are not classical genetic models. Breeding and screening programs may be accelerated by the ability to rapidly assay the consequences of a specific, targeted gene disruption. Gene disruptions may be used to discover the function of the target gene, to produce disease models in which the target gene are involved in causing or preventing a pathological condition, and to produce organisms with improved economic properties.

The cell with the target gene may be derived from or contained in any mammal.

Examples of , mammals include cattle, goat, pig, sheep, rodent, hamster, mouse, rat, primate, and human.

The cell having the target gene may be from non-human germ line cells or somatic, pluripotent cells, dividing or non-dividing, parenchyma or epithelium, immortalized or transformed, or the like. The cell may be a stem cell or a differentiated cell. Cell types that are differentiated include adipocytes, fibroblasts, myocytes, cardiomyocytes, endothelium, neurons, glia, blood cells, megakaryocytes, lymphocytes, macrophages, neutrophils, eosinophils, basophils, mast cells, leukocytes, granulocytes, keratinocytes, chondrocytes, osteoblasts, osteoclasts, hepatocytes, and cells of the endocrine or exocrine glands.

### C. Targeted Genes

The target gene may be a gene derived from the cell, an endogenous gene, a transgene, or a gene of a pathogen which is present in the cell after infection thereof. Depending on the particular target gene and the dose of double stranded RNA material delivered, the procedure may provide partial or complete loss of function for the target gene. Lower doses of injected material and longer times after administration of dsRNA may result in inhibition in a smaller fraction of cells. Quantitation of gene expression in a cell may show similar amounts of inhibition at the level of accumulation of target mRNA or translation of target protein.

"Inhibition of gene expression" refers to the absence (or observable decrease) in the level of protein and/or mRNA product from a target gene. "Specificity" refers to the ability to inhibit the target gene without manifest effects on other genes of the cell. The consequences of inhibition can be confirmed by examination of the outward properties of the cell or organism (as presented below in the examples) or by biochemical techniques such as RNA solution hybridization, nuclease protection, Northern hybridization, reverse transcription, gene expression monitoring with a microarray, antibody binding, enzyme linked immunosorbent assay (ELISA), Western blotting, radioimmunoassay (RIA), other immunoassays, and fluorescence activated cell analysis (FACS). For RNA-mediated inhibition in a cell line or whole organism, gene expression is conveniently assayed by use of a reporter or drug resistance gene whose protein product is easily assayed. Such reporter genes include acetohydroxyacid synthase (AHAS), alkaline phosphatase (AP), beta galactosidase (LacZ), beta glucoronidase (GUS), chloramphenicol acetyltransferase (CAT), green fluorescent protein (GFP), horseradish peroxidase (HRP), luciferase (Luc), nopaline synthase (NOS), octopine synthase (OCS), and derivatives thereof. Multiple selectable markers are available that confer resistance to ampicillin, bleomycin, chloramphenicol, gentamycin, hygromycin, kanamycin, lincomycin, methotrexate, phosphinothricin, puromycin, and tetracyclin.

Depending on the assay, quantitation of the amount of gene expression allows one to determine a degree of inhibition which is greater than 10%, 33%, 50%, 90%, 95% or 99% as compared to a cell not treated according to the present invention. Lower doses of injected material and longer times after administration of dsRNA may result in inhibition in a smaller fraction of cells (e.g., at least 10%, 20%, 50%, 75%, 90%, or 95% of targeted cells). Quantitation of gene expression in a cell may show similar amounts of inhibition at the level of accumulation of target mRNA or translation of target protein. As an example, the efficiency of inhibition may be determined by assessing the amount of gene product in the cell: mRNA may be detected with a hybridization probe having a nucleotide sequence outside the region used for the inhibitory double-stranded RNA, or translated polypeptide may be detected with an antibody raised against the polypeptide sequence of that region.

As disclosed herein, the present invention is not limited to any type of target gene or nucleotide sequence. But the following classes of possible target genes are listed for illustrative purposes: developmental genes (e.g., adhesion molecules, cyclin kinase inhibitors, Writ family members, Pax family members, Winged helix family members, Hox family members, cytokines/lymphokines and their receptors, growth/differentiation factors and their receptors, neurotransmitters and their receptors); oncogenes (e.g., ABLI, BCLI, BCL2, BCL6, CBFA2, CBL, CSFIR, ERBA, ERBB, EBRB2, ETSI, ETS1, ETV6, FGR, FOS, FYN, HCR, HRAS, JUN, KRAS, LCK, LYN, MDM2, MLL, MYB, MYC, MYCLI, MYCN, NRAS, PIM 1, PML, RET, SRC, TALI, TCL3, and YES); tumor suppressor genes (e.g., APC, BRCA 1, BRCA2, MADH4, MCC, NF 1, NF2, RB 1, TP53, and WTI); and enzymes (e.g., ACC synthases and oxidases, ACP desaturases and hydroxylases, ADP-glucose pyrophorylases, ATPases, alcohol dehydrogenases, amylases, amyloglucosidases, catalases, cellulases, chalcone synthases, chitinases, cyclooxygenases, decarboxylases, dextrinases, DNA and RNA polymerases, galactosidases, glucanases, glucose oxidases, granule-bound starch synthases, GTPases, helicases, hemicellulases, integrases, inulinases, invertases, isomerases, kinases, lactases, lipases, lipoxygenases, lysozymes, nopaline synthases, octopine synthases, pectinesterases, peroxidases, phosphatases, phospholipases, phosphorylases, phytases, plant growth regulator synthases, polygalacturonases, proteinases and peptidases, pullanases, recombinases, reverse transcriptases, RUBISCOs, topoisomerases, and xylanases).

### D. dsRNA constructs

The dsRNA construct may comprise one or more strands of polymerized ribonucleotide. It may include modifications to either the phosphate-sugar backbone or the nucleoside. For example, the phosphodiester linkages of natural RNA may be modified to include at least one of a nitrogen or sulfur heteroatom. Modifications in RNA structure may be tailored to allow specific genetic inhibition while avoiding a general panic response in some organisms which is generated by dsRNA. Likewise, bases may be modified to block the activity of adenosine deaminase. The dsRNA construct may be produced enzymatically or by partial/total organic synthesis, any modified ribonucieotide can be introduced by in vitro enzymatic or organic synthesis.

The dsRNA construct may be directly introduced into the cell (i.e., intracellularly); or introduced extracellularly into a cavity, interstitial space, into the circulation of an organism, introduced orally, or may be introduced by bathing an organism in a solution containing RNA. Methods for oral introduction include direct mixing of RNA with food of the organism, as well as engineered approaches in which a species that is used as food is engineered to express an RNA, then fed to the organism to be affected. Physical methods of introducing nucleic acids include injection of an RNA solution directly into the cell or extracellular injection into the organism.

The double-stranded structure is formed by a single self-complementary RNA strand . RNA duplex formation may be initiated either inside or outside the cell. The RNA may be introduced in an amount which allows delivery of at least one copy per cell. Higher doses (e.g., at least 5, 10, 100, 500 or 1000 copies per cell) of double-stranded material may yield more effective inhibition; lower doses may also be useful for specific applications. Inhibition is sequence-specific in that nucleotide sequences corresponding to the duplex region of the RNA are targeted for genetic inhibition.

dsRNA constructs containing a nucleotide sequences identical to a portion, of the coding sequence, of the target gene are preferred for inhibition. RNA sequences with insertions, deletions, and single point mutations relative to the target sequence (ds RNA similar to the target gene) have also been found to be effective for inhibition. Thus, sequence identity may be optimized by sequence comparison and alignment algorithms known in the art (see Gribskov and Devereux, Sequence Analysis Primer, Stockton Press, 1991, and references cited therein) and calculating the percent difference between the nucleotide sequences by, for example, the Smith-Waterman algorithm as implemented in the BESTFIT software program using default parameters (e.g., University of Wisconsin Genetic Computing Group). Greater than 90% sequence identity, or even 100% sequence identity, between the inhibitory RNA and the portion of the target gene is preferred. Alternatively, the duplex region of the RNA may be defined functionally as a nucleotide sequence that is capable of hybridizing with a portion of the target gene transcript (e.g., 400 mM NaCl, 40 mM PIPES pH 6.4, 1 mM EDTA, 50 °C or 70 °C hybridization for 12-16 hours; followed by washing). In certain preferred embodiments, the length of the dsRNA is at least 20, 21 or 22 nucleotides in length, e.g., corresponding in size to RNA products produced by Dicer-dependent cleavage. In certain embodiments, the dsRNA construct is at least 25 bases.

100% sequence identity between the RNA and the target gene is not required to practice the present invention. Thus the invention has the advantage of being able to tolerate sequence variations that might be expected due to genetic mutation, strain polymorphism, or evolutionary divergence.

The dsRNA construct may be synthesized either in vivo or in vitro. Endogenous RNA polymerase of the cell may mediate transcription in vivo, or cloned RNA polymerase can be used for transcription in vivo or in vitro. For transcription from a transgene in vivo or an expression construct, a regulatory region (e.g., promoter, enhancer, silencer, splice donor and acceptor, polyadenylation) may be used to transcribe the dsRNA strand (or strands). Inhibition may be targeted by specific transcription in an organ, tissue, or cell type; stimulation of an environmental condition (e.g., infection, stress, temperature, chemical inducers); and/or engineering transcription at a developmental stage or age. The RNA strands may or may not be polyadenylated; the RNA strands may or may not be capable of being translated into a polypeptide by a cell's translational apparatus. The dsRNA construct may be chemically or enzymatically synthesized by manual or automated reactions. The dsRNA construct may be synthesized by a cellular RNA polymerase or a bacteriophage RNA polymerase (e.g., T3, T7, SP6). The use and production of an expression construct are known in the art (see also WO 97/32016; U.S. Pat. Nos. 5,593,874, 5,698,425, 5,712,135, 5,789,214, and 5,804,693; and the references cited therein). If synthesized chemically or by in vitro enzymatic synthesis, the RNA may be purified prior to introduction into the cell. For example, RNA can be purified from a mixture by extraction with a solvent or resin, precipitation, electrophoresis, chromatography or a combination thereof. Alternatively, the dsRNA construct may be used with no or a minimum of purification to avoid losses due to sample processing. The dsRNA construct may be dried for storage or dissolved in an aqueous solution. The solution may contain buffers or salts to promote annealing, and/or stabilization of the duplex strands.

Physical methods of introducing nucleic acids include injection of a solution containing the dsRNA construct, bombardment by particles covered by the dsRNA construct, soaking the cell or organism in a solution of the RNA, or electroporation of cell membranes in the presence of the dsRNA construct. A viral construct packaged into a viral particle would accomplish both efficient introduction of an expression construct into the cell and transcription of dsRNA construct encoded by the expression construct. Other methods known in the art for introducing nucleic acids to cells may be used, such as lipid-mediated carrier transport, chemical mediated transport, such as calcium phosphate, and the like. Thus the dsRNA construct may be introduced along with components that perform one or more of the following activities: enhance RNA uptake by the cell, promote annealing of the duplex strands, stabilize the annealed strands, or other-wise increase inhibition of the target gene.

### E. Illustrative Uses

One utility of the present invention is as a method of identifying gene function in an organism, especially higher eukaryotes, comprising the use of double-stranded RNA to inhibit the activity of a target gene of previously unknown function. Instead of the time consuming and laborious isolation of mutants by traditional genetic screening, functional genomics would envision determining the function of uncharacterized genes by employing the invention to reduce the amount and/or alter the timing of target gene activity. The invention could be used in determining potential targets for pharmaceuticals, understanding normal and pathological events associated with development, determining signaling pathways responsible for postnatal development/aging, and the like. The increasing speed of acquiring nucleotide sequence information from genomic and expressed gene sources, including total sequences for mammalian genomes, can be coupled with the invention to determine gene function in a cell or in a whole organism. The preference of different organisms to use particular codons, searching sequence databases for related gene products, correlating the linkage map of genetic traits with the physical map from which the nucleotide sequences are derived, and artificial intelligence methods may be used to define putative open reading frames from the nucleotide sequences acquired in such sequencing projects.

A simple assay would be to inhibit gene expression according to the partial sequence available from an expressed sequence tag (EST). Functional alterations in growth, development, metabolism, disease resistance, or other biological processes would be indicative of the normal role of the EST's gene product.

The ease with which the dsRNA construct can be introduced into an intact cell/organism containing the target gene allows the present invention to be used in high throughput screening (HTS). For example, duplex RNA can be produced by an amplification reaction using primers flanking the inserts of any gene library derived from the target cell or organism. Inserts may be derived from genomic DNA or mRNA (e.g., cDNA and cRNA). Individual clones from the library can be replicated and then isolated in separate reactions, but preferably the library is maintained in individual reaction vessels (e.g., a 96 well microtiter plate) to minimize the number of steps required to practice the invention and to allow automation of the process.

In an exemplary area, the subject invention may provide an arrayed library of RNAi constructs. The array may be in the form of solutions, such as multi-well plates, or may be "printed" on solid substrates upon which cells can be grown. To illustrate, solutions containing duplex RNAs that are capable of inhibiting the different expressed genes can be placed into individual wells positioned on a microtiter plate as an ordered array, and intact cells/organisms in each well can be assayed for any changes or modifications in behavior or development due to inhibition of target gene activity.

In one area, the subject method uses an arrayed library of RNAi constructs to screen for combinations of RNAi that are lethal to host cells. Synthetic lethality is a bedrock principle of experimental genetics. A synthetic lethality describes the properties of two mutations which, individually, are tolerated by the organism but which, in combination, are lethal. The subject arrays can be used to identify loss-of-function mutations that are lethal in combination with alterations in other genes, such as activated oncogenes or loss-of-function mutations to tumor suppressors. To achieve this, one can create "phenotype arrays" using cultured cells. Expression of each of a set of genes, such as the host cell's genome, can be individually systematically disrupted using RNA interference. Combination with alterations in oncogene and tumor suppressor pathways can be used to identify synthetic lethal interactions that may identify novel therapeutic targets.

In certain applications, the RNAi constructs can be fed directly to, or injected into, the cell/organism containing the target gene. Alternatively, the duplex RNA can be produced by in vivo or in vitro transcription from an expression construct used to produce the library. The construct can be replicated as individual clones of the library and transcribed to produce the RNA; each clone can then be fed to, injected into, or delivered by another method known in the art to,the cell/organism containing the target gene. The function of the target gene can be assayed from the effects it has on the cell/organism when gene activity is inhibited. This screening could be amenable to small subjects that can be processed in large number, for example, tissue culture cells derived from mammals, especially primates, and most preferably humans.

If a characteristic of an organism is determined to be genetically linked to a polymorphism through RFLP or QTL analysis, the present invention can be used to gain insight regarding whether that genetic polymorphism might be directly responsible for the characteristic. For example, a fragment defining the genetic polymorphism or sequences in the vicinity of such a genetic polymorphism can be amplified to produce an RNA, the duplex RNA can be introduced to the organism or cell, and whether an alteration in the characteristic is correlated with inhibition can be determined. Of course, there may be trivial explanations for negative results with this type of assay, for example: inhibition of the target gene causes lethality, inhibition of the target gene may not result in any observable alteration, the fragment contains nucleotide sequences that are not capable of inhibiting the target gene, or the target gene's activity is redundant.

The present invention may be useful in allowing the inhibition of essential genes. Such genes may be required for cell or organism viability at only particular stages of development or only in specific cellular compartments or tissues. The functional equivalent of conditional mutations may be produced by inhibiting activity of the target gene when or where it is not required for viability. The invention allows addition of RNA at specific times of development and locations in the organism without introducing permanent mutations into the target genome.

The present invention may be useful in allowing the inhibition of genes that have been difficult to inhibit using other methods due to gene redundancy. Since the present methods may be used to deliver more than one dsRNA to a cell or organism, dsRNA identical or similar to more than one gene, wherein the genes have a redundant function during normal development, may be delivered.

If alternative splicing produced a family of transcripts that were distinguished by usage of characteristic exons, the present invention can target inhibition through the appropriate exons to specifically inhibit or to distinguish among the functions of family members. For example, a protein factor that contained an alternatively spliced transmembrane domain may be expressed in both membrane bound and secreted forms. Instead of isolating a nonsense mutation that terminates translation before the transmembrane domain, the functional consequences of having only secreted hormone can be determined according to the invention by targeting the exon containing the transmembrane domain and thereby inhibiting expression of membrane-bound hormone. That is, the subject method can be used for selected ablation of splicing variants.

The present invention may be used alone or as a component of a kit having at least one of the reagents necessary to carry out the in vitro or in vivo introduction of RNA to test samples or subjects. Preferred components are the dsRNA and a vehicle that promotes introduction of the dsRNA. Such a kit may also include instructions to allow a user of the kit to practice the invention.

Alternatively, an organism may be engineered to produce dsRNA which produces commercially or medically beneficial results, for example, resistance to a pathogen or its pathogenic effects, improved growth, or novel developmental patterns.

### IV. Exemp/ification

The invention, now being generally described, will be more readily understood by reference to the following examples, which are included merely for purposes of illustration of certain aspects and embodiments of the present invention, as well as for background purposes (particularly in relation to non-intgregrating vectors) where appropriate and are not intended to limit the invention.

### Example 1: An RNA-Directed Nuclease Mediates RNAi Gene Silencing

In a diverse group of organisms that includes *Caenorhabditis elegans, Drosophila,* planaria, hydra, trypanosomes, fungi and plants, the introduction of double-stranded RNAs inhibits gene expression in a sequence-specific manner (Sharp (1999) Genes and Development 13: 139-141; Sanchez-Alvarado and Newmark (1999) PNAS 96: 5049-5054; Lohman et al. (1999) Developmental Biology 214: 211-214; Cogoni and Macino (1999) Nature 399: 166-169; Waterhouse et al. (1998) PNAS 95: 13959-13964; Montgomery and Fire (1998) Trends Genet, 14: 225-228; Ngo et al. (1998) PNAS 95: 14687-14692). These responses, called RNA interference or post-transcriptional gene silencing, may provide anti-viral defence, modulate transposition or regulate gene expression (Sharp (1999) Genes and Development 13: 139-141; Montgomery and Fire (1998) Trends Genet. 14: 225-228; Tabara et al. (1999) Cell 99: 123-132; Ketting et al. (1999) Cell 99: 133-141; Ratcliff et al. (1997) Science 276: 1559-1560). We have taken a biochemical approach towards elucidating the mechanisms underlying this genetic phenomenon. Here we show that 'loss-of-function' phenotypes can be created in cultured *Drosophila* cells by transfection with specific double-stranded RNAs. This coincides with a marked reduction in the level of cognate cellular messenger RNAs. Extracts of transfected cells contain a nuclease activity that specifically degrades exogenous transcripts homologous to transfected double-stranded RNA. This enzyme contains an essential RNA component. After partial purification, the sequence-specific nuclease co-fractionates with a discrete, ∼25-nucleotide RNA species which may confer specificity to the enzyme through homology to the substrate mRNAs.

Although double-stranded RNAs (dsRNAs) can provoke gene silencing in numerous biological contexts including *Drosophila* (Kennerdell et al. (1998) Cell 95: 1017-1026; Misquitta and Paterson (1999) PNAS 96: 1451-1456), the mechanisms underlying this phenomenon have remained mostly unknown. We therefore wanted to establish a biochemically tractable model in which such mechanisms could be investigated.

Transient transfection of cultured, *Drosophila* S2 cells with a *lacZ* expression vector resulted in β-galactosidase activity that was easily detectable by an *in situ* assay (Fig. 1a). This activity was greatly reduced by co-transfection with a dsRNA corresponding to the first 300 nucleotides of the *lacZ* sequence, whereas co-transfection with a control dsRNA *(CD8)* (Fig. 1a) or with single-stranded RNAs of either sense or antisense orientation (data not shown) had little or no effect. This indicated that dsRNAs could interfere, in a sequence-specific fashion, with gene expression in cultured cells.

To determine whether RNA interference (RNAi) could be used to target endogenous genes, we transfected S2 cells with a dsRNA corresponding to the first 540 nucleotides of *Drosophila cyclin E,* a gene that is essential for progression into S phase of the cell cycle. During log-phase growth, untreated S2 cells reside primarily in G2/M (Fig. 1b). Transfection with *lacZ* dsRNA had no effect on cell-cycle distribution, but transfection with the *cyclin E* dsRNA caused a G1-phase cell-cycle arrest (Fig. 1b). The ability of *cyclin E* dsRNA to provoke this response was length-dependent. Double-stranded RNAs of 540 and 400 nucleotides were quite effective, whereas dsRNAs of 200 and 300 nucleotides were less potent. Double-stranded *cyclin E* RNAs of 50 or 100 nucleotides were inert in our assay, and transfection with a single-stranded, antisense *cyclin E* RNA had virtually no effect.

One hallmark of RNAi is a reduction in the level of mRNAs that are homologous to the dsRNA. Cells transfected with the *cyclin E* dsRNA (bulk population) showed diminished endogenous *cyclin E* mRNA as compared with control cells (Fig. 1c). Similarly, transfection of cells with dsRNAs homologous to *fizzy,* a component of the anaphase-promoting complex (APC) or *cyclin A,* a cyclin that acts in S, G2 and M, also caused reduction of their cognate mRNAs (Fig.1c). The modest reduction in *fizzy* mRNA levels in cells transfected with *cyclin A* dsRNA probably resulted from arrest at a point in the division cycle at which *fizzy* transcription is low (Wolf and Jackson (1998) Current Biology 8: R637-R639; Kramer et al. (1998) Current Biology 8: 1207-1210). These results indicate that RNAi may be a generally applicable method for probing gene function in cultured *Drosophila* cells.

The decrease in mRNA levels observed upon transfection of specific dsRNAs into *Drosophila* cells could be explained by effects at transcriptional or post-transcriptional levels. Data from other systems have indicated that some elements of the dsRNA response may affect mRNA directly (reviewed in Sharp (1999) Genes and Development 13: 139-141; Montgomery and Fire (1998) Trends Genet. 14: 225-228). We therefore sought to develop a cell-free assay that reflected, at least in part, RNAi.

S2 cells were transfected with dsRNAs corresponding to either *cyclin E* or *lacZ.* Cellular extracts were incubated with synthetic mRNAs of *lacZ* or *cyclin E.* Extracts prepared from cells transfected with the 540-nucleotide *cyclin E* dsRNA efficiently degraded the *cyclin E* transcript; however, the *lacZ* transcript was stable in these lysates (Fig. 2a). Conversely, lysates from cells transfected with the *lacZ* dsRNA degraded the *lacZ* transcript but left the *cyclin E* mRNA intact. These results indicate that RNAi ablates target mRNAs through the generation of a sequence-specific nuclease activity. We have termed this enzyme RISC (RNA-induced silencing complex). Although we occasionally observed possible intermediates in the degradation process (see Fig. 2), the absence of stable cleavage end-products indicates an exonuclease (perhaps coupled to an endonuclease). However, it is possible that the RNAi nuclease makes an initial endonucleolytic cut and that non-specific exonucleases in the extract complete the degradation process (Shuttleworth and Colman (1988) EMBO J. 7: 427-434). In addition, our ability to create an extract that targets *lacZ in vitro* indicates that the presence of an endogenous gene is not required for the RNAi response.

To examine the substrate requirements for the dsRNA-induced, sequence-specific nuclease activity, we incubated a variety of *cyclin-E*-derived transcripts with an extract derived from cells that had been transfected with the 540-nucleotide *cyclin E* dsRNA (Fig. 2b, c). Just as a length requirement was observed for the transfected dsRNA, the RNAi nuclease activity showed a dependence on the size of the RNA substrate. Both a 600-nucleotide transcript that extends slightly beyond the targeted region (Fig. 2b) and an ∼1-kilobase (kb) transcript that contains the entire coding sequence (data not shown) were completely destroyed by the extract. Surprisingly, shorter substrates were not degraded as efficiently. Reduced activity was observed against either a 300- or a 220-nucleotide transcript, and a 100-nucleotide transcript was resistant to nuclease in our assay. This was not due solely to position effects because -100-nucleotide transcripts derived from other portions of the transfected dsRNA behaved similarly (data not shown). As expected, the nuclease activity (or activities) present in the extract could also recognize the antisense strand of the *cyclin E* mRNA. Again, substrates that contained a substantial portion of the targeted region were degraded efficiently whereas those that contained a shorter stretch of homologous sequence (∼130 nucleotides) were recognized inefficiently (Fig. 2c, as600). For both the sense and antisense strands, transcripts that had no homology with the transfected dsRNA ( Fig. 2b, Eout; Fig. 2c, as300) were not degraded. Although we cannot exclude the possibility that nuclease specificity could have migrated beyond the targeted region, the resistance of transcripts that do not contain homology to the dsRNA is consistent with data from *C. elegans.* Double-stranded RNAs homologous to an upstream cistron have little or no effect on a linked downstream cistron, despite the fact that unprocessed, polycistronic mRNAs can be readily detected (Tabara et al. (1998) Science 282: 430-432; Bosher et al. (1999) Genetics 153: 1245-1256). Furthermore, the nuclease was inactive against a dsRNA identical to that used to provoke the RNAi response *in vivo* (Fig. 2b). In the *in vitro* system, neither a 5' cap nor a poly(A) tail was required, as such transcripts were degraded as efficiently as uncapped and non-polyadenylated RNAs.

Gene silencing provoked by dsRNA is sequence specific. A plausible mechanism for determining specificity would be incorporation of nucleic-acid guide sequences into the complexes that accomplish silencing (Hamilton and Baulcombe (1999) Science 286: 950-952). In accord with this idea, pre-treatment of extracts with a Ca²⁺-dependent nuclease (micrococcal nuclease) abolished the ability of these extracts to degrade cognate mRNAs (Fig. 3). Activity could not be rescued by addition of non-specific RNAs such as yeast transfer RNA. Although micrococcal nuclease can degrade both DNA and RNA, treatment of the extract with DNAse I had no effect (Fig. 3). Sequence-specific nuclease activity, however, did require protein (data not shown). Together, our results support the possibility that the RNAi nuclease is a ribonucleoprotein, requiring both RNA and protein components. Biochemical fractionation (see below) is consistent with these components being associated in extract rather than being assembled on the target mRNA after its addition.

In plants, the phenomenon of co-suppression has been associated with the existence of small (∼25-nucleotide) RNAs that correspond to the gene that is being silenced (Hamilton and Baulcombe (1999) Science 286: 950-952). To address the possibility that a similar RNA might exist in *Drosophila* and guide the sequence-specific nuclease in the choice of substrate, we partially purified our activity through several fractionation steps. Crude extracts contained both sequence-specific nuclease activity and abundant, heterogeneous RNAs homologous to the transfected dsRNA (Figs 2 and 4a). The RNAi nuclease fractionated with ribosomes in a high-speed centrifugation step. Activity could be extracted by treatment with high salt, and ribosomes could be removed by an additional centrifugation step. Chromatography of soluble nuclease over an anion-exchange column resulted in a discrete peak of activity (Fig. 4b, *cyclin E*). This retained specificity as it was inactive against a heterologous mRNA (Fig. 4b, *lacZ*). Active fractions also contained an RNA species of 25 nucleotides that is homologous to the *cyclin E* target (Fig. 4b, northern). The band observed on northern blots may represent a family of discrete RNAs because it could be detected with probes specific for both the sense and antisense *cyclin E* sequences and with probes derived from distinct segments of the dsRNA (data not shown). At present, we cannot determine whether the 25-nucleotide RNA is present in the nuclease complex in a double-stranded or single-stranded form.

RNA interference allows an adaptive defence against both exogenous and endogenous dsRNAs, providing something akin to a dsRNA immune response. Our data, and that of others (Hamilton and Baulcombe (1999) Science 286: 950-952), is consistent with a model in which dsRNAs present in a cell are converted, either through processing or replication, into small specificity determinants of discrete size in a manner analogous to antigen processing. Our results suggest that the post-transcriptional component of dsRNA-dependent gene silencing is accomplished by a sequence-specific nuclease that incorporates these small RNAs as guides that target specific messages based upon sequence recognition. The identical size of putative specificity determinants in plants (Hamilton and Baulcombe (1999) Science 286: 950-952) and animals predicts a conservation of both the mechanisms and the components of dsRNA-induced, post-transcriptional gene silencing in diverse organisms. In plants, dsRNAs provoke not only post-transcriptional gene silencing but also chromatin remodelling and transcriptional repression (Jones et al. (1998) EMBO J. 17: 6385-6393; Jones et al. (1999) Plant Cell 11: 2291-2301). It is now critical to determine whether conservation of gene-silencing mechanisms also exists at the transcriptional level and whether chromatin remodelling can be directed in a sequence-specific fashion by these same dsRNA-derived guide sequences.

### Methods:

**Cell culture and RNA methods S2** cells (Schneider (1972) J. Embryol Exp Morpho 27: 353-365) were cultured at 27 °C in 90% Schneider's insect media (Sigma), 10% heat inactivated fetal bovine serum (FBS). Cells were transfected with dsRNA and plasmid DNA by calcium phosphate co-precipitation (DiNocera and Dawid (1983) PNAS 80: 7095-7098). Identical results were observed when cells were transfected using lipid reagents (for example, Superfect, Qiagen). For FACS analysis, cells were additionally transfected with a vector that directs expression of a green fluorescent protein (GFP)-US9 fusion protein (Kalejta et al. (1999) Exp Cell Res. 248: 322-328). These cells were fixed in 90% ice-cold ethanol and stained with propidium iodide at 25 µg/ml. FACS was performed on an Elite flow cytometer (Coulter). For northern blotting, equal loading was ensured by over-probing blots with a control complementary DNA (RP49). For the production of dsRNA, transcription templates were generated by polymerase chain reaction such that they contained T7 promoter sequences on each end of the template. RNA was prepared using the RiboMax kit (Promega). Confirmation that RNAs were double stranded came from their complete sensitivity to RNAse III (a gift from A. Nicholson). Target mRNA transcripts were synthesized using the Riboprobe kit (Promega) and were gel purified before use.

**Extract preparation** Log-phase S2 cells were plated on 15-cm tissue culture dishes and transfected with 30 µg dsRNA and 30 µg carrier plasmid DNA. Seventy-two hours after transfection, cells were harvested in PBS containing 5 mM EGTA, washed twice in PBS and once in hypotonic buffer (10 mM HEPES pH 7.3, 6 mM β-mercaptoethanol). Cells were suspended in 0.7 packed-cell volumes of hypotonic buffer containing *Complete* protease inhibitors (Boehringer) and 0.5 units/ml of RNasin (Promega). Cells were disrupted in a dounce homogenizer with a type B pestle, and lysates were centrifuged at 30,000g for 20 min. Supernatants were used in an *in vitro* assay containing 20 mM HEPES pH 7.3, 110 mM KOAc, 1 mM Mg(OAc)₂, 3 mM EGTA, 2 mM CaCl₂, 1 mM DTT. Typically, 5 µl extract was used in a 10 µl assay that contained also 10,000 c.p.m. synthetic mRNA substrate.

**Extract fractionation** Extracts were centrifuged at 200,000g for 3 h and the resulting pellet (containing ribosomes) was extracted in hypotonic buffer containing also 1 mM MgCl₂ and 300 mM KOAc. The extracted material was spun at 100,000g for 1 h and the resulting supernatant was fractionated on Source 15Q column (Pharmacia) using a KCl gradient in buffer A (20 mM HEPES pH 7.0, 1 mM dithiothreitol, 1 mM MgCl₂). Fractions were assayed for nuclease activity as described above. For northern blotting, fractions were proteinase K/SDS treated, phenol extracted, and resolved on 15% acrylamide 8M urea gels. RNA was electroblotted onto Hybond N+ and probed with strand-specific riboprobes derived from cyclin E mRNA. Hybridization was carried out in 500 mM NaPO₄ pH 7.0, 15% formamide, 7% SDS, 1% BSA. Blots were washed in 1X SSC at 37-45 °C.

### Example 2: Role for a Bidentate Ribonuclease in the Initiation Step of RNA Interference

Genetic approaches in worms, fungi and plants have identified a group of proteins that are essential for double-stranded RNA-induced gene silencing. Among these are ARGONAUTE family members (e.g. RDE1, QDE2) (Tabara et al. (1999) Cell 99: 123-132; Catalanotto et al. (2000) Nature 404: 245; Fagard et al. (2000) PNAS 97: 11650-11654), recQ-family helicases (MUT-7, QDE3) (Ketting et al. (1999) Cell 99: 133-141; Cogoni and Macino. (1999) Science 286: 2342-2344), and RNA-dependent RNA polymerases (e.g. EGO-1, QDE1, SGS2/SDE1) (Cogoni and Macino (1999) Nature 399: 166-169; Smardon et al. (2000) Current Biology 10: 169-178; Mourrain et al. (2000) Cell 101: 533-542; Dalmay et al. (2000) Cell 101: 543-553). While potential roles have been proposed, none of these genes has been assigned a definitive function in the silencing process. Biochemical studies have suggested that PTGS is accomplished by a multicomponent nuclease that targets mRNAs for degradation (Hammond et al. (2000) Nature 404: 293-296; Zamore et al. (2000) Cell 101 25-33; Tuschl et al. (1999) Genes and Development 13: 3191-3197). We have shown that the specificity of this complex may derive from the incorporation of a small guide sequence that is homologous to the mRNA substrate (Hammond et al. (2000) Nature 404: 293-296). Originally identified in plants that were actively silencing transgenes (Hamilton and Baulcombe (1999) Science 286: 950-952), these ∼22 nt. RNAs have been produced during RNAi *in vitro* using an extract prepared from *Drosophila* embryos (Zamore et al. (2000) Cell 101 25-33). Putative guide RNAs can also be produced in extracts from *Drosophila* S2 cells (Fig. 5a). With the goal of understanding the mechanism of post-transcriptional gene silencing, we have undertaken both biochemical fractionation and candidate gene approaches to identify the enzymes that execute each step of RNAi.

Our previous studies resulted in the partial purification of a nuclease, RISC, that is an effector of RNA interference. See Example 1. This enzyme was isolated from *Drosophila* S2 cells in which RNAi had been initiated *in vivo* by transfection with dsRNA. We first sought to determine whether the RISC enzyme and the enzyme that initiates RNAi via processing of dsRNA into 22mers are distinct activities. RISC activity could be largely cleared from extracts by high-speed centrifugation (100,000xg for 60 min.) while the activity that produces 22mers remained in the supernatant (Fig. 5b,c). This simple fractionation indicated that RISC and the 22mer-generating activity are separable and thus distinct enzymes. However, it seems likely that they might interact at some point during the silencing process.

RNAse III family members are among the few nucleases that show specificity for double-stranded RNA (Nicholson (1999) FEMS Microbiol Rev 23: 371-390). Analysis of the *Drosophila* and *C. elegans* genomes reveals several types of RNAse III enzymes. First is the canonical RNAse III which contains a single RNAse III signature motif and a double-stranded RNA binding domain (dsRBD; e.g. RNC_CAEEL). Second is a class represented by Drosha (Filippov et al. (2000) Gene 245: 213-221), a *Drosophila* enzyme that contains two RNAse III motifs and a dsRBD (CeDrosha in *C. elegans*). A third class contains two RNAse III signatures and an amino terminal helicase domain (e.g. *Drosophila* CG4792, CG6493, *C*. *elegans* K12H4.8), and these had previously been proposed by Bass as candidate RNAi nucleases (Bass (2000) Cell 101: 235-238). Representatives of all three classes were tested for the ability to produce discrete, ∼22 nt. RNAs from dsRNA substrates.

Partial digestion of a 500 nt. cyclin E dsRNA with purified, bacterial RNAse III produced a smear of products while nearly complete digestion produced a heterogeneous group of ∼11-17 nucleotide RNAs (not shown). In order to test the dual-RNAse III enzymes, we prepared T7 epitope-tagged versions of Drosha and CG4792. These were expressed in transfected S2 cells and isolated by immunoprecipitation using antibody-agarose conjugates. Treatment of the dsRNA with the CG4792 immunoprecipitate yielded ∼22 nt. fragments similar to those produced in either S2 or embryo extracts (Fig. 6a). Neither activity in extract nor activity in immunoprecipitates depended on the sequence of the RNA substrate since dsRNAs derived from several genes were processed equivalently (see Supplement 1). Negative results were obtained with Drosha and with immunoprecipitates of a DExH box helicase (Homeless (Gillespie et al. (1995) Genes and Development 9: 2495-2508); see Fig 6a,b). Western blotting confirmed that each of the tagged proteins was expressed and immunoprecipitated similarly (see Supplement 2). Thus, we conclude that CG4792 may carry out the initiation step of RNA interference by producing ∼22 nt. guide sequences from dsRNAs. Because of its ability to digest dsRNA into uniformly sized, small RNAs, we have named this enzyme Dicer (*Dcr*). *Dicer* mRNA is expressed in embryos, in S2 cells, and in adult flies, consistent with the presence of functional RNAi machinery in all of these contexts (see Supplement 3).

The possibility that Dicer might be the nuclease responsible for the production of guide RNAs from dsRNAs prompted us to raise an antiserum directed against the carboxy-terminus of the Dicer protein (Dicer-1, CG4792). This antiserum could immunoprecipitate a nuclease activity from either Drosophila embryo extracts or from S2 cell lysates that produced ∼22 nt. RNAs from dsRNA substrates (Fig. 6C). The putative guide RNAs that are produced by the Dicer-1 enzyme precisely comigrate with 22mers that are produced in extract and with 22mers that are associated with the RISC enzyme (Fig. 6D,F). It had previously been shown that the enzyme that produced guide RNAs in Drosophila embryo extracts was ATP-dependent (Zamore et al. (2000) Cell 101 25-33). Depletion of this cofactor resulted in an ∼6-food lower rate of dsRNA cleavage and in the production of RNAs with a slightly lower mobility. Of interest was the fact that both Dicer-1 immunoprecipitates and extracts from S2 cells require ATP for the production of ∼22mers (Fig. 6D). We do not observe the accumulation of lower mobility products in these cases, although we do routinely observe these in ATP-depleted embryo extracts. The requirement of this nuclease for ATP is a quite unusual property. We hypothesize that this requirement could indicate that the enzyme may act processively on the dsRNA, with the helicase domain harnessing the energy of ATP hydrolysis both for unwinding guide RNAs and for translocation along the substrate.

Efficient induction of RNA interference in *C. elegans* and in *Drosophila* has several requirements. For example, the initiating RNA must be double-stranded, and it must be several hundred nucleotides in length. To determine whether these requirements are dictated by Dicer, we characterized the ability of extracts and of immunoprecipitated enzyme to digest various RNA substrates. Dicer was inactive against single stranded RNAs regardless of length (see Supplement 4). The enyzme could digest both 200 and 500 nucleotide dsRNAs but was significantly less active with shorter substrates (see Supplement 4). Double-stranded RNAs as short as 35 nucleotides could be cut by the enzyme, albeit very inefficiently (data not shown). In contrast, E. coli RNAse III could digest to completion dsRNAs of 35 or 22 nucleotides (not shown). This suggests that the substrate preferences of the Dicer enzyme may contribute to but not wholly determine the size dependence of RNAi.

To determine whether the Dicer enzyme indeed played a role in RNAi *in vivo*, we sought to deplete Dicer activity from S2 cells and test the effect on dsRNA-induced gene silencing. Transfection of S2 cells with a mixture of dsRNAs homologous to the two Drosophila Dicer genes (CG4792 and CG6493) resulted in an ∼6-7 fold reduction of Dicer activity either in whole cell lysates or in Dicer-1 immunoprecipitates (Fig. 7A,B). Transfection with a control dsRNA (murine caspase 9) had no effect. Qualitatively similar results were seen if Dicer was examined by Northern blotting (not shown). Depletion of Dicer in this manner substantially compromised the ability of cells to silence subsequently an exogenous, GFP transgene by RNAi (Fig. 7C). These results indicate that Dicer is involved in RNAi *in vivo.* The lack of complete inhibition of silencing could result from an incomplete suppression of Dicer (which is itself required for RNAi) or could indicate that *in vivo,* guide RNAs can be produced by more than one mechanism (e.g. through the action of RNA-dependent RNA polymerases).

Our results indicate that the process of RNA interference can be divided into at least two distinct steps. According to this model, initiation of PTGS would occur upon processing of a double-stranded RNA by Dicer into ∼22 nucleotide guide sequences, although we cannot formally exclude the possibility that another, Dicer-associated nuclease may participate in this process. These guide RNAs would be incorporated into a distinct nuclease complex (RISC) that targets single-stranded mRNAs for degradation. An implication of this model is that guide sequences are themselves derived directly from the dsRNA that triggers the response. In accord with this model, we have demonstrated that ³²P-labeled, exogenous dsRNAs that have been introduced into S2 cells by transfection are incorporated into the RISC enzyme as 22 mers (Fig. 7E). However, we cannot exclude the possibility that RNA-dependent RNA polymerases might amplify 22mers once they have been generated or provide an alternative method for producing guide RNAs.

The structure of the Dicer enzyme provokes speculation on the mechanism by which the enzyme might produce discretely sized fragments irrespective of the sequence of the dsRNA (see Supplement 1, Fig. 8a). It has been established that bacterial RNAse III acts on its substrate as a dimer (Nicholson (1999) FEMS Microbiol Rev 23: 371-390; Robertson et al. (1968) J Biol Chem 243: 82-91; Dunn (1976) J Biol Chem 251: 3807-3814). Similarly, a dimer of Dicer enzymes may be required for cleavage of dsRNAs into ∼22 nt. pieces. According to one model, the cleavage interval would be determined by the physical arrangement of the two RNAse III domains within Dicer enzyme (Fig. 8a). A plausible alternative model would dictate that cleavage was directed at a single position by the two RIII domains in a single Dicer protein. The 22 nucleotide interval could be dictated by interaction of neighboring Dicer enzymes or by translocation along the mRNA substrate. The presence of an integral helicase domain suggests that the products of Dicer cleavage might be single-stranded 22 mers that are incorporated into the RISC enzyme as such.

A notable feature of the Dicer family is its evolutionary conservation. Homologs are found in *C. elegans* (K12H4.8), *Arabidopsis* (e.g., CARPEL FACTORY (Jacobson et al. (1999) Development 126: 5231-5243), T25K16.4, AC012328_1), mammals (Helicase-MOI (Matsuda et al. (2000) Biochim Biophys Acta 1490: 163-169) and *S. pombe* (YC9A_SCHPO) (Fig 8b, see Supplements 6,7 for sequence comparisons). In fact, the human Dicer family member is capable of generating ∼22 nt. RNAs from dsRNA substrates (Supplement 5) suggesting that these structurally similar proteins may all share similar biochemical functions. It has been demonstrated that exogenous dsRNAs can affect gene function in early mouse embryos (Wianny et al. (2000) Nature Cell Biology 2: 70-75), and our results suggest that this regulation may be accomplished by an evolutionarily conserved RNAi machinery.

In addition to RNAseIII and helicase motifs, searches of the PFAM database indicate that each Dicer family member also contains a ZAP domain (Fig 8c) (Sonnhammer et al. (1997) Proteins 28: 405-420). This sequence was defined based solely upon its conservation in the Zwille/ARGONAUTE/Piwi family that has been implicated in RNAi by mutations *in C. elegans* (Rde-1) and *Neurospora* (Qde-2) (Tabara et al. (1999) Cell 99: 123-132; Catalanotto et al (2000) Nature 404: 245). Although the function of this domain is unknown, it is intriguing that this region of homology is restricted to two gene families that participate in dsRNA-dependent silencing. Both the ARGONAUTE and Dicer families have also been implicated in common biological processes, namely the determination of stem-cell fates. A hypomorphic allele of *carpel factory,* a member of the Dicer family in *Arabidopsis,* is characterized by increased proliferation in floral meristems (Jacobsen et al. (1999) Development 126 5231-5243). This phenotype and a number of other characteristic features are also shared *by Arabidopsis ARGONAUTE (agol-1)* mutants (Bohmert et al. (1998) EMBO J 17: 170-180; C. Kidner and R. Martiennsen, pers. comm.). These genetic analyses begin to provide evidence that RNAi may be more than a defensive response to unusual RNAs but may also play important roles in the regulation of endogenous genes.

With the identification of Dicer as a catalyst of the initiation step of RNAi, we have begun to unravel the biochemical basis of this unusual mechanism of gene regulation. It will be of critical importance to determine whether the conserved family members from other organisms, particularly mammals, also play a role in dsRNA-mediated gene regulation.

### Methods:

**Plasmid constructs.** A full-length cDNA encoding Drosha was obtained by PCR from an EST sequenced by the Berkeley Drosophila genome project. The *Homeless* clone was a gift from Gillespie and Berg (Univ. Washington). The T7 epitope-tag was added to the amino terminus of each by PCR, and the tagged cDNAs were cloned into pRIP, a retroviral vector designed specifically for expression in insect cells (E. Bernstein, unpublished). In this vector, expression is driven by the *Orgyia pseudotsugata* IE2 promoter (Invitrogen). Since no cDNA was available for CG4792/Dicer, a genomic clone was amplified from a bacmid (BACR23F10; obtained from the BACPAC Resource Center in the Dept. of Human Genetics at the Roswell Park Cancer Institute). Again, during amplification, a T7 epitope tag was added at the amino terminus of the coding sequence. The human Dicer gene was isolated from a cDNA library prepared from HaCaT cells (GJH, unpublished). A T7-tagged version of the complete coding sequence was cloned into pCDNA3 (Invitrogen) for expression in human cells (LinX-A).

**Cell culture and extract preparation**. *S2 and embryo culture.* S2 cells were cultured at 27°C in 5% CO₂ in Schneider's insect media supplemented with 10% heat inactivated fetal bovine serum (Gemini) and 1% antibiotic-antimycotic solution (Gibco BRL). Cells were harvested for extract preparation at 10x10⁶ cells/ml. The cells were washed 1X in PBS and were resuspended in a hypotonic buffer (10 mM Hepes pH 7.0, 2 mM MgCl₂, 6 mM βME) and dounced. Cell lysates were spun 20,000xg for 20 minutes. Extracts were stored at -80°C. *Drosophila* embryos were reared in fly cages by standard methodologies and were collected every 12 hours. The embryos were dechorionated in 50% chlorox bleach and washed thoroughly with distilled water. Lysis buffer (10 mM Hepes, 10 mM KCl, 1.5 mM MgCl₂, 0.5 mM EGTA, 10 mM β-glycerophosphate, 1 mM DTT, 0.2 mM PMSF) was added to the embryos, and extracts were prepared by homogenization in a tissue grinder. Lysates were spun for two hours at 200,000xg and were frozen at - 80°C. LinX-A cells, a highly-transfectable derivative of human 293 cells, (Lin Xie and GJH, unpublished) were maintained in DMEM/10% FCS.

**Transfections and immunoprecipitations.** S2 cells were transfected using a calcium phosphate procedure essentially as previously described (Hammond et al. (2000) Nature 404: 293-296). Transfection rates were ∼90% as monitored in controls using an *in situ* β-galactosidase assay. LinX-A cells were also transfected by calcium phosphate co-precipitation. For immunoprecipitations, cells (∼ 5x10⁶ per IP) were transfected with various clones and lysed three days later in IP buffer (125 mM KOAc, 1 mM MgOAc, 1 mM CaCl₂, 5 mM EGTA, 20 mM Hepes pH 7.0, 1 mM DTT, 1% NP-40 plus Complete protease inhibitors (Roche)). Lysates were spun for 10 minutes at 14,000xg and supernatants were added to T7 antibody-agarose beads (Novagen). Antibody binding proceeded for 4 hours at 4°C. Beads were centrifuged and washed in lysis buffer three times, and once in reaction buffer. The Dicer antiserum was raised in rabbits using a KLH-conjugated peptide corresponding to the C-terminal 8 amino acids of Drosophila Dicer-1 (CG4792).

**Cleavage reactions.** *RNA preparation.* Templates to be transcribed into dsRNA were generated by PCR with forward and reverse primers, each containing a T7 promoter sequence. RNAs were produced using Riboprobe (Promega) kits and were uniformly labeling during the transcription reaction with ³²P-UTP. Single-stranded RNAs were purified from 1% agarose gels. *dsRNA cleavage.* Five microliters of embryo or S2 extracts were incubated for one hour at 30°C with dsRNA in a reaction containing 20 mM Hepes pH 7.0, 2 mM MgOAc, 2 mM DTT, 1 mM ATP and 5% Superasin (Ambion). Immunoprecipitates were treated similarly except that a minimal volume of reaction buffer (including ATP and Superasin) and dsRNA were added to beads that had been washed in reaction buffer (see above).' For ATP depletion, *Drosophila* embryo extracts were incubated for 20 minutes at 30°C with 2mM glucose and 0.375 U of hexokinase (Roche) prior to the addition of dsRNA.

**Northern and Western analysis.** Total RNA was prepared from *Drosophila* embryos (0-12 hour), from adult flies, and from S2 cells using Trizol (Lifetech). Messenger RNA was isolated by affinity selection using magnetic oligo-dT beads (Dynal). RNAs were electrophoresed on denaturing formaldehyde/agarose gels, blotted and probed with randomly primed DNAs corresponding to Dicer. For Western analysis, T7-tagged proteins were immunoprecipitated from whole cell lysates in IP buffer using anti-T7-antibody-agarose conjugates. Proteins were released from the beads by boiling in Laemmli buffer and were separated by electrophoresis on 8% SDS PAGE. Following transfer to nitrocellulose, proteins were visualized using an HRP-conjugated anti-T7 antibody (Novagen) and chemiluminescent detection (Supersignal, Pierce).

**RNAi of Dicer.** Drosophila S2 cells were transfected either with a dsRNA corresponding to mouse caspase 9 or with a mixture of two dsRNAs corresponding to Drosophila Dicer-1 and Dicer-2 (CG4792 and CG6493). Two days after the initial transfection, cells were again transfected with a mixture containing a GFP expression plasmid and either luciferase dsRNA or GFP dsRNA as previously described (Hammond et al. (2000) Nature 404: 293-296). Cells were assayed for Dicer activity or fluorescence three days after the second transfection. Quantification of fluorescent cells was done on a Coulter EPICS cell sorter after fixation. Control transfections indicated that Dicer activity was not affected by the introduction of caspase 9 dsRNA.

### Example 3: A Simplified Method for the Creation of Hairpin Constructs for RNA Interference.

In numerous model organisms, double stranded RNAs have been shown to cause effective and specific suppression of gene function (Bosher and Labouesse (2000) Nature Cell Biology 2: E31-E36). This response, termed RNA interference or post-transcriptional gene silencing, has evolved into a highly effective reverse genetic tool in *C*. *elegans, Drosophila,* plants and numerous other systems. In these cases, double-stranded RNAs can be introduced by injection, transfection or feeding; however, in all cases, the response is both transient and systemic. Recently, stable interference with gene expression has been achieved by expression of RNAs that form snap-back or hairpin structures (Fortier and Belote (2000) Genesis 26: 240-244; Kennerdell and Carthew (2000) Nature Biotechnology 18: 896-898; Lam and Thummel (2000) Current Biology 10: 957-963; Shi et al. (2000) RNA 6: 1069-1076; Smith et al. (2000) Nature 407: 319-320; Tavemarakis et al. (2000) Nature Genetics 24: 180-183). This has the potential not only to allow stable silencing of gene expression but also inducible silencing as has been observed in trypanosomes and adult *Drosophila* (Fortier and Belote (2000) Genesis 26: 240-244; Lam and Thummel (2000) Current Biology 10: 957-963; Shi et al. (2000) RNA 6: 1069-1076). The utility of this approach is somewhat hampered by the difficulties that arise in the construction of bacterial plasmids containing the long inverted repeats that are necessary to provoke silencing. In a recent report, it was stated that more than 1,000 putative clones were screened to identify the desired construct (Tavemarakis et al. (2000) Nature Genetics 24: 180-183).

The presence of hairpin structures often induces plasmid rearrangement, in part due to the E. coli sbc proteins that recognize and cleave cruciform DNA structures (Connelly et al. (1996) Genes Cell 1: 285-291). We have developed a method for the construction of hairpins that does not require cloning of inverted repeats, per se. Instead, the fragment of the gene that is to be silenced is cloned as a direct repeat, and the inversion is accomplished by treatment with a site-specific recombinase, either *in vitro* (or potentially *in vivo*) (see Fig 27). Following recombination, the inverted repeat structure is stable in a bacterial strain that lacks an intact SBC system (DL759). We have successfully used this strategy to construct numerous hairpin expression constructs that have been successfully used to provoke gene silencing in Drosophila cells.

In the following examples, we use this method to express long dsRNAs in a variety of mammalian cell types. We show that such long dsRNAs mediate RNAi in a variety of cell types. Additionally, since the vector described in Figure 27 contains a selectable marker, dsRNAs produced in this manner can be stably expressed in cells. Accordingly, this method allows not only the examination of transient effects of RNA suppression in a cell, but also the effects of stable and prolonged RNA suppression.

### Methods:

Plasmids expressing hairpin RNAs were constructed by cloning the first 500 basepairs of the GFP coding region into the FLIP cassette of pRIP-FLIP as a direct repeat. The FLIP cassette contains two directional cloning sites, the second of which is flanked by LoxP sites. The Zeocin gene, present between the cloning sites, maintains selection and stability. To create an inverted repeat for hairpin production, the direct repeat clones were exposed to Cre recombinase (Stratagene) in vitro and, afterwards, transformed into DL759 E.Coli. These bacteria permit the replication of DNA containing cruciform structures, which tend to form inverted repeats.

### Example 4: Long dsRNAs Suppress Gene Expression in Mammalian Cells

Previous experiments have demonstrated that dsRNA, produced using a variety of methods including via the construction of hairpins, can suppress gene expression in Drosophila cells. We now demonstrate that dsRNA can also suppress gene expression in mammalian cells in culture. Additionally, the power of RNAi as a genetic tool would be greatly enhanced by the ability to engineer stable silencing of gene expression. We therefore undertook an effort to identify mammalian cells in which long dsRNAs could be used as RNAi triggers in the hope that these same cell lines would provide a platform upon which to develop stable silencing strategies. We demonstrate that RNA suppression can be mediated by stably expressing a long hairpin in a mammalian cell line. The ability to engineer stable silencing of gene expression in cultured mammalian cells, in addition to the ability to transiently silence gene expression, has many important applications.

### A. RNAi in Pluripotent Murine P19 Cells.

We first sought to determine whether long dsRNA triggers could induce sequence-specific silencing in cultured murine cells, both to develop this approach as a tool for probing gene function and to allow mechanistic studies of dsRNA-induced silencing to be propagated to mammalian systems. We, therefore, attempted to extend previous studies in mouse embryos (Wianny et al. 2000, Nat. Cell Biol. 2: 70-75; Svoboda et al. 2000, Development 127: 4147-4156) by searching for RNAi-like mechanisms in pluripotent, embryonic cell types. We surveyed a number of cell lines of embryonic origin for the degree to which generalized suppression of gene expression occurred upon introduction of dsRNA. As an assay, we tested the effects of dsRNA on the expression of GFP as measured *in situ* by counting fluorescent cells. As expected, in both human embryonic kidney cells (293) and mouse embryo fibroblasts, GFP expression was virtually eliminated irrespective of the sequence of the cotransfected dsRNA. In some pluripotent teratocarcinoma and teratoma cell lines (e.g., N-Teral, F9), the PKR response was attenuated but still evident; however, in contrast, transfection of nonhomologous dsRNAs had no effect on the expression of reporter genes (e.g., GFP or luciferase) either in mouse embryonic stem cells or in p19 embryonal carcinoma cells (Fig. 28).

Transfection of P 19 embryonal carcinoma cells with GFP in the presence of cognate dsRNA corresponding to the first ≈500 nts of the GFP coding sequence had a strikingly different effect. GFP expression was eliminated in the vast majority of cotransfected cells (Fig. 28), suggesting that these cultured murine cells might respond to dsRNA in a manner similar to that which we had previously demonstrated in cultured, *Drosophila* S2 cells (Hammond et al. 2000, Nature 404: 293-296).

To quantify the extent to which dsRNA could induce sequence-specific gene silencing, we used a dual luciferase reporter assay similar to that which had first been used to demonstrate RNAi in *Drosophila* embryo extracts (Tuschl et al. 1999, Genes Dev. 13: 3191-3197). P19 EC cells were transfected with a mixture of two plasmids that individually direct the expression of firefly luciferase and *Renilla* luciferase. These were cotransfected with no dsRNA, with dsRNA that corresponds to the first ≈500 nts of the firefly luciferase, or with dsRNA corresponding to the first ≈500 nts of GFP as a control. Cotransfection with GFP dsRNA gave luciferase activities that were similar to the no-dsRNA control, both in the firefly/*Renilla* activity ratio and in the absolute values of both activities. In contrast, in cells that received the firefly luciferase dsRNA, the ratio of firefly to *Renilla* luciferase activity was reduced by up to 30-fold (250 ng, Fig. 29B). For comparison, we carried out an identical set of experiments in *Drosophila* S2 cells. Although qualitatively similar results were obtained, the silencing response was more potent. At equivalent levels of dsRNA, S2 cells suppressed firefly luciferase activity to virtually background levels.

The complementary experiment, in which dsRNA was homologous to *Renilla* luciferase, was also performed. Again, in this case, suppression of the expression of the *Renilla* enzyme was ≈10-fold (Fig. 29D). Thus, the dsRNA response in P19 cells was flexible, and the silencing machinery was able to adapt to dsRNAs directed against any of the reporters that were tested.

We took two approaches to test whether this response was specific for dsRNA. Pretreatment of the trigger with purified RNase III, a dsRNA-specific ribonuclease, before transfection greatly reduced its ability to provoke silencing. Furthermore, transfection of cells with single-stranded antisense RNAs directed against either firefly or *Renilla* luciferase had little or no effect on expression of the reporters (Fig. 29C and 29D). Considered together, these results provided a strong indication that double-stranded RNAs provoke a potent and specific silencing response in P19 embryonal carcinoma cells. Efficient silencing could be provoked with relatively low concentrations of dsRNA (25 ng/ml culture media; see Fig. 29A). The response was concentration-dependent, with maximal suppression of ≈20-fold being achieved at a dose of 1.5 µg/ml culture media. Silencing was established rapidly and was evident by 9 h posttransfection (the earliest time point examined). Furthermore, the response persisted without significant changes in the degree of suppression for up to 72 h following a single dose of dsRNA.

Figure 30 further shows wildtype P19 cells which have been co-transfected with either RFP or GFP (right panel). Note the robust expression of RFP or GFR respectively approximately 42 hours post-transfection. We isolated P 19 clones which stably express a 500 nt. GFP hairpin. Such clones were then transfected with either RFP or GFP, and expression of RFP or GFP was assessed by visual inspection of the cells. The left panel demonstrates that a 500 nt GFP hairpin specifically suppresses expression of GFP in P19 cells.

### B. RNAi in Embryonic Stem Cells.

To assess whether the presence of a sequence-specific response to dsRNA was a peculiarity of P19 cells or whether it also extended to normal murine embryonic cells, we performed similar silencing assays in mouse embryonic stem cells. Cotransfection of embryonic stem cells with noncognate dsRNAs (e.g., GFP), again, had no dramatic effect on either the absolute values or the ratios of *Renilla* and firefly luciferase activity (Fig. 31). However, transfection with either firefly or *Renilla* luciferase dsRNA dramatically and specifically reduced the activity of the targeted enzyme (Fig. 31).

This result suggests that RNAi can operate in multiple murine cell types of embryonic origin, including normal embryonic stem cells. The ability to provoke silencing in a cell type that is normally used for the generation of genetic, mosaic animals suggests the possibility of eventually testing the biological effects of silencing both in culture and in reconstituted animal models. Our ability to successfully manipulate ES cell via RNAi allows the use of RNAi in the generation of transgenic and knock-out mice.

### C. RNAi in Murine Somatic Cells.

RNAi effector pathways are likely to be present in mammalian somatic cells, based on the ability of siRNAs to induce transient silencing (Elbashir et al. 2001, Nature 411; 494-498). Furthermore, we have shown that RNAi initiator and effector pathways clearly exist in embryonic cells that can enforce silencing in response to long dsRNA triggers. We therefore sought to test whether the RNAi machinery might exist intact in some somatic cell lines.

Transfection of HeLa cells with luciferase reporters in combination with long dsRNA triggers caused a nearly complete suppression of activity, irrespective of the RNA sequence. In a murine myoblast cell line, C2C12, we noted a mixture of two responses. dsRNAs homologous to firefly luciferase provoked a sequence-specific effect, producing a degree of suppression that was slightly more potent than was observed upon transfection with cognate ≈21-nt siRNA (Elbashir et al. 2001, Nature 411: 494-498) (see Fig. 32A). However, with long dsRNA triggers, the specific effect was superimposed upon a generalized suppression of reporter gene expression that was presumably because of PKR activation (Fig. 32B).

Numerous mammalian viruses have evolved the ability to block PKR as an aid to efficient infection. For example, adenoviruses express VA RNAs, which mimic dsRNA with respect to binding but not to activation of PKR (Clarke et al. 1995, RNA 1: 7-20). Vaccinia virus uses two strategies to evade PKR. The first is expression of E3L, which binds and masks dsRNAs (Kawagishi-Kobayashi et al. 2000, Virology 276: 424-434). The second is expression of K3L, which binds and inhibits PKR via its ability to mimic the natural substrate of this enzyme, eIF2α (Kawagishi-Kobayashi et al. 2000, *supra*).

Transfection of C2C12 cells with a vector that directs K3L expression attenuates the generalized repression of reporter genes in response to dsRNA. However, this protein had no effect on the magnitude of specific inhibition by RNAi (Fig. 32C).

Figure 33 further shows the results of a transient co-transfection assay performed in Hela cells, CHO cells, and P19 cells. The cell lines were each transfected with plasmids expressing *Photinus pyralis* (firefly) and *Renila reniformis* (sea pansy) luciferases. The cells lines were additionally transfected with 400 ng of 500nt dsRNAs corresponding to either firefly luciferase (dsLUC) or dsGFP. The results demonstrate that dsRNA can specifically mediate suppression in a multiple mammalian cells types in culture.

These results raise the possibility that, at least in some cell lines and/or cell types, blocking nonspecific responses to dsRNA will enable the use of long dsRNAs for the study of gene function. This might be accomplished through the use of viral inhibitors, as described here, or through the use of cells isolated from animals that are genetically modified to lack undesirable responses.

### D. Stable Suppression of Gene Expression Using RNAi.

To date, dsRNAs have been used to induce sequence-specific gene silencing in either cultured mammalian cells or in embryos only in a transient fashion. However, the most powerful applications of genetic manipulation are realized only with the creation of stable mutants. The ability to induce silencing by using long dsRNAs offers the opportunity to translate into mammalian cells work from model systems such as *Drosophila,* plants, and *C*. *elegans* wherein stable silencing has been achieved by enforced expression of hairpin RNAs (Kennerdell et al. 2000, Nat. Biotechnol. 18: 896-898; Smith et al. 2000, Nature 407: 319-320; Tavemarakis et al. 2000, Nat. Genet. 24: 180-183).

P 19 EC cells were transfected with a control vector or with an expression vector that directs expression of a ≈500-nt GFP hairpin RNA from an RNA polymerase II promoter (cytomegalovirus). Colonies arising from cells that had stably integrated either construct were selected and expanded into clonal cell lines. Each cell line was assayed for persistent RNAi by transient cotransfection with a mixture of two reporter genes, dsRED to mark transfected cells and GFP to test for stable silencing.

Transfection of clonal P19 EC cells that had stably integrated the control vector produced equal numbers of red and green cells, as would be expected in the absence of any specific silencing response (Fig. 34B), whereas cells that express the GFP hairpin RNA gave a very different result. These cells expressed the dsRED protein with an efficiency comparable to that observed in cells containing the control vector. However, the cells failed to express the cotransfected GFP reporter (Fig. 34B). These data provide a strong indication that continuous expression of a hairpin dsRNA can provoke stable, sequence-specific silencing of a target gene.

In *Drosophila* S2 cells and *C. elegans,* RNAi is initiated by the Dicer enzyme, which processes dsRNA into ≈22-nt siRNAs (Bernstein et al. 2001, Nature 409: 363-366; Grishok et al. 2001, Cell 106: 23-34; Hutvagner et al. 2001, Science 293: 834-838; Ketting et al. 2001, Genes Dev. 15: 2654-2659; Knight et al. 2001, Science 293: 2269-2271). In both, S2 cells and *C. elegans* experiments by using dsRNA to target Dicer suppress the RNAi response. Whether Dicer plays a central role in hairpin-induced gene silencing in P19 cells was tested by transfecting P19 cells stably transfected with GFP hairpin constructs with mouse *Dicer* dsRNA. Treatment with *Dicer* dsRNA, but not control dsRNA, resulted in derepression of GFP (Fig. 34C).

### E. dsRNA Induces Posttranscriptional Silencing.

A key feature of RNAi is that it exerts its effect at the posttranscriptional level by destruction of targeted mRNAs (Hammond et al. 2001, Nat. Rev. Genet. 2: 110-119). To test whether dsRNAs induced silencing in mouse cells via posttranscriptional mechanisms, we used an assay identical to that, used initially to characterize RNAi responses in *Drosophila* embryo extracts (Tuschl et al. 1999, Genes Dev. 13: 3191-3197). We prepared lysates from P19 EC cells that were competent for *in vitro* translation of capped mRNAs corresponding to *Renilia* and firefly luciferase. Addition of nonspecific dsRNAs to these extracts had no substantial effect on either the absolute amount of luciferase expression or on the ratio of firefly to *Renilla* luciferase (Fig. 35). In contrast, addition of dsRNA homologous to the firefly luciferase induced a dramatic and dose-dependent suppression of activity. Addition of RNA corresponding to only the antisense strand of the dsRNA had little effect, comparable to a nonspecific dsRNA control, and pretreatment of the dsRNA silencing trigger with RNase III greatly reduced its potential to induce silencing *in vitro.* A second hallmark of RNAi is the production of small, ≈22-nt siRNAs, which determine the specificity of silencing. We found that such RNA species were generated from dsRNA in P19 cell extracts (Fig. 34D, *in vitro*), indicative of the presence of a mouse Dicer activity. These species were also produced in cells that stably express GFP hairpin RNAs (Fig. 34D, *in vivo*). Considered together, the posttranscriptional nature of dsRNA-induced silencing, the association of silencing with the production of ≈22-nt siRNAs, and the dependence of this response on Dicer, a key player in the RNAi pathway, strongly suggests that dsRNA suppresses gene expression in murine cells via a conventional RNAi mechanism.

### F. RNAi-Mediated Gene Silencing Is Specific and Requires dsRNAs.

We carried out experiments to verify that the suppressive effects observed in the in vitro system were specific to double stranded RNA. Briefly, experiments were performed in accordance with the methods outlined above. Either dsRNA (ds), single-stranded RNA (ss), or antisense-RNA (as) corresponding to firefly (FF) or *Renilla* (Ren) luciferase was added to the translation reaction. Following reactions performed at 30°C for 1 hour, dual luciferase assays were performed using an Analytical Scientific Instruments model 3010 Luminometer.

Figure 36 summarizes the results of these experiments which demonstrate that the suppression of gene expression observed in this in vitro assay is specific for dsRNA. These results further support the conclusion that dsRNA suppresses gene expression in this mammalian in vitro system in a manner consistent with post-transcriptional silencing.

### G. Mammalian Cells Soaked with dsRNAs Results in Gene Silencing.

Studies of post-transcriptional silencing in invertebrates have demonstrated that transfection or injection of the dsRNA is not necessary to achieve the suppressive affects. For example, dsRNA suppression in C. elegans can be observed by either soaking the worms in dsRNA, or by feeding the worms bacteria expressing the dsRNA of interest. We addressed whether dsRNA suppression in mammalian cells could be observed without transfection of the dsRNA. Such a result would present additional potential for easily using dsRNA suppression in mammalian cells, and would also allow the use of dsRNA to suppress gene expression in cell types which have been difficult to transfect (i.e., cell types with a low transfection efficiency, or cell types which have proven difficult to transfect at all).

P 19 cells were grown in 6-well tissue culture plates to approximately 60% confluency in growth media (αMEM/10% FBS). Varying concentrations of firefly dsRNA were added to the cultures, and cells were cultured for 12 hours in growth media + dsRNA. Cells were then transfected with plasmids expressing firefly or sea pansy luciferase, as described in detail above. Dual luciferase assays were carried out 12 hours post-transfection using an Analytical Scientific Instruments model 3010 Luminometer.

Figure 37 summarizes these results which demonstrate that dsRNA can suppress gene expression in mammalian cells without transfection. Culturing cells in the presence of dsRNA resulted in a dose dependent suppression of firefly luciferase gene expression.

### Methods:

**Cell Culture.** P19 mouse embryonic carcinoma cells (American Type Culture Collection, CRL-1825) were cultured in α-MEM (GIBCO/BRL) supplemented with 10% heat-inactivated FBS and 1% antibiotic/antimycotic solution (GIBCO/BRL). Mouse embryo stem cells (J1, provided by S. Kim, Cold Spring Harbor Laboratory) were cultured in DMEM containing ESgro (Chemicon) according to the manufacturer's instructions. C2C12 murine myoblast cells (gift of N. Tonks, Cold Spring Harbor Laboratory) were cultured in DMEM (GIBCO/BRL) supplemented with 10% heat-inactivated FBS and 1% antibiotic/antimycotic solution (GIBCO/BRL).

**RNA Preparation**. For the production of dsRNA, transcription templates were generated by PCR; they contained T7 promoter sequences on each end of the template (see Hammond et al. 2000, Nature 404: 293-296). dsRNAs were prepared by using the RiboMax kit (Ambion, Austin, TX). Firefly and *Renilla* luciferase mRNA transcripts were synthesized by using the Riboprobe kit (Promega) and were gel purified before use.

**Transfection and Gene Silencing Assays**. Cells were transfected with indicated amounts of dsRNA and plasmid DNA by using FuGENE6 (Roche Biochemicals) according to the manufacturer's instructions. Cells were transfected at 50-70% confluence in 12-well plates containing either 1 or 2 ml of medium per well. Dual luciferase assays (Promega) were carried out by cotransfecting cells with plasmids contain firefly luciferase under the control of SV40 promoter (pGL3-Control, Promega) and *Renilla* luciferase under the control of the SV40 early enhancer/promoter region (pSV40, Promega). These plasmids were cotransfected by using a 1:1 or 10:1 ratio of pGL3-control (250 ng/well) to pRL-SV40. Both ratios yielded similar results. For some experiments, cells were transfected with vectors that direct expression of enhanced green fluorescent protein (EGFP)-US9 fusion protein (Kalejta et al. 1999, Exp. Cell Res. 248: 322-328) or red fluorescent protein (RFP) (pDsRed N1, CLONTECH). RNAi in S2 cells was performed as described (Hammond et al. 2000, Nature 404: 293-296).

Plasmids expressing hairpin RNAs (RNAs with a self complimentary stem loop) were constructed by cloning the first 500 bp of the EGFP coding region (CLONTECH) into the FLIP cassette of pRIP-FLIP as a direct repeat. The FLIP cassette contains two directional cloning sites, the second of which sports flanking LoxP sites (see Fig. 35A). The Zeocin gene (Stratagene), present between the cloning sites, maintains selection and, thus, stability of the FLIP cassette. The FLIP cassette containing EGFP direct repeats was subcloned into pcDNA3 (Invitrogen). To create an inverted repeat for hairpin production, EGFP direct repeat clones were exposed to Cre recombinase (Stratagene) *in vitro* and, afterward, transformed into DL759 *Escherichia coli* (Connelly et al. 1996, Genes Cells 1: 285-291). These bacteria permit the replication of DNA containing cruciform structures, which tend to form from inverted repeats. DL759 transformants were screened for plasmids containing inverted repeats (≈50%).

Silencing of Dicer was accomplished by using a dsRNA comprising exon 25 of the mouse Dicer gene and corresponding to nucleotides 5284-5552 of the human Dicer cDNA.

***In Vitro* Translation and *in Vitro* Dicer Assays.** Logarithmically growing cells were harvested in PBS containing 5 mM EGTA washed twice in PBS and once in hypotonic buffer (10 mM Hepes, pH 7.3/6 mM β-mercaptoethanol). Cells were suspended in 0.7 packed-cell volumes of hypotonic buffer containing *Complete* protease inhibitors (Roche Molecular Biochemicals) and 0.5 units/ml of RNasin (Promega). Cells were disrupted in a Dounce homogenizer with a type B pestle, and lysates were centrifuged at 30,000 x g for 20 min. Supernatants were used in an *in vitro* translation assay containing capped m7G(5')pppG firefly and *Renilla* luciferase mRNA or in *in vitro* Dicer assays containing ³²P-labeled dsRNA. For *in vitro* translation assays, 5 µl of extract were mixed with 100 ng of firefly and *Renilla* mRNA along with 1 µg of dsRNA (or buffer)/10 mM DTT/0.5 mM spermidine/200 mM Hepes, 3.3 mM MgOAc/800 mM KOAc/1 mM ATP/1 mM GTP/4 units of Rnasin/215 µg of creatine phosphate/1 µg of creatine phosphate kinase/1 mM amino acids (Promega). Reactions were carried out for 1 h at 30°C and quenched by adding 1 x passive lysis buffer (Promega). Extracts were then assayed for luciferase activity. *In vitro* assays for Dicer activity were performed as described (Bernstein et al. 2001, Nature 409: 363-366).

**Construction of Stable Silencing Lines**. Ten-centimeter plates of P 19 cells were transfected with 5 µg of GFP hairpin expression plasmid and selected for stable integrants by using G-418 (300 ng/ml) for 14 days. Clones were selected and screened for silencing of GFP.

### Example 5: Compositions and Methods for Synthesizing siRNAs

Previous results have indicated that short synthetic RNAs (siRNAs) can efficiently induce RNA suppression. Since short RNAs do not activate the non-specific PKR response, they offer a means for efficiently silencing gene expression in a range of cell types. However, the current state of the art with respect to siRNAs has several limitations. Firstly, siRNAs are currently chemically synthesized at great cost (approx. $400/siRNA). Such high costs make siRNAs impractical for either small laboratories or for use in large scale screening efforts. Accordingly, there is a need in the art for methods for generating siRNAs at reduced cost.

We provide compositions and methods for synthesizing siRNAs by T7 polymerase. This approach allows for the efficient sythesis of siRNAs at a cost consistent with standard RNA transcription reactions (approx. $16/siRNA). This greatly reduced cost makes the use of siRNA a reasonable approach for small laboratories, and also will facilitate their use in large-scale screening projects.

Figure 38 shows the method for producing siRNAs using T7 polymerase. Briefly, T7 polymerase is used to transcribe both a sense and antisense transcript. The transcripts are then annealed to provide an siRNA. One of skill in the art will recognize that any one of the available RNA polymerases can be readily substituted for T7 to practice the invention (i.e., T3, Sp6, etc.).

This approach is amenable to the generation of a single siRNA species, as well as to the generation of a library of siRNAs. Such a library of siRNAs can be used in any number of high-throughput screens including cell based phenotypic screens and gene array based screens.

### Example 6: Generation of Short Hairpin dsRNA and Suppression of Gene Expression Using Such Short Hairpins

Since the realization that small, endogenously encoded hairpin RNAs could regulate gene expression via elements of the RNAi machinery, we have sought to exploit this biological mechanism for the regulation of desired target genes. Here we show that short hairpin RNAs (shRNAs) can induce sequence-specific gene silencing in mammalian cells. As is normally done with siRNAs, silencing can be provoked by transfecting exogenously synthesized hairpins into cells. However, silencing can also be triggered by endogenous expression of shRNAs. This observation opens the door to the production of continuous cells lines in which RNAi is used to stably suppress gene expression in mammalian cells. Furthermore, similar approaches should prove efficacious in the creation of transgenic animals and potentially in therapeutic strategies in which long-term suppression of gene function is essential to produce a desired effect.

Several groups (Grishok et al. 2001, Cell 106: 23-34; Ketting et al. 2001, Genes & Dev. 15: 2654-2659; Knight et al. 2001, Science 293: 2269-2271; Hutvagner et al. 2001, Science 293: 834-838) have shown that endogenous triggers of gene silencing, specifically small temporal RNAs (stRNAs) *let-7* and *lin-4,* function at least in part through RNAi pathways. Specifically, these small RNAs are encoded by hairpin precursors that are processed by Dicer into mature, ∼21-nt forms. Moreover, genetic studies in *C*. *elegans* have shown a requirement for Argonaute-family proteins in stRNA function. Specifically, alg-1 and alg-2, members of the EIF2c subfamily, are implicated both in stRNA processing and in their downstream effector functions (Grishok et al., 2001, *supra*). We have recently shown that a component of RISC, the effector nuclease of RNAi, is a member of the Argonaute family, prompting a model in which stRNAs may function through RISC-like complexes, which regulate mRNA translation rather than mRNA stability (Hammond et al. 2001, Science 293: 1146-1150).

### A. Short Hairpin RNAs Triggeedr Gene Silencing in Drosophila Cells.

We wished to test the possibility that we might retarget these small, endogenously encoded hairpin RNAs to regulate genes of choice with the ultimate goal of subverting this regulatory system for manipulating gene expression stably in mammalian cell lines and in transgenic animals. Whether triggered by long dsRNAs or by siRNAs, RNAi is generally more potent in the suppression of gene expression in *Drosophila* S2 cells than in mammalian cells. We therefore chose this model system in which to test the efficacy of short hairpin RNAs (shRNAs) as inducers of gene silencing.

Neither stRNAs nor the broader group of miRNAs that has recently been discovered form perfect hairpin structures. Indeed, each of these RNAs is predicted to contain several bulged nucleotides within their rather short (∼30-nt) stem structures. Because the position and character of these bulged nucleotides have been conserved throughout evolution and among at least a subset of miRNAs, we sought to design retargeted miRNA mimics to conserve these predicted structural features. Only the *let-7* and *lin-4* miRNAs have known mRNA targets (Wightman et al. 1993, Cell 75: 855-862; Slack et al. 2000, Mol. Cell 5: 659-669). In both cases, pairing to binding sites within the regulated transcripts is imperfect, and in the case of *lin-4,* the presence of a bulged nucleotide is critical to suppression (Ha et al. 1996, Genes & Dev. 10: 3041-3050). We therefore also designed shRNAs that paired imperfectly with their target substrates. A subset of these shRNAs is depicted in Figure 39A.

To permit rapid testing of large numbers of shRNA variants and quantitative comparison of the efficacy of suppression, we chose to use a dual-luciferase reporter system, as previously described for assays of RNAi in both *Drosophila* extracts (Tuschl et al. 1999, Genes & Dev. 13: 3191-3197) and mammalian cells (Caplen et al. 2001, Proc. Natl. Acad. Sci. 98: 9742-9747; Elbashir et al., 2001, Nature 411: 494-498). Cotransfection of firefly and *Renilla* luciferase reporter plasmids with either long dsRNAs or with siRNAs homologous to the firefly luciferase gene yielded an ∼95% suppression of firefly luciferase without effect on *Renilla* luciferase (Fig. 39B; data not shown). Firefly luciferase could also be specifically silenced by cotransfection with homologous shRNAs. The most potent inhibitors were those composed of simple hairpin structures with complete homology to the substrate. Introduction of G-U basepairs either within the stem or within the substrate recognition sequence had little or no effect (Fig. 39A and 39B; data not shown).

These results show that short hairpin RNAs can induce gene silencing in *Drosophila* S2 cells with potency similar to that of siRNAs (Fig. 39B). However, in our initial observation of RNA interference in *Drosophila* S2 cells, we noted a profound dependence of the efficiency of silencing on the length of the dsRNA trigger (Hammond et al. 2000, Nature 404: 293-296). Indeed, dsRNAs of fewer than ∼200 nt triggered silencing very inefficiently. Silencing is initiated by an RNase III family nuclease, Dicer, that processes long dsRNAs into ∼22-nt siRNAs. In accord with their varying potency as initiators of silencing, long dsRNAs are processed much more readily than short RNAs by the Dicer enzyme (Bernstein et al. 2001, Nature 409: 363-366). We therefore tested whether shRNAs were substrates for the Dicer enzyme.

We had noted previously that *let-7* (Ketting et al. 2001, Genes & Dev. 15: 2654-2659) and other miRNAs (E. Bernstein, unpublished data) are processed by Dicer with an unexpectedly high efficiency as compared with short, nonhairpin dsRNAs. Similarly, Dicer efficiently processed shRNAs that targeted firefly luciferase, irrespective of whether they were designed to mimic a natural Dicer substrate (*let-7*) or whether they were simple hairpin structures (Fig. 39C). These data suggest that recombinant shRNAs can be processed by Dicer into siRNAs and are consistent with the idea that these short hairpins trigger gene silencing via an RNAi pathway.

### B. Short Hairpin RNAs Activated Gene Silencing in Mammalian Cells.

Mammalian cells contain several endogenous systems that were predicted to hamper the application of RNAi. Chief among these is a dsRNA-activated protein kinase, PKR, which effects a general suppression of translation via phosphorylation of EIF-2a (Williams 1997, Biochem. Soc. Trans. 25: 509-513; Gil et al. 2000, Apoptosis 5: 107-114). Activation of these, and other dsRNA-responsive pathways, generally requires duplexes exceeding 30 bp in length, possibly to permit dimerization of the enzyme on its allosteric activator (e.g., Clarke et al. 1995, RNA 1: 7-20). Small RNAs that mimic Dicer products, siRNAs, presumably escape this limit and trigger specific silencing, in part because of their size. However, short duplex RNAs that lack signature features of siRNAs can efficiently induce silencing in *Drosophila* S2 cells but not in mammalian cells (A.A. Caudy, unpublished data). Endogenously encoded miRNAs may also escape PKR surveillance because of their size but perhaps also because of the discontinuity of their duplex structure. Given that shRNAs of <30 bp were effective inducers of RNAi in *Drosophila* S2 cells, we tested whether these RNAs could also induce sequence-specific silencing in mammalian cells.

Human embryonic kidney (HEK293T) cells were cotransfected with chemically synthesized shRNAs and with a mixture of firefly and *Renilla* luciferase reporter plasmids. As had been observed in S2 cells, shRNAs were effective inducers of gene silencing. Once again, hairpins designed to mimic *let-7* were consistently less effective than were simple hairpin RNAs, and the introduction of mismatches between the antisense strand of the shRNA and the mRNA target abolished silencing (Fig. 40A; data not shown). Overall, shRNAs were somewhat less potent silencing triggers than were siRNAs. Whereas siRNAs homologous to firefly luciferase routinely yielded ∼90%-95% suppression of gene expression, suppression levels achieved with shRNAs ranged from 80%-90% on average. As we also observe with siRNAs, the most important determinant of the potency of the silencing trigger is its sequence. We find that roughly 50% of both siRNAs and shRNAs are competent for suppressing gene expression. However, neither analysis of the predicted structures of the target mRNA nor analysis of alternative structures in siRNA duplexes or shRNA hairpins has proved of predictive value for choosing effective inhibitors of gene expression.

We have adopted as a standard, shRNA duplexes containing 29 bp. However, the size of the helix can be reduced to ∼25 nt without significant loss of potency. Duplexes as short as 22 bp can still provoke detectable silencing, but do so less efficiently than do longer duplexes. In no case did we observe a reduction in the internal control reporter (*Renilla* luciferase) that would be consistent with an induction of nonspecific dsRNA responses.

The ability of shRNAs to induce gene silencing was not confined to 293T cells. Similar results were also obtained in a variety of other mammalian cell lines, including human cancer cells (HeLa), transformed monkey epithelial cells (COS-1), murine fibroblasts (NIH 3T3), and diploid human fibroblasts (IMR90; Fig. 40; data not shown).

### C. Synthesis of Effective Inhibitors of Gene Expression Using T7 RNA Polymerse.

The use of siRNAs to provoke gene silencing is developing into a standard methodology for investigating gene function in mammalian cells. To date, siRNAs have been produced exclusively by chemical synthesis (e.g., Caplen et al. 2001, Proc. Natl. Acad. Sci. 98: 9742-9747; Elbashir et al. 2001, Nature 411: 494-498). However, the costs associated with this approach are significant, limiting its potential utility as a tool for investigating in parallel the functions of large numbers of genes. Short hairpin RNAs are presumably processed into active siRNAs in vivo by Dicer. Thus, these may be more tolerant of terminal structures, both with respect to nucleotide overhangs and with respect to phosphate termini. We therefore tested whether shRNAs could be prepared by in vitro transcription with T7 RNA polymerase.

Transcription templates that were predicted to generate siRNAs and shRNAs similar to those prepared by chemical RNA synthesis were prepared by DNA synthesis (Fig. 41A,C). These were tested for efficacy both in S2 cells (data not shown) and in human 293 cells (Fig. 41B,D). Overall, the performance of the T7-synthesized hairpin or siRNAs closely matched the performance of either produced by chemical synthesis, both with respect to the magnitude of inhibition and with respect to the relative efficiency of differing sequences. Because T7 polymerase prefers to initiate at twin guanosine residues, however, it was critical to consider initiation context when designing in vitro transcribed siRNAs (Fig. 41B). In contrast, shRNAs, which are processed by Dicer (see Fig. 39C), tolerate the addition of these bases at the 5' end of the transcript.

Studies in *Drosophila* embryo extracts indicate that siRNAs possess 5' phosphorylated termini, consistent with their production by an RNase III family nuclease. In vitro, this terminus is critical to the induction of RNAi by synthetic RNA oligonucleotides (Elbashir et al. 2001, EMBO J. 20: 6877-6888; Nykanen et al. 2001, Cell 107: 309-321). Chemically synthesized siRNAs are nonphosphorylated, and enzymatic addition of a 5' phosphate group in vitro prior to transfection does not increase the potency of the silencing effect (A.A. Caudy, unpublished data). This suggests either that the requirement for phosphorylated termini is less stringent in mammalian cells or that a kinase efficiently phosphorylates siRNAs in vivo. RNAs synthesized with T7 RNA polymerase, however, possess 5' triphosphate termini. We therefore explored the possibility of synthesizing siRNAs with T7 polymerase followed by treatment in vitro with pyrophosphatase to modify the termini to resemble those of siRNAs. Surprisingly, monophosphorylated siRNAs (data not shown) were as potent in inducing gene silencing as transcription products bearing triphosphate termini (Fig. 41 B). This may suggest either that the requirement for monophosphorylated termini is less stringent in mammalian cells or that siRNAs are modified in vivo to achieve an appropriate terminal structure.

Considered together, our data suggest that both shRNAs and siRNA duplexes can be prepared by synthesis with T7 RNA polymerase in vitro. This significantly reduces the cost of RNAi in mammalian cells and paves the way for application of RNAi on a whole-genome scale.

### D. Transcription of Small Hairpin RNAs In Vivo by RNA Polymerase III.

Although siRNAs are an undeniably effective tool for probing gene function in mammalian cells, their suppressive effects are by definition of limited duration. Delivery of siRNAs can be accomplished by any of a number of transient transfection methodologies, and both the timing of peak suppression and the recovery of protein levels as silencing decays can vary with both the cell type and the target gene. Therefore, one limitation on siRNAs is the development of continuous cell lines in which the expression of a desired target is stably silenced.

Hairpin RNAs, consisting of long duplex structures, have been proved as effective triggers of stable gene silencing in plants, in *C. elegans,* and in *Drosophila* (Kennerdell et al. 2000, Nat. Biotechnol. 18: 896-898; Smith et al. 2000, Nature 407: 319-320; Tavernarakis et al. 2000, Nat. Genet. 24: 180-183). We have recently shown stable suppression of gene expression in cultured mammalian cells by continuous expression of a long hairpin RNA (Paddison et al. 2002, Proc. Natl. Acad. Sci. 99: 1443-1448). However, the scope of this approach was limited by the necessity of expressing such hairpins only in cells that lack a detectable PKR response. In principle, shRNAs could bypass such limitations and provide a tool for evoking stable suppression by RNA in mammalian somatic cells.

To test this possibility, we initially cloned sequences encoding a firefly luciferase shRNA into a CMV-based expression plasmid. This was predicted to generate a capped, polyadenylated RNA polymerase II transcript in which the hairpin was extended on both the 5' and 3' ends by vector sequences and poly(A). This construct was completely inert in silencing assays in 293T cells.

During our studies on chemically and T7-synthesized shRNAs, we noted that the presence of significant single-stranded extensions (either 5' or 3' of the duplex) reduced the efficacy of shRNAs. We therefore explored the use of alternative promoter strategies in an effort to produce more defined hairpin RNAs. In particular, RNA polymerase III promoters have well-defined initiation and termination sites and naturally produce a variety of small, stable RNA species. Although many Pol III promoters contain essential elements within the transcribed region, limiting their utility for our purposes; class III promoters use exclusively nontranscribed promoter sequences. Of these, the U6 snRNA promoter and the H1 RNA promoter have been well studied (Lobo et al. 1990, Nucleic Acids Res. 18: 2891-2899; Hannon et al. 1991, J. Biol. Chem. 266: 22796-22799; Chong et al. 2001, J. Biol. Chem. 276: 20727-20734).

By placing a convenient cloning site immediately behind the U6 snRNA promoter, we have constructed pShh-1, an expression vector in which short hairpins are harnessed for gene silencing. Into this vector either of two shRNA sequences derived from firefly luciferase were cloned from synthetic oligonucleotides. These were cotransfected with firefly and *Renilla* luciferase expression plasmids into 293T cells. One of the two encoded shRNAs provoked effective silencing of firefly luciferase without altering the expression of the internal control (Fig. 42C). The second encoded shRNA also produced detectable, albeit weak, repression. In both cases, silencing was dependent on insertion of the shRNA in the correct orientation with respect to the promoter (Fig. 42C; data not shown). Although the shRNA itself is bilaterally symmetric, insertion in the incorrect orientation would affect Pol III termination and is predicted to produce a hairpin with both 5' and 3' single-stranded extensions. Similar results were also obtained in a number of other mammalian cell lines including HeLa, COS-1, NIH 3T3, and IMR90 (Fig. 42; data not shown). pShh1-Ff1 was, however, incapable of effecting suppression of the luciferase reporter in *Drosophila* cells, in which the human U6 promoter is inactive.

### E. Dicer Is Required for shRNA-Mediated Gene Silencing.

As a definitive test of whether the plasmid-encoded shRNAs brought about gene silencing via the mammalian RNAi pathway, we assessed the dependence of suppression on an essential component of the RNAi pathway. We transfected pShh1-Ff1 along with an siRNA homologous to human *Dicer.* Figure 43 shows that treatment of cells with *Dicer* siRNAs is able to completely depress the silencing induced by pShh1-Ff1. Addition of an unrelated siRNA had no effect on the magnitude of suppression by pShh1-Ff1. Importantly, *Dicer* siRNAs had no effect on siRNA-induced silencing of firefly luciferase. These results are consistent with shRNAs operating via an RNAi pathway similar to those provoked by stRNAs and long dsRNAs. Furthermore, it suggests that siRNA-mediated silencing is less sensitive to depletion of the Dicer enzyme.

### F. Stable shRNA-Mediated Gene Silencing of An Endogenous Gene.

The ultimate utility of encoded short hairpins will be in the creation of stable mutants that permit the study of the resulting phenotypes. We therefore tested whether we could create a cellular phenotype through stable suppression. Expression of activated alleles of the *ras* oncogene in primary mouse embryo fibroblasts (MEFs) induces a stable growth arrest that resembles, as a terminal phenotype, replicative senescence (Serrano et al. 1997, Cell 88: 593-602). Cells cease dividing and assume a typical large, flattened morphology. Senescence can be countered by mutations that inactivate the p53 tumor suppressor pathway (Serrano et al. 1997, *supra*). As a test of the ability of vector-encoded shRNAs to stably suppress an endogenous cellular gene, we generated a hairpin that was targeted to the mouse *p53* gene. As shown in Figure 44, MEFs transfected with pBabe-RasV12 fail to proliferate and show a senescent morphology when cotransfected with an empty control vector. As noted previously by Serrano et al., the terminally arrested state is achieved in 100% of drug-selected cells in culture by 8 d posttransfection. However, upon cotransfection of an activated *ras* expression construct with the pShh-p53, cells emerged from drug selection that not only fail to adopt a senescent morphology but also maintain the ability to proliferate for a minimum of several weeks in culture (Fig. 44). These data strongly suggest that shRNA expression constructs can be used for the creation of continuous mammalian cell lines in which selected target genes are stably suppressed.

### G. Simultaneous Introduction of Multiple Hairpin RNAs Does Not Produce Synergy.

In an attempt to further understand the mechanisms by which short hairpins suppress gene expression, we examined the effects of transfecting cells with a mixture of two different short hairpins corresponding to firefly luciferase. Figure 45 summarizes the results of experiments which suggest that there is no synergistic affects on suppression of firefly luciferase gene expression obtained when cells are exposed to a mixture of such short hairpins.

### Methods:

**Cell culture**. HEK 293T, HeLa, COS-1, MEF, and IMR90 cells were cultured in DMEM (GIBCO BRL) supplemented with 10% heat-inactivated fetal bovine serum (FBS) and 1% antibiotic/antimycotic solution (GIBCO BRL). NIH 3T3 cells were cultured in DMEM supplemented with 10% heat-inactivated calf serum and 1% antibiotic/antimycotic solution.

**RNA preparation.** Both shRNAs and siRNAs were produced in vitro using chemically synthesized DNA oligonucleotide templates (Sigma) and the T7 Megashortscript kit (Ambion). Transcription templates were designed such that they contained T7 promoter sequences at the 5' end. shRNA transcripts subjected to in vitro Dicer processing were synthesized using a Riboprobe kit (Promega). Chemically synthesized RNAs were obtained from Dharmacon, Inc.

**Transfection and gene silencing assays.** Cells were transfected with indicated amounts of siRNA, shRNA, and plasmid DNA using standard calcium phosphate procedures at 50%-70% confluence in 6-well plates. Dual luciferase assays (Promega) were carried out by cotransfecting cells with plasmids containing firefly luciferase under the control of the SV40 promoter (pGL3-Control, Promega) and *Renilla* luciferase under the control of the SV40 early enhancer/promoter region (pSV40, Promega). Plasmids were cotransfected using a 1:1 ratio of pGL3-Control (250 ng/well) to pRL-SV40. RNAi in S2 cells was performed as previously described (Hammond et al., 2000, Nature 404: 293-296). For stable silencing, primary MEFs (a gift from S. Lowe, Cold Spring Harbor Laboratory, NY) were cotransfected using Fugene 6 with pBabe-Ha-rasV12 and pShh-p53 (no resistance marker), according to the manufacturer's recommendations. Selection was for the presence of the activated *Ha-rasV12* plasmid, which carries a puromycin-resistance marker. The pShh-p53 plasmid was present in excess, as is standard in a cotransfection experiment. We have now generated a version of the U6 promoter vector (pSHAG-1) that is compatible with the GATEWAY system (Invitrogen), and this can be used to transport the shRNA expression cassette into a variety of recipient vectors that carry cis-linked selectable markers. Furthermore, we have validated delivery of shRNAs using retroviral vectors. Updated plasmid information can be obtained at http://www.cshl.org/public/science/hannon.html.

**Plasmids expressing hairpin RNAs**. The U6 promoter region from -265 to +1 was amplified by PCR, adding 5' *Kpn*I and 3' *Eco*RV sites for cloning into pBSSK+. A linker/terminator oligonucleotide set bearing the U6 terminator sequence and linker ends of 5' *Eco*RV and 3' *Not*I was cloned into the promoter construct, resulting in a U6 cassette with an *Eco*RV site for insertion of new sequences. This vector has been named pShh1. Blunt-ended, double-stranded DNA oligonucleotides encoding shRNAs with between 19 and 29 bases of homology to the targeted gene were ligated into the *Eco*RV site to produce expression constructs. The oligonucleotide sequence used to construct Ff1 was: TCCAATTCAGCGGGAGCCACCTGATGAAGCTTGATCGGGTGGCTCTCGCT GAGTTGGAATCCATTTTTTTT. This sequence is preceded by the sequence GGAT, which is supplied by the vector, and contains a tract of more than five Ts as a Pol III terminator.

**In vitro Dicer assays.** In vitro assays for Dicer activity were performed as described (Bernstein et al., 2001, Nature 409: 363-366).

### Example 7: Encoded Short Hairpins Function In Vivo

An object of the present invention is to improve methods for generating siRNAs and short hairpins for use in specifically suppressing gene expression. Example 6 demonstrates that siRNAs and short hairpins are highly effective in specifically suppressing gene expression. Accordingly, it would be advantageous to combine the efficient suppression of gene expression attainable using short hairpins and siRNAs with a method to encode such RNA on a plasmid and express it either transiently or stably.

Figure 46 demonstrates that short hairpins encoded on a plasmid are effective in suppressing gene expression. DNA oligonucleotides encoding 29 nucleotide hairpins corresponding to firefly luciferase were inserted into a vector containing the U6 promoter. Three independent constructs were examined for their ability to specifically suppress firefly luciferase gene expression in 293T cells. siOligo1-2, siOligo1-6, and siOligo1-19 (construct in the correct orientation) each suppressed gene expression as effectively as siRNA. In contrast, siOligo1-10 (construct in the incorrect orientation) did not suppress gene expression. Additionally, an independent construct targeted to a different portion of the firefly luciferase gene did not effectively suppress gene expression in either orientation (siOligo2-23, siOligo2-36).

The results summarized in Figure 46 demonstrate that transient expression of siRNAs and short hairpins encoded on a plasmid can efficiently suppress gene expression. One of skill can choose from amongst a range of vectors to either transiently or stably express an siRNA or short hairpin. Non-limiting examples of vectors and strategies to stably express short dsRNAs are presented in Figures 47-49.

### Example 8: dsRNA Suppression in the Absence of a PKR Response

One potential impediment to the use of RNAi to suppress gene expression in some cell types, is the non-specific PKR response that can be triggered by long dsRNAs. Numerous mammalian viruses have evolvd the ability to block PKR inorder to aid in the infection of potential host cells. For example, adenoviruses express RNAs which mimic dsRNA but do not activate the PKR response. Vaccinia virus uses two strategies to evade PKR: the expression of E3L which binds and masks dsRNA; the expression of K3L to mimic the natural PKR substrate eIF2α.

Our understanding of the mechanisms by which viruses avoid the PKR response allows us to design approaches to circumvent the PKR response in cell types in which in might be advantageous to suppression gene expression with long dsRNAs. Possible approaches include treating cells with an agent that inhibits protein kinase RNA-activated (PKR) apoptosis, such as by treatment with agents which inhibit expression of PKR, cause its destruction, and/or inhibit the kinase activity of PKR. Accordingly, RNAi suppression of gene expression in such cell types could involve first inhibiting the PKR response, and then delivering a dsRNA identical or similar to a target gene.

A. In a murine myoblast cell line, C2C12, we noted that the cells responded to long dsRNAs with a mixture of specific and non-specific (presumably PKR) responses. In order to attenuate the non-specific PKR response while maintaining the robust and specific suppression due to the long dsRNA, C2C12 cells were transfected with a vector that directs K3L expression. This additional step successfully attenuated the PKR response, however expression of K3L protein had no effect on the magnitude of specific inhibition.

B. However, since the efficacy of such a two step approach had not been previously demonstrated, it was formerly possible that dsRNA suppression would not be possible in cells with a PKR response. Figure 50 summarizes results which demonstrate that such a two step approach is possible, and that robust and specific dsRNA mediated suppression is possible in cells which had formerly possessed a robust PKR response.

Briefly, dual luciferase assay were carried out as described in detail above. The experiments were carried out using PKR -/- MEFs harvested from E13.5 PKR-/- mosue embryos. MEFs typically have a robust PKR response, and thus treatment with long dsRNAs typically results in non-specific suppression of gene expression and apoptosis. However, in PKR -/- cells examined 12, 42, and 82 hours after transfection, expression of ds*Renilla* luciferase RNA specifically suppresses expression *Renilla reniformis* (sea pansy) luciferase. This suppression is stable over time.

These results demonstrate that the non-specific PKR response can be blocked without affecting specific suppression of gene expression mediated by dsRNA. This allows the use of long dsRNAs to suppress gene expression in a diverse range of cell types, including those that would be previously intractable due to the confounding influences of the non-specific PKR response to long dsRNA.

### Example 9: Suppression of Gene Expression using dsRNA which Corresponds to Non-Coding Sequence

Current models for the mechanisms which drive RNAi have suggested that the dsRNA construct must contain coding sequence corresponding to the gene of interest. Although evidence has demonstrated that such coding sequence need not be a perfect match to the endogenous coding sequence (i.e., it may be similar), it has been widely held that the dsRNA construct must correspond to coding sequence. We present evidence that contradicts the teachings of the prior art, and demonstrate that dsRNA corresponding to non-coding regions of a gene can suppress gene function in vivo. These results are significant not only because they demonstrate that dsRNA identical or similar to non-coding sequences (i.e., promoter sequences, enhancer sequences, or intronic sequences) can mediate suppression, but also because we demonstrate the in vivo suppression of gene expression using dsRNA technology in a mouse model.

We generated doubled stranded RNA corresponding to four segments of the mouse tyrosinase gene promoter. Three of these segments correspond to the proximal promoter and one corresponds to an enhancer (Fig. 51). The tyrosinase gene encodes the rate limiting enzyme involved in the melanin biosynthetic pathway (Bilodeau et al. (2001) Pigment Cell Research 14: 328-336). Accordingly, suppression of the tyrosinase gene is expected to inhibit pigmentation.

Double stranded RNA corresponding to each of the above promoter segments was injected into the pronuclei of fertilized eggs. Pups were born after 19 days. In total 42/136 (31%) of the embryos were carried to term. This number is within the expected range for transgenesis (30-40%). Two pups out of 42 (5%) appear totally unpigmented at birth, consistent with suppression of tyrosinase function.

### Methods:

**dsRNA from non-coding promoter region of tyrosinase gene**. Four segments of the mouse tyrosinase gene promoter were amplified by PCR using primers which incorporated T7 RNA polymerase promoters into the PCR products (shown in bold - Fig. 51). Sequences of the mouse tyrosinase gene 5' flanking regions were obtained from GenBank (accession number D00439 and X51743). The sequence of the tyrosinase enhancer, located approximately 12 kb upstream of the transcriptional start site, was also obtained from GenBank (accession number X76647).

The sequences of the primers used were as follows: note the sequence of the T7 RNA polymerase promoter is shown in bold
(a) Tyrosinase enhancer (∼12 kb upstream):
   5' **TAATACGACTCACTATAGGG**CAAGGTCATAGTTCCTGCCAGCTG 3'
   5' **TAATACGACTCACTATAGGG**CAGATATTTTCTTACCACCCACCC 3'
(b) -1404 to -1007:
   5' **TAATACGACTCACTATAGGG**TTAAGTTTAACAGGAGAAGCTGGA 3'
   5' **TAATACGACTCACTATAGGG**AAATCATTGCTTTCCTGATAATGC 3'
(c) -1003 to -506:
   5' **TAATACGACTCACTATAGGG**TAGATTTCCGCAGCCCCAGTGTTC 3'
   5' **TAATACGACTCACTATAGGGG**TTGCCTCTCATTTTTCCTTGATT 3'
(d) -505 to -85:
   5' **TAATACGACTCACTATAGGG**TATTTTAGACTGATTACTTTTATAA 3'
   5' **TAATACGACTCACTATAGGG**TCACATGTTTTGGCTAAGACCTAT 3'

PCR products were gel purified from 1% TAE agarose gels using QiaExII Gel Extraction Kit (Qiagen). Double stranded RNA was produced from these templates using T7-Megashortscript Kit (Ambion). Enzymes and unincorporated nucleotides were removed using Qiaquick MinElute PCR Purification Kit. RNA was phenol/chloroform extracted twice, and ethanol precipitated. Pellets were resuspended in injection buffer ((10 mM Tris (pH 7.5), 0.15 nM EDTA (pH 8.0)) at a concentration of 20 ng/ul and run on a 1% TAE agarose gel to confirm integrity. **Generation of mice**: An equal mixture of double stranded RNA from each of the above primer sets was injected into the pronuclei of fertilized eggs from C57BL6J mice. A total of 136 injections was performed, and 34 embryos were implanted into each of 4 pseudopregnant CD-1 females. Pups were born after 19 days. In total, 42/136 (31%) of the embryos were carried to term. 2/42 pups (5%) appear totally unpigmented at birth.

It is not clear whether the RNAi mediated by dsRNA identical or similar to non-coding sequence works via the same mechanism as PTGS observed in the presence of dsRNA identical or similar to coding sequence. However, whether these results ultimately reveal similar or differing mechanisms does not diminish the tremendous utility of the compositions and methods of the present invention to suppress expression of one or more genes in vitro or in vivo.

The present invention demonstrates that dsRNA ranging in length from 20-500 nt can readily suppress expression of target genes both in vitro and in vivo. Furthermore, the present invention demonstrates that the dsRNAs can be generated using a variety of methods including the formation of hairpins, and that these dsRNAs can be expressed either stably or transiently. Finally, the present invention demonstrates that dsRNA identical or similar to non-coding sequences can suppress target gene expression.

### Example 10: RNA interference in adult mice

RNA interference is an evolutionarily conserved surveillance mechanism that responds to double-stranded RNA by sequence-specific silencing of homologous genes. Here we show that transgene expression can be suppressed in adult mice by synthetic small interfering RNAs and by small-hairpin RNAs transcribed *in vivo* from DNA templates. We also show the therapeutic potential of this technique by demonstrating effective targeting of a sequence from hepatitis C virus by RNA interference *in vivo.*

Small interfering RNAs (siRNAs) mimic intermediates in the RNA-interference (RNAi) pathway and can silence genes in somatic cells without activating non-specific suppression by double-stranded RNA-dependent protein kinase (Elbashir et al. 2001, Nature 411: 494-498). To investigate whether siRNAs also inhibit gene expression *in vivo,* we used a modification of hydrodynamic transfection methods (Zhang et al. 1999, Hum. Gene Therapy 10: 1735-1737;

Liu et al. 1999, Gene Therapy 6: 1258-1266;Chang et al. 2001, J. Virol. 75: 3469-3473) to deliver naked siRNAs to the livers of adult mice. Either an siRNA derived from firefly luciferase or an unrelated siRNA was co-injected with a luciferase-expression plasmid (for construct description and sequences, see Figure 52). We monitored luciferase expression in living animals using quantitative whole-body imaging (Contag, et al. 1997, Photochem. Photobiol. 66: 523-531) (see Fig. 53a, 54a), and found that it was dependent on reporter-plasmid dose.

In each experiment, serum measurements of a co-injected human ∝-1 antitrypsin (hAAT) plasmid (Yant et al. 2000, Nature Genet. 25: 35-41) served to normalize transfection efficiency and to monitor non-specific translational inhibition. Average serum concentrations of hAAT after 74 h were similar in all groups.

Our results indicate that there was specific, siRNA-mediated inhibition of luciferase expression in adult mice (P < 0.0115) and that unrelated siRNAs had no effect (P < 0.864; Fig. 53a, 53b). In 11 independent experiments, luciferase siRNAs reduced luciferase expression (as judged by emitted light) by an average of 81% (± 2.2%). These findings indicate that RNAi can downregulate gene expression in adult mice.

As RNAi degrades respiratory syncitial virus RNAs in culture (Bitko et al. 2001, BMC Microbiol. 1: 34), we investigated whether RNAi could be directed against a human pathogenic RNA expressed in a mouse, namely that of hepatitis C virus (HCV). Infection by HCV (an RNA virus that infects 1 in 40 people worldwide) is the most common reason for liver transplantation in the United States and Europe. We fused the NS5B region (non-structural protein 5B, viral-polymerase-encoding region) of this virus with luciferase RNA and monitored RNAi by co-transfection *in vivo.* An siRNA targeting the NS5B region reduced luciferase expression from the chimaeric HCV NS5B protein-luciferase fusion by 75% (± 6.8%; 6 animals per group). This result suggests that it may be feasible to use RNAi as a therapy against other important human pathogens.

Although our results show that siRNAs are functional in mice, delivery remains a major obstacle. Unlike siRNAs, functional small-hairpin RNAs (shRNAs) can be expressed *in vivo* from DNA templates using RNA polymerase III promoters (Paddison et al. 2002, Genes Dev. 16: 948-958; Tuschl 2002, Nature Biotechnol. 20: 446-448); they are as effective as siRNAs in inducing gene suppression. Expression of a cognate shRNA (pShh1-Ff1) inhibited luciferase expression by up to 98% (± 0.6%), with an average suppression of 92.8% (± 3.39%) in three independent experiments (see Fig. 54a, 54b). An empty shRNA-expression vector had no effect; reversing the orientation of the shRNA (pShh1-Ff1rev) insert prevents gene silencing because it alters the termination by RNA polymerase III and generates an improperly structured shRNA. These findings indicate that plasmid-encoded shRNAs can induce a potent and specific RNAi response in adult mice.

RNAi may find application in functional genomics or in identifying targets for designer drugs. It is a more promising system than gene-knockout mice because groups of genes can be simultaneously rendered ineffective without the need for time-consuming crosses. Gene therapy currently depends on the ectopic expression of exogenous proteins; however, RNAi may eventually complement this gain-of function approach by silencing disease-related genes with DNA constructs that direct the expression of shRNAs. Our method of RNAi delivery could also be tailored to take advantage of developing viral and non-viral gene-transfer vectors in a clinical context.

### Example 11: Germline transmission of RNAi in mice

MicroRNA molecules (miRNAs) are small, noncoding RNA molecules that have been found in a diverse array of eukaryotes, including mammals. miRNA precursors share a characteristic secondary structure, forming short 'hairpin' RNAs. Genetic and biochemical studies have indicated that miRNAs are processed to their mature forms by Dicer, an RNAse III family nuclease, and function through RNA-mediated interference (RNAi) and related pathways to regulate the expression of target genes (Hannon 2002, Nature 418: 244-251; Pasquinelli et al. 2002, Annu. Rev. Cell. Dev. Biol. 18: 495-513). Recently, we and others have remodeled miRNAs to permit experimental manipulation of gene expression in mammalian cells and have dubbed these synthetic silencing triggers 'short hairpin RNAs' (shRNAs) (Paddison et al. 2002, Cancer Cell 2: 17-23). Silencing by shRNAs requires the RNAi machinery and correlates with the production of small interfering RNAs (siRNAs), which are a signature of RNAi.

Expression of shRNAs can elicit either transient or stable silencing, depending upon whether the expression cassette is integrated into the genome of the recipient cultured cell (Paddison et al. 2002, Cancer Cell 2: 17-23). shRNA expression vectors also induce gene silencing in adult mice following transient delivery (Lewis et al. 2002,.Nat. Genet. 32: 107-108; McCaffrey et al. 2002, Nature 418: 38-39). However, for shRNAs to be a viable genetic tool in mice, stable manipulation of gene expression is essential. Hemann and colleagues have demonstrated long-term suppression of gene expression *in vivo* following retroviral delivery of shRNA-expression cassettes to hematopoietic stem cells (Hemann et al. 2003, Nat. Genet. in the press). Here we sought to test whether shRNA-expression cassettes that were passed through the mouse germline could enforce heritable gene silencing.

We began by taking standard transgenesis approaches (Gordon et al. 1993, Methods Enzymol. 225: 747-771) using shRNAs directed against a variety of targets with expected phenotypes, including the genes encoding tyrosinase (albino), myosin VIIa (shaker), Bmp-5 (crinkled ears), Hox a-10 (limb defects), homogentisate 1,2,-dioxygenase (urine turns black upon exposure to air), Hairless (hair loss) and melanocortin 1 receptor (yellow). Three constructs per gene were linearized and injected into pronuclei to produce transgenic founder animals. Although we noted the presence of the transgene in some animals, virtually none showed a distinct or reproducible phenotype that was expected for a hypomorphic allele of the targeted gene.

Therefore, we decided to take another approach: verifying the presence of the shRNA and its activity toward a target gene in cultured embryonic stem (ES) cells and then asking whether those cells retained suppression in a chimeric animal *in vivo.* We also planned to test whether such cells could pass a functional RNAi-inducing construct through the mouse germline. For these studies, we chose to examine a novel gene, *Neill,* which is proposed to have a role in DNA repair. Oxidative damage accounts for 10,000 DNA lesions per cell per day in humans and is thought to contribute to carcinogenesis, aging and tissue damage following ischemia (Ames et al. 1993, Proc. Natl. Acad. Sci. USA 90: 7915-7922; Jackson et al. 2001, Mutat. Res. 477: 7-21). Oxidative DNA damage includes abasic sites, strand breaks and at least 20 oxidized bases, many of which are cytotoxic or pro-mutagenic (Dizdaroglu et al. 2002, Free Radic. Biol. Med. 32: 1102-1115). DNA N-glycosylases initiate the base excision repair pathway by recognizing specific bases in DNA and cleaving the sugar base bond to release the damaged base (David et al. 1998, Chem. Rev. 98: 1221-1262).

The *Neil* genes are a newly discovered family of mammalian DNA *N-*glycosylases related to the Fpg/Nei family of proteins from *Escherichia coli* (Hazra et al. 2002, Proc. Natl. Acad. Sci. USA 99: 3523-3528; Bandaru et al. 2002, DNA Repair 1: 517-529). Neil1 recognizes and removes a wide spectrum of oxidized pyrimidines and ring-opened purines from DNA, including thymine glycol (Tg), 2,6-diamino-4-hydroxy-5-formamidopyrimidine (FapyG) and 4,6-diamino-5-formidopyrimidine (FapyA). Tg, FapyG and FapyA are among the most prevalent oxidized bases produced by ionizing radiation (Dizdaroglu et al. 2002, Free Radic. Biol. Med. 32: 1102-1115) and can block replicative DNA polymerases, which can, in turn, cause cell death (Asagoshi et al. 2002, J. Biol. Chem. 277: 14589-14597; Clark et al. 1989, Biochemistry 28: 775-779).

The Nth1 and Ogg1 glycosylases each remove subsets of oxidized DNA bases that overlap with substrates of Neil1 (Nishimura 2002, Free Radic. Biol. Med. 32: 813-821;
Asagoshi et al. 2000, Biochemistry 39: 11389-11398; Dizdaroglu et al. 1999, Biochemistry 38: 243-246). However, mice with null mutations in either *Nth1* (Ocampo et al. 2002, Mol. Cell. Biol. 22: 6111-6121; Takao et al. 2002, EMBO J. 21: 3486-3493) or *Ogg1* (Klungland et al. 1999, Proc. Natl. Acad. Sci. USA 96: 13300-13305; Minowa et al. 2000, Proc. Natl. Acad. Sci. USA 97: 4156-4161) are viable, raising the possibility that Neil1 activity tempers the loss of Nth1 or Ogg1. Recently, a residual Tg-DNA glycosylase activity in *Nth1*^{*-*/}*⁻* mice has been identified as Neil1 (Takao et al. 2002, J. Biol. Chem. 277: 42205-42213).

We constructed a single shRNA expression vector targeting a sequence near the 5' end of the *Neill* coding region. This vector was introduced into mouse embryonic stem cells by electroporation, and individual stable integrants were tested for expression of the Neil1 protein (see the weblink: http://www.cshl.edu/public/SCIENCE/hannon.html for detailed procedures). The majority of cell lines showed an -80% reduction in Neil1 protein, which correlated with a similar change in levels of *Neill* mRNA. These cells showed an approximately two-fold increase in their sensitivity to ionizing radiation, consistent with a role for Neil1 in DNA repair. Two independent ES cell lines were injected into BL/6 blastocysts, and several high-percentage chimeras were obtained. These chimeras were out-crossed, and germline transmission of the shRNA-expression construct was noted in numerous F₁ progeny (13/27 for one line and 12/26 for the other).

To determine whether the silencing of *Neill* that had been observed in ES cells was transmitted faithfully, we examined *Neill* mRNA and protein levels. Both were reduced by approximately the same extent that had been observed in the engineered ES cells (Figs. 55, 56). Consistent with this having occurred through the RNAi pathway, we detected the presence of siRNAs corresponding to the shRNA sequence in F₁ animals that carry the shRNA expression vector but not in those that lack the vector (Fig. 56b).

The aforementioned data demonstrate that shRNAs can be used to create germline transgenic mice in which RNAi has silenced a target gene. These observations open the door to using of RNAi as a complement to standard knock-out methodologies and provide a means to rapidly assess the consequences of suppressing a gene of interest in a living animal. Coupled with activator-dependent U6 promoters, the use of shRNAs will ultimately provide methods for tissue-specific, inducible and reversible suppression of gene expression in mice.

### V. Equivalents

Those skilled in the art will recognize, or be able to ascertain using no more than routine experimentation, many equivalents to the specific embodiments of the invention described herein. Such equivalents are intended to be encompassed by the following claims.

## Claims

1. A method for attenuating expression of a target gene in a mammalian cell, the method comprising introducing into the mammalian cell *in vitro,* a chromosomally integrating expression vector comprising a sequence encoding a short hairpin RNA (shRNA), wherein the shRNA is expressed in the mammalian cell and comprises a double stranded region of between 20 and 29 bases in length, the double stranded region comprising a sequence that hybridizes under stringent conditions to a coding region of the target gene, whereby expression of the target gene is attenuated.

2. The method of claim 1, wherein the shRNA is stably expressed in the mammalian cell.

3. The method of claim 1 or 2, wherein the expression vector further comprises a transcription regulatory sequence operably linked to the sequence encoding the shRNA.

4. The method of claim 1, wherein the double stranded region is at least 21, or 22 nucleotides in length.

5. The method of claim 1, wherein the double stranded region is at least 25 nucleotides in length.

6. The method of claim 1, wherein the double stranded region is 29 nucleotides in length.

7. The method according to any proceeding claim wherein expression of the target gene is attenuated by at least 10 fold relative to an untreated cell or a cell treated with a ds RNA construct which does not correspond to the target gene.

8. A chromosomally integrating expression vector for use in a method of attenuating expression of a target gene in a mammalian cell, the expression vector comprising a sequence encoding a short hairpin RNA (shRNA), wherein the shRNA comprises a double stranded region of between 20 and 29 base pairs in length, the double stranded region comprising a sequence that hybridizes under stringent conditions to a coding region of the target gene.

9. An expression vector according to claim 8 for attenuating expression of a target gene In a mammalian cell *in vivo.*

10. Use of a chromosomally integrating expression vector for the manufacture of a medicament for use in therapy, the expression vector comprising a sequence encoding a short hairpin RNA (shRNA), wherein the shRNA comprises a double stranded region of between 20 and 29 base pairs in length, the double stranded region comprising a sequence that hybridizes under stringent conditions to a coding region of the target gene, whereby expression of the target gene is attenuated.

## Patentansprüche

1. Verfahren zur Verminderung der Expression eines Zielgens in einer Säugerzelle, wobei das Verfahren das in vitro Einführen eines chromosal integrierten Expressionsvektors in die Säugerzelle umfasst, der eine short hairpin RNA (shRNA) kodierende Sequenz umfasst, wobei die shRNA in der Säugerzelle exprimiert wird und eine doppelsträngige Region von einer Länge von zwischen 20 und 29 Basen umfasst, wobei die doppelsträngige Region eine Sequenz umfasst, die unter stringenten Bedingungen mit einer kodierenden Region des Zielgens hybridisiert, wodurch die Expression des Zielgens vermindert wird.

2. Verfahren nach Anspruch 1, wobei die shRNA stabil in der Säugerzelle exprimiert ist.

3. Verfahren nach Anspruch 1 oder 2, wobei der Expressionsvektor ferner eine regulatorische Transkriptionssequenz umfasst, die funktionsfähig mit der die shRNA kodierenden Sequenz verknüpft ist.

4. Verfahren nach Anspruch 1, wobei die doppelsträngige Region eine Länge von mindestens 21 oder 22 Nukleotiden hat.

5. Verfahren nach Anspruch 1, wobei die doppelsträngige Region eine Länge von mindestens 25 Nukleotiden hat.

6. Verfahren nach Anspruch 1, wobei die doppelsträngige Region eine Länge von 29 Nukleotiden hat.

7. Verfahren nach einem der vorangehenden Ansprüche, wobei die Expression des Zielgens mindestens 10-fach vermindert ist, bezüglich einer unbehandelten Zelle oder mit einer dsRNA-Konstruktion behandelten Zelle, die dem Zielgen nicht entspricht.

8. Chromosomal integrierter Expressionsvektor zur Anwendung in einem Verfahren zur Verminderung der Expression eines Zielgens in einer Säugerzelle, wobei der Expressionsvektor eine short hairpin RNA (shRNA) kodierende Sequenz umfasst, wobei die shRNA eine doppelsträngige Region von einer Länge von zwischen 20 und 29 Basenpaaren umfasst, wobei die doppelsträngige Region eine Sequenz umfasst, die unter stringenten Bedingungen mit einer kodierenden Region des Zielgens hybridisiert.

9. Expressionsvektor nach Anspruch 8 zur Verminderung der in vivo Expression eines Zielgens in einer Säugerzelle.

10. Anwendung eines chromosal integrierten Expressionsvektors zur Herstellung eines Arzneimittels zur Anwendung in der Therapie, wobei der Expressionsvektor eine short hairpin RNA (shRNA) kodierende Sequenz umfasst, wobei die shRNA eine doppelsträngige Region von einer Länge von zwischen 20 und 29 Basenpaaren umfasst, wobei die doppelsträngige Region eine Sequenz umfasst, die unter stringenten Bedingungen mit einer kodierenden Region des Zielgens hybridisiert, wodurch die Expression des Zielgens vermindert wird.

## Revendications

1. Procédé pour atténuer l'expression d'un gène cible dans une cellule de mammifère, le procédé comprenant l'introduction dans la cellule de mammifère in vitro, d'un vecteur d'expression chromosomiquement intégré comprenant une séquence codant pour un ARN short hairpin (shRNA), dans lequel le shRNA est exprimé dans la cellule de mammifère et comprend une région à double brin d'une longueur de 20 à 29 bases, la région à double brin comprenant une séquence qui s'hybride dans des conditions stringentes avec une région codante du gène cible, l'expression du gène cible étant ainsi atténuée.

2. Procédé selon la revendication 1, dans lequel le shRNA est exprimé de manière stable dans la cellule de mammifère.

3. Procédé selon la revendication 1 ou 2, dans lequel le vecteur d'expression comprend en outre une séquence régulatrice de transcription fonctionnellement liée à la séquence codant pour le shRNA.

4. Procédé selon la revendication 1, dans lequel la région à double brin a une longueur d'au moins 21 ou 22 nucléotides.

5. Procédé selon la revendication 1, dans lequel la région à double brin a une longueur d'au moins 25 nucléotides.

6. Procédé selon la revendication 1, dans lequel la région à double brin a une longueur de 29 nucléotides.

7. Procédé selon l'une quelconque des revendications précédentes, dans lequel l'expression du gène cible est atténuée au moins dix fois par rapport à une cellule non traitée ou une cellule traitée avec une construction de ds RNA qui ne correspond pas au gène cible.

8. Vecteur d'expression chromosomiquement intégré pour une utilisation dans une méthode pour atténuer l'expression d'un gène cible dans une cellule de mammifère, le vecteur d'expression comprenant une séquence codant pour un ARN short hairpin (shRNA), le shRNA comprenant une région à double brin d'une longueur de 20 à 29 paires de base, la région double brin comprenant une séquence qui s'hybride dans des conditions stringentes avec une région codante du gène cible.

9. Vecteur d'expression selon la revendication 8 pour atténuer l'expression d'un gène cible dans une cellule de mammifère in vivo.

10. Utilisation d'un vecteur d'expression chromosomiquement intégré pour la fabrication d'un médicament pour une utilisation en thérapie, le vecteur d'expression comprenant une séquence codant pour un ARN short hairpin (shRNA), dans laquelle le shRNA comprend une région à double brin d'une longueur de 20 à 29 paires de base, la région à double brin comprenant une séquence qui s'hybride dans des conditions stringentes avec une région codante du gène cible, l'expression du gène cible étant ainsi atténuée.
